(19) Europäisches Patentamt
European Patent Office
Office européen des brevets

(11) **EP 4 446 321 A1**

(12) **EUROPEAN PATENT APPLICATION**
published in accordance with Art. 153(4) EPC

(43) Date of publication:
**16.10.2024 Bulletin 2024/42**

(21) Application number: **23831791.1**

(22) Date of filing: **20.06.2023**

(51) International Patent Classification (IPC):
***C07D 407/10*** (2006.01) ***C07D 405/10*** (2006.01)
***C07D 409/14*** (2006.01) ***C07D 491/048*** (2006.01)
***C07D 413/10*** (2006.01) ***C07D 409/10*** (2006.01)
***H10K 85/60*** (2023.01)

(52) Cooperative Patent Classification (CPC):
**C07D 405/10; C07D 407/10; C07D 409/10; C07D 409/14; C07D 413/10; C07D 491/048; H10K 50/00; H10K 85/60; H10K 99/00**

(86) International application number:
**PCT/KR2023/008514**

(87) International publication number:
**WO 2024/005431 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.06.2022 KR 20220079957**

(71) Applicant: **LG Chem, Ltd.**
**Yeongdeungpo-gu**
**Seoul 07336 (KR)**

(72) Inventors:
- **KIM, Seonwoo**
  **Daejeon 34122 (KR)**
- **HA, Jae Seung**
  **Daejeon 34122 (KR)**
- **GEUM, Sujeong**
  **Daejeon 34122 (KR)**
- **CHO, Woo Jin**
  **Daejeon 34122 (KR)**
- **CHA, Yongbum**
  **Daejeon 34122 (KR)**
- **HWANG, Sunghyun**
  **Daejeon 34122 (KR)**
- **LEE, Woochul**
  **Daejeon 34122 (KR)**

(74) Representative: **Hoffmann Eitle**
**Patent- und Rechtsanwälte PartmbB**
**Arabellastraße 30**
**81925 München (DE)**

(54) **COMPOUND AND ORGANIC LIGHT-EMITTING DEVICE COMPRISING SAME**

(57) The present specification relates to a compound of Chemical Formula 1 and an organic light emitting device including the same.

[Figure 1]

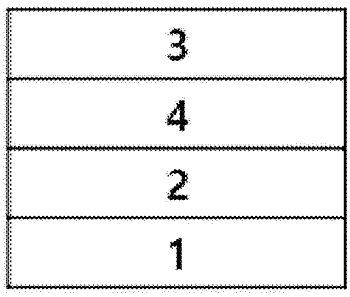


EP 4 446 321 A1

## Description

[Technical Field]

This application claims priority to and the benefit of Korean Patent Application No. 10-2022-0079957 filed in the Korean Intellectual Property Office on June 29, 2022, the entire contents of which are incorporated herein by reference.
The present specification relates to a compound and an organic light emitting device including the same.

[Background Art]

An organic light emission phenomenon generally refers to a phenomenon converting electrical energy to light energy using an organic material. An organic light emitting device using an organic light emission phenomenon normally has a structure including a positive electrode, a negative electrode, and an organic material layer therebetween. Here, the organic material layer has in many cases a multi-layered structure composed of different materials in order to improve the efficiency and stability of the organic light emitting device, and for example, may be composed of a hole injection layer, a hole transport layer, a light emitting layer, an electron transport layer, an electron injection layer, and the like. In such a structure of the organic light emitting device, if a voltage is applied between the two electrodes, holes are injected from the positive electrode into the organic material layer and electrons are injected from the negative electrode into the organic material layer, and when the injected holes and electrons meet each other, an exciton is formed, and light is emitted when the exciton falls down again to a ground state.
There is a continuous need for developing a new material for the aforementioned organic light emitting device.

[Detailed Description of the Invention]

[Technical Problem]

The present specification provides a compound and an organic light emitting device including the same.

[Technical Solution]

The present specification provides a compound of the following Chemical Formula 1.

[Chemical Formula 1]

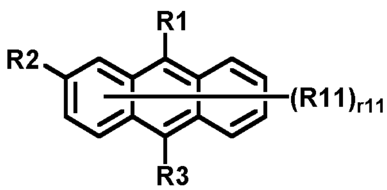

In Chemical Formula 1,

at least one of R1 to R3 is -L-Arl, at least one of the rest of R1 to R3, which are not -L-Arl, is a substituted or unsubstituted heteroaryl group, and the other and R11 are the same as or different from each other, and are each independently hydrogen; deuterium; a substituted or unsubstituted aryl group; or a substituted or unsubstituted heteroaryl group,
r11 is an integer from 1 to 7, and when r11 is 2 or higher, two or more R11's are the same as or different from each other,
L is a direct bond; a substituted or unsubstituted arylene group; or a substituted or unsubstituted heteroarylene group, and
Ar1 is the following Chemical Formula A,

[Chemical Formula A]

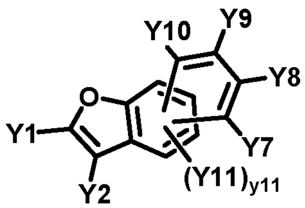

in Chemical Formula A,

any one of Y1, Y2 and Y7 to Y11 is a moiety bonded to L, and the others of Y1, Y2 and Y7 to Y11 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring, and

y11 is 1 or 2, and when y11 is 2, two Y11's are the same as or different from each other.

Further, an exemplary embodiment of the present specification provides an organic light emitting device including: a first electrode; a second electrode; and an organic material layer having one or more layers provided between the first electrode and the second electrode, in which one or more layers of the organic material layer include the above-described compound.

[Advantageous Effects]

The compound according to an exemplary embodiment of the present specification can be used for an organic material layer of an organic light emitting device to lower the driving voltage of the organic light emitting device and improve the light efficiency. Further, the service life characteristics of the device can be improved by the thermal stability of the compound.

[Brief Description of Drawings]

FIGS. 1 and 2 illustrate an example of an organic light emitting device according to an exemplary embodiment of the present specification.

[Explanation of Reference Numerals and Symbols]

1: Substrate
2: First electrode
3: Second electrode
4: Light emitting layer
5: Hole injection layer
6: Hole transport layer
7: Hole adjusting layer
8: Electron adjusting layer
9: Electron transport layer
10: Electron injection layer
11: Capping layer

[Best Mode]

Hereinafter, the present specification will be described in more detail.

An exemplary embodiment of the present specification provides the compound of Chemical Formula 1.

Since Chemical Formula 1 according to an exemplary embodiment of the present specification includes a compound in which naphthofuran is bonded to at least one of Positions 2, 9 and 10 of anthracene through L, and at least one of the rest of Positions 2, 9 and 10 of the anthracene, which are not -L-naphthofuran, is a substituted or unsubstituted heteroaryl group, Chemical Formula 1 has structural characeristics in which the mobility of electrons and holes is enhanced and molecular stability is improved. Therefore, an organic light emitting device including the same is excellent in terms of driving voltage, efficiency and service life.

Throughout the specification of the present application, the term "combination thereof" included in the Markush type expression means a mixture or combination of one or more selected from the group consisting of constituent elements described in the Markush type expression, and means including one or more selected from the group consisting of the above-described constituent elements.

In the present specification,

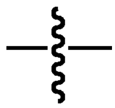

means a moiety to be linked.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the term "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; and a heterocyclic group, being substituted with a substituent to which two or more substituents among the exemplified substituents are linked, or having no substituent.

In the present specification, the fact that two or more substituents are linked indicates that hydrogen of any one substituent is linked to another substituent. For example, when two substituents are linked to each other, a phenyl group and a naphthyl group may be linked to each other to become a substituent of

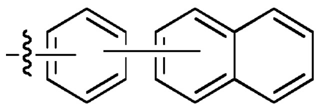

or

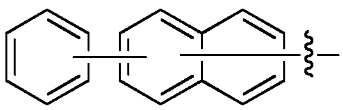

.

Further, the case where three substituents are linked to one another includes not only a case where (Substituent 1)-(Substituent 2)-(Substituent 3) are consecutively linked to one another, but also a case where (Substituent 2) and (Substituent 3) are linked to (Substituent 1). For example, a phenyl group, a naphthyl group, and an isopropyl group may be linked to one another to become a substituent of

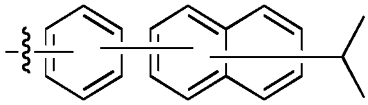

,

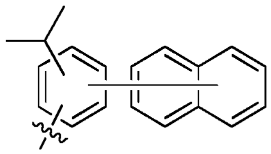, or 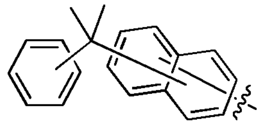.

The above-described definition also applies equally to the case where four or more substituents are linked to one another.

In the present specification, examples of a halogen group include fluorine, chlorine, bromine or iodine.

In the present specification, the alkyl group may be straight-chained or branched, and the number of carbon atoms thereof is not particularly limited, but is preferably 1 to 30. Specific examples thereof include methyl, ethyl, propyl, n-propyl, isopropyl, butyl, n-butyl, isobutyl, tert-butyl, sec-butyl, 1-methyl-butyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present specification, a cycloalkyl group is not particularly limited, but has preferably 3 to 30 carbon atoms, and specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, an adamantyl group, a bicyclo[2.2.1]heptyl group, a bicyclo[2.2.1]octyl group, a norbornyl group, and the like, but are not limited thereto.

In the present specification, the alkoxy group may be straight-chained, branched, or cyclic. The number of carbon atoms of the alkoxy group is not particularly limited, but is preferably 1 to 30. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the alkenyl group may be straight-chained or branched, and the number of carbon atoms thereof is not particularly limited, but is preferably 2 to 30. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, the haloalkyl group means that at least one halogen group is substituted instead of hydrogen in an alkyl group in the definition of the alkyl group.

In the present specification, the haloalkoxy group means that at least one halogen group is substituted instead of hydrogen in an alkoxy group in the definition of the alkoxy group.

In the present specification, an aryl group is not particularly limited, but has preferably 6 to 30 carbon atoms, and the aryl group may be monocyclic or polycyclic. When the aryl group is a monocyclic aryl group, the number of carbon atoms thereof is not particularly limited, but is preferably 6 to 30. Specific examples of the monocyclic aryl group include a phenyl group, a biphenyl group, a terphenyl group, and the like, but are not limited thereto.

When the aryl group is a polycyclic aryl group, the number of carbon atoms thereof is not particularly limited, but is preferably 10 to 30. Specific examples of the polycyclic aryl group include a naphthyl group, an anthracene group, a phenanthrene group, a triphenylene group, a pyrene group, a phenalene group, a perylene group, a chrysene group, a fluorene group, and the like, but are not limited thereto.

In the present specification, the fluorene group may be substituted, and adjacent groups may be bonded to each other to form a ring.

Examples of the fluorene group include

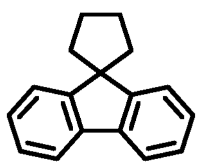, 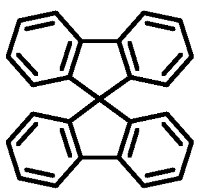, 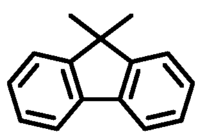, 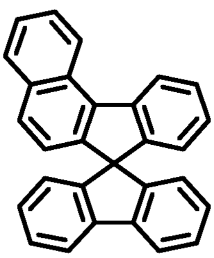,

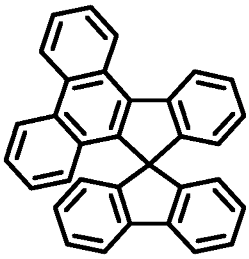, 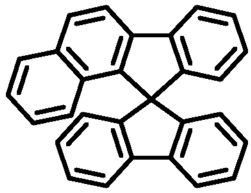, 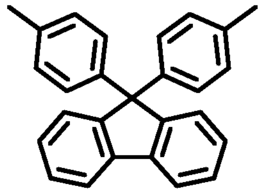, 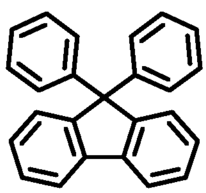,

and the like, but are not limited thereto.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, an arylalkyl group means that the alkyl group is substituted with an aryl group, and examples of the aryl group and the alkyl group described above may be applied to the aryl group and the alkyl group of the arylalkyl group.

In the present specification, an aryloxy group means that in the definition of the alkoxy group, the alkoxy group is substituted with an aryl group instead of an alkyl group, and examples of the aryloxy group include a phenoxy group, a p-tolyloxy group, an m-tolyloxy group, a 3,5-dimethyl-phenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group, and the like, but are not limited thereto.

In the present specification, the alkyl group in the alkylthioxy group is the same as the above-described examples of the alkyl group. Specific examples of the alkylthioxy group include a methylthioxy group, an ethylthioxy group, a tert-butylthioxy group, a hexylthioxy group, an octylthioxy group, and the like, but are not limited thereto.

In the present specification, the aryl group in the arylthioxy group is the same as the above-described examples of the aryl group. Specific examples of the arylthioxy group include a phenylthioxy group, a 2-methylphenylthioxy group, a 4-tert- butylphenylthioxy group, and the like, but are not limited thereto.

In the present specification, a heterocyclic group includes one or more atoms other than carbon, that is, one or more heteroatoms, and specifically, the heteroatom may include one or more atoms selected from the group consisting of O, N, Se, S, and the like, and includes an aromatic heterocyclic group, or an aliphatic heterocyclic group. The aromatic heterocyclic group may be represented by a heteroaryl group. The number of carbon atoms of the heterocyclic group is not particularly limited, but is preferably 2 to 30, and the heterocyclic group may be monocyclic or polycyclic. Examples of the heterocyclic group include a thiophene group, a furan group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthridine group, a phenanthroline group, an isoxazole group, a thiadiazole group, a dibenzofuran group, a dibenzosilole group, a phenoxathiin group, a phenoxazine group, a phenothiazine group, a decahydrobenzocarbazole group, a hexahydrocarbazole group, a dihydrobenzoazasiline group, a dihydroindenocarbazole group, a spirofluorenexanthene group, a spirofluorenethioxanthene group, a tetrahydronaphthothiophene group, a tetrahydronaphthofuran group, a tetrahydrobenzothiophene group, a tetrahydrobenzofuran group, and the like, but are not limited thereto.

In the present specification, the silyl group may be an alkylsilyl group, an arylsilyl group, an alkylarylsilyl group, a heteroarylsilyl group, and the like. The above-described examples of the alkyl group may be applied to the alkyl group in the alkylsilyl group, the above-described examples of the aryl group may be applied to the aryl group in the arylsilyl group, the examples of the alkyl group and the aryl group may be applied to the alkyl group and the aryl group in the alkylarylsilyl group, and the examples of the heterocyclic group may be applied to the heteroaryl group in the heteroarylsilyl group.

In the present specification, a boron group may be - $BY_{100}Y_{101}$, and $Y_{100}$ and $Y_{101}$ are the same as or different from each other, and may be each independently selected from the group consisting of hydrogen; deuterium; halogen; a nitrile group; a substituted or unsubstituted monocyclic or polycyclic cycloalkyl group having 3 to 30 carbon atoms; a substituted or unsubstituted straight-chained or branched alkyl group having 1 to 30 carbon atoms; a substituted or

unsubstituted monocyclic or polycyclic aryl group having 6 to 30 carbon atoms; and a substituted or unsubstituted monocyclic or polycyclic heterocyclic group having 2 to 30 carbon atoms. Specific examples of the boron group include a dimethylboron group, a diethylboron group, a t-butylmethylboron group, a diphenylboron group, and the like, but are not limited thereto.

In the present specification, an amine group may be selected from the group consisting of $-NH_2$, an alkylamine group, an N-alkylarylamine group, an arylamine group, an N-arylheteroarylamine group, an N-alkylheteroarylamine group, and a heteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a ditolylamine group, an N-phenyltolylamine group, an N-phenylbiphenylamine group, an N-phenylnaphthylamine group, an N-biphenylnaphthylamine group, an N-naphthylfluorenylamine group, an N-phenylphenanthrenylamine group, an N-biphenylphenanthrenylamine group, an N-phenylfluorenylamine group, an N-phenyl terphenylamine group, an N-phenanthrenylfluorenylamine group, an N-biphenylfluorenylamine group, and the like, but are not limited thereto.

In the present specification, an N-alkylarylamine group means an amine group in which an alkyl group and an aryl group are substituted with N of the amine group. The alkyl group and the aryl group in the N-alkylarylamine group are the same as the above-described examples of the alkyl group and the aryl group.

In the present specification, an N-arylheteroarylamine group means an amine group in which an aryl group and a heteroaryl group are substituted with N of the amine group. The aryl group and heteroaryl group in the N-arylheteroarylamine group are the same as the above-described examples of the aryl group and the heterocyclic group.

In the present specification, an N-alkylheteroarylamine group means an amine group in which an alkyl group and a heteroaryl group are substituted with N of the amine group. The alkyl group and the heteroaryl group in the N-alkylheteroarylamine group are the same as the above-described examples of the alkyl group and the heterocyclic group.

In the present specification, examples of an alkylamine group include a substituted or unsubstituted monoalkylamine group or a substituted or unsubstituted dialkylamine group. The alkyl group in the alkylamine group may be a straight-chained or branched alkyl group. The alkylamine group including two or more alkyl groups may include a straight-chained alkyl group, a branched alkyl group, or both a straight-chained alkyl group and a branched alkyl group. For example, the alkyl group in the alkylamine group may be selected from the above-described examples of the alkyl group.

In the present specification, examples of a heteroarylamine group include a substituted or unsubstituted monoheteroarylamine group or a substituted or unsubstituted diheteroarylamine group. The heteroarylamine group including two or more heteroaryl groups may include a monocyclic heteroaryl group, a polycyclic heteroaryl group, or both a monocyclic heteroaryl group and a polycyclic heteroaryl group. For example, the heteroaryl group in the heteroarylamine group may be selected from the above-described examples of the heterocyclic group.

In the present specification, the alkyl group in the N-alkylarylamine group, the alkylthioxy group, and the N-alkylheteroarylamine group is the same as the above-described examples of the alkyl group. Specific examples of the alkylthioxy group include a methylthioxy group, an ethylthioxy group, a tert-butylthioxy group, a hexylthioxy group, an octylthioxy group, and the like, but are not limited thereto.

In the present specification, the aryl group in the aryloxy group, the arylthioxy group, the N-arylalkylamine group, and the N-arylheteroarylamine group is the same as the above-described examples of the aryl group. Specifically, examples of the aryloxy group include a phenoxy group, a p-tolyloxy group, an m-tolyloxy group, a 3,5-dimethyl-phenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group, and the like, and examples of the arylthioxy group include a phenylthioxy group, a 2-methylphenylthioxy group, a 4-tert-butylphenylthioxy group, and the like, but the examples are not limited thereto.

In the present specification, a hydrocarbon ring group may be an aromatic hydrocarbon ring group, an aliphatic hydrocarbon ring group, or a fused group of an aromatic hydrocarbon ring and an aliphatic hydrocarbon ring and may be selected among examples of the cycloalkyl group, the aryl group, and a combination thereof, and examples of the hydrocarbon ring group include a phenyl group, a cyclohexyl group, an adamantyl group, a bicylo[2.2.1]heptyl group, a bicyclo[2.2.1]octyl group, a tetrahydronaphthalene group, a tetrahydroanthracene group, a 1,2,3,4-tetrahydro-1,4-methanonaphthalene group, a 1,2,3,4-tetrahydro-1,4-ethanonaphthalene group, a spirocyclopentanefluorene group, a spiroadamantanefluorene group, a spirocyclohexanefluorene group, and the like, but are not limited thereto.

In the present specification, the meaning of "adjacent" in "bonded to an adjacent group to form a ring" is as described above, and the "ring" means a substituted or unsubstituted hydrocarbon ring; or a substituted or unsubstituted hetero ring.

In the present specification, the hydrocarbon ring may be an aromatic hydrocarbon ring, an aliphatic hydrocarbon ring, or a fused ring of the aromatic hydrocarbon ring and the aliphatic hydrocarbon ring and may be selected among

examples of the cycloalkyl group, the aryl group, and a combination thereof except for those which are not monovalent, and examples of the hydrocarbon ring include benzene, cyclohexane, adamantane, bicyclo[2.2.1]heptane, bicyclo[2.2.1]octane, tetrahydronaphthalene, tetrahydroanthracene, 1,2,3,4-tetrahydro-1,4-methanonaphthalene, 1,2,3,4-tetrahydro-1,4-ethanonaphthalene, spiro cyclopentane fluorene, spiro adamantane fluorene, spiro cyclohexane fluorene, and the like, but are not limited thereto.

In the present specification, a hetero ring includes one or more atoms other than carbon, that is, one or more heteroatoms, and specifically, the heteroatom may include one or more atoms selected from the group consisting of O, N, Se, S, and the like. The hetero ring may be monocyclic or polycyclic and may be an aromatic ring, an aliphatic ring, or a fused ring of the aromatic ring and the aliphatic ring, and the aromatic hetero ring may be selected from the examples of the heteroaryl group in the heterocyclic group, except for the aromatic hetero ring which is not monovalent.

In the present specification, an aliphatic hetero ring means an aliphatic ring including one or more of hetero atoms. Further, the aliphatic hetero ring means any hetero ring excluding an aromatic hetero ring, and a hetero ring, which includes a double bond, but is not aromatic, is an aliphatic hetero ring. Examples of the aliphatic hetero ring include oxirane, tetrahydrofuran, 1,4-dioxane, pyrrolidine, piperidine, morpholine, oxepane, azocane, thiocane, tetrahydronaphthothiophene, tetrahydronaphthofuran, tetrahydrobenzothiophene, tetrahydrobenzofuran, and the like, but are not limited thereto.

In the present specification, the aliphatic hetero ring (group) means an aliphatic ring (group) including one or more heteroatoms. Furthermore, the aliphatic hetero ring (group) means any hetero ring (group) excluding an aromatic hetero ring (group), and a hetero ring (group), which includes a double bond, but is not aromatic, is an aliphatic hetero ring (group).

In the present specification, examples of an aliphatic ring, or a fused hetero ring of the aromatic ring and the aliphatic ring include tetrahydrobenzonaphthothiophene, tetrahydrobenzonaphthofuran, and the like, but are not limited thereto.

In the present specification, an arylene group means a group having two bonding positions in an aryl group, that is, a divalent group. The above-described description on the aryl group may be applied to the arylene group, except for a divalent group.

In the present specification, a heteroarylene group means a group having two bonding positions in a heteroaryl group, that is, a divalent group. The above-described description on the heteroaryl group in the heterocyclic group may be applied to the heteroarylene group, except for a divalent heteroarylene group.

Unless otherwise defined in the present specification, all technical and scientific terms used in the present specification have the same meaning as commonly understood by one with ordinary skill in the art to which the present invention pertains. Although methods and materials similar to or equivalent to those described in the present specification may be used in the practice or in the test of exemplary embodiments of the present invention, suitable methods and materials will be described below. All publications, patent applications, patents, and other references mentioned in the present specification are hereby incorporated by reference in their entireties, and in the case of conflict, the present specification, including definitions, will control unless a particular passage is mentioned. In addition, the materials, methods, and examples are illustrative only and are not intended to be limiting.

Hereinafter, the compound of Chemical Formula 1 will be described in detail.

According to an exemplary embodiment of the present specification, Chemical Formula A is any one of the following Chemical Formulae A-1 to A-3.

[Chemical Formula A-1]

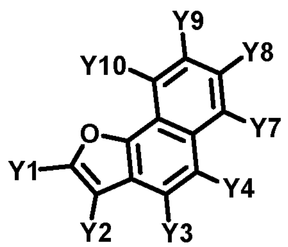

[Chemical Formula A-2]

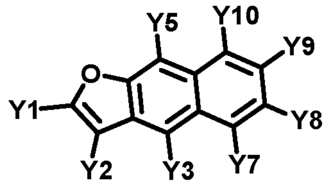

[Chemical Formula A-3]

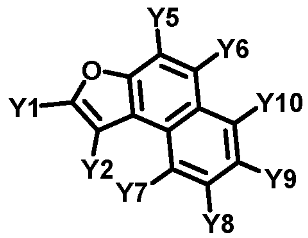

In Chemical Formula A-1,

any one of Y1 to Y4 and Y7 to Y10 is a moiety bonded to L, and the others of Y1 to Y4 and Y7 to Y10 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
in Chemical Formula A-2,

any one of Y1 to Y3, Y5 and Y7 to Y10 is a moiety bonded to L, and the others of Y1 to Y3, Y5 and Y7 to Y10 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; or a substituted or unsubstituted heteroaryl group, or any one or more adjacent pairs are bonded to each other to form a substituted or unsubstituted ring,
in Chemical Formula A-3,
any one of Y1, Y2 and Y5 to Y10 is a moiety bonded to L, and the others of Y1, Y2 and Y5 to Y10 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring.

According to an exemplary embodiment of the present specification, in Chemical Formula A-1, Y1 is a moiety bonded to L.
According to an exemplary embodiment of the present specification, in Chemical Formula A-1, Y2 is a moiety bonded to L.
According to an exemplary embodiment of the present specification, in Chemical Formula A-1, Y3 is a moiety

bonded to L.

According to an exemplary embodiment of the present specification, in Chemical Formula A-1, Y4 is a moiety bonded to L.

According to an exemplary embodiment of the present specification, in Chemical Formula A-1, Y7 is a moiety bonded to L.

According to an exemplary embodiment of the present specification, in Chemical Formula A-1, Y8 is a moiety bonded to L.

According to an exemplary embodiment of the present specification, in Chemical Formula A-1, Y9 is a moiety bonded to L.

According to an exemplary embodiment of the present specification, in Chemical Formula A-1, Y10 is a moiety bonded to L.

According to an exemplary embodiment of the present specification, in Chemical Formula A-2, Y1 is a moiety bonded to L.

According to an exemplary embodiment of the present specification, in Chemical Formula A-2, Y2 is a moiety bonded to L.

According to an exemplary embodiment of the present specification, in Chemical Formula A-2, Y3 is a moiety bonded to L.

According to an exemplary embodiment of the present specification, in Chemical Formula A-2, Y5 is a moiety bonded to L.

According to an exemplary embodiment of the present specification, in Chemical Formula A-2, Y7 is a moiety bonded to L.

According to an exemplary embodiment of the present specification, in Chemical Formula A-2, Y8 is a moiety bonded to L.

According to an exemplary embodiment of the present specification, in Chemical Formula A-2, Y9 is a moiety bonded to L.

According to an exemplary embodiment of the present specification, in Chemical Formula A-2, Y10 is a moiety bonded to L.

According to an exemplary embodiment of the present specification, in Chemical Formula A-3, Y1 is a moiety bonded to L.

According to an exemplary embodiment of the present specification, in Chemical Formula A-3, Y2 is a moiety bonded to L.

According to an exemplary embodiment of the present specification, in Chemical Formula A-3, Y5 is a moiety bonded to L.

According to an exemplary embodiment of the present specification, in Chemical Formula A-3, Y6 is a moiety bonded to L.

According to an exemplary embodiment of the present specification, in Chemical Formula A-3, Y7 is a moiety bonded to L.

According to an exemplary embodiment of the present specification, in Chemical Formula A-3, Y8 is a moiety bonded to L.

According to an exemplary embodiment of the present specification, in Chemical Formula A-3, Y9 is a moiety bonded to L.

According to an exemplary embodiment of the present specification, in Chemical Formula A-3, Y10 is a moiety bonded to L.

According to an exemplary embodiment of the present specification, Chemical Formula 1 is any one of the following Chemical Formulae 1-1 to 1-3.

[Chemical Formula 1-1]

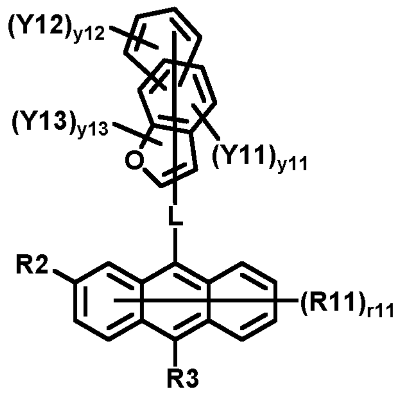

[Chemical Formula 1-2]

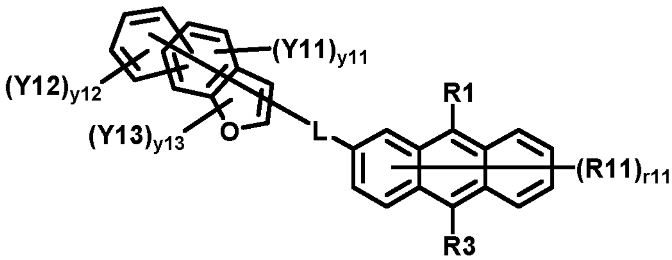

[Chemical Formula 1-3]

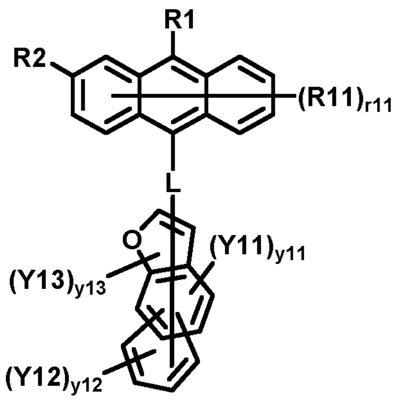

In Chemical Formulae 1-1 to 1-3,

the definitions of R1 to R3, R11, r11 and L are the same as the definitions in Chemical Formula 1,

any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,

y11 and y13 are each 1 or 2,

y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

According to an exemplary embodiment of the present specification, Chemical Formula 1 is any one of the following Chemical Formulae 1-4 to 1-6.

[Chemical Formula 1-4]

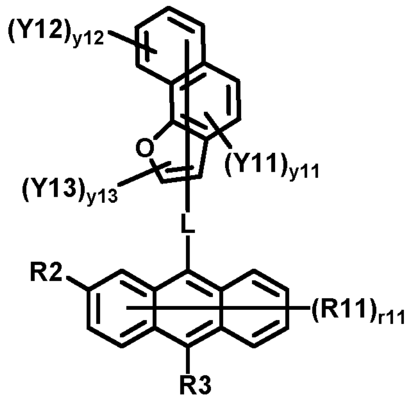

[Chemical Formula 1-5]

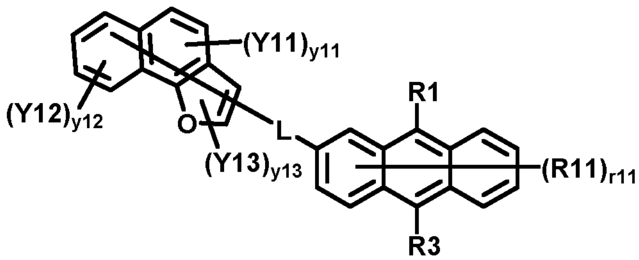

[Chemical Formula 1-6]

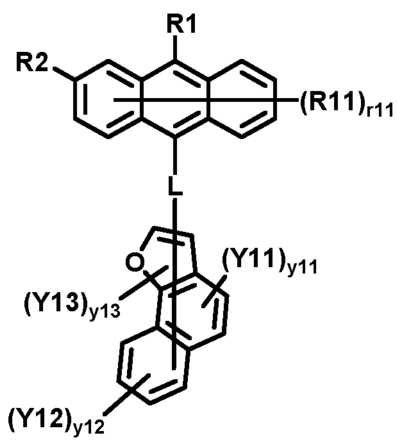

In Chemical Formulae 1-4 to 1-6,

the definitions of R1 to R3, R11, r11 and L are the same as the definitions in Chemical Formula 1,
any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
y11 and y13 are each 1 or 2,
y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

According to an exemplary embodiment of the present specification, Chemical Formula 1 is any one of the following Chemical Formulae 1-7 to 1-9.

[Chemical Formula 1-7]

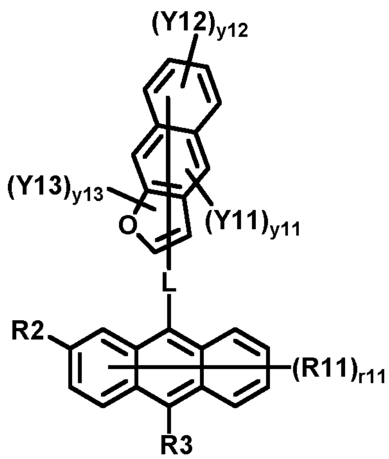

[Chemical Formula 1-8]

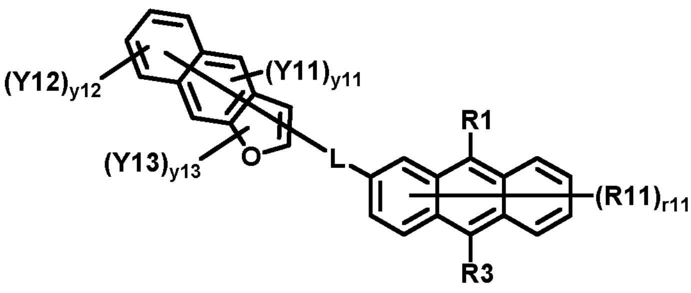

[Chemical Formula 1-9]

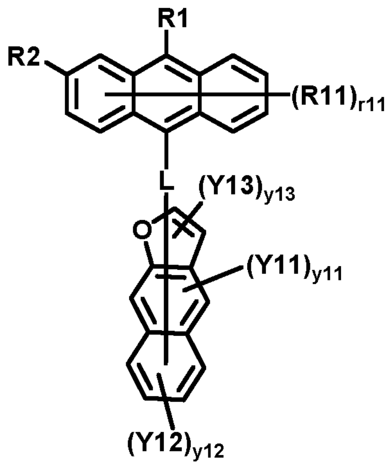

In Chemical Formulae 1-7 to 1-9,
the definitions of R1 to R3, R11, r11 and L are the same as the definitions in Chemical Formula 1,

any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
y11 and y13 are each 1 or 2,
y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

According to an exemplary embodiment of the present specification, Chemical Formula 1 is any one of the following Chemical Formulae 1-10 to 1-12.

[Chemical Formula 1-10]

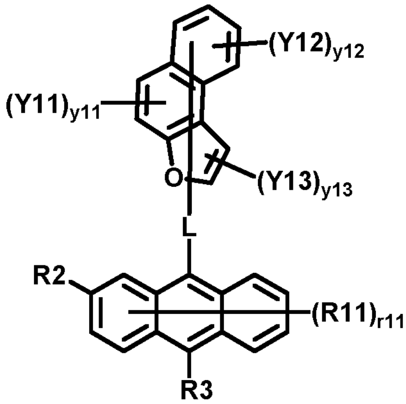

[Chemical Formula 1-11]

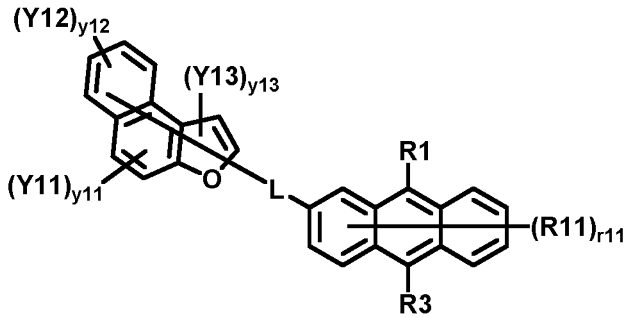

[Chemical Formula 1-12]

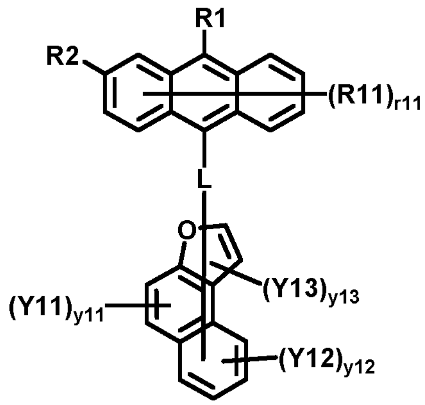

In Chemical Formulae 1-10 to 1-12,

the definitions of R1 to R3, R11, r11 and L are the same as the definitions in Chemical Formula 1,
any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
y11 and y13 are each 1 or 2,
y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

According to an exemplary embodiment of the present specification, at least one of R1 to R3 is -L-Arl, at least one of the rest of R1 to R3, which are not -L-Arl, is a substituted or unsubstituted monocyclic or polycyclic heteroaryl group having 2 to 30 carbon atoms, and the other and R11 are the same as or different from each other, and are each independently hydrogen; deuterium; or a substituted or unsubstituted monocyclic or polycyclic aryl group having 6 to 30 carbon atoms, and
L is a direct bond; a substituted or unsubstituted monocyclic or polycyclic arylene group having 6 to 30 carbon atoms;

or a substituted or unsubstituted monocyclic or polycyclic heteroarylene group having 2 to 30 carbon atoms.

According to an exemplary embodiment of the present specification, at least one of R1 to R3 is -L-Arl, at least one of the rest of R1 to R3, which are not -L-Arl, is a substituted or unsubstituted monocyclic or polycyclic heteroaryl group having 2 to 20 carbon atoms, and the other and R11 are the same as or different from each other, and are each independently hydrogen; deuterium; or a substituted or unsubstituted monocyclic or polycyclic aryl group having 6 to 20 carbon atoms, and

L is a direct bond; a substituted or unsubstituted monocyclic or polycyclic arylene group having 6 to 20 carbon atoms; or a substituted or unsubstituted monocyclic or polycyclic heteroarylene group having 2 to 20 carbon atoms.

According to an exemplary embodiment of the present specification, at least one of R1 to R3 is -L-Arl, at least one of the rest of R1 to R3, which are not -L-Arl, is a monocyclic or polycyclic heteroaryl group having 2 to 30 carbon atoms, which is unsubstituted or substituted with deuterium or a monocyclic or polycyclic aryl group having 6 to 30 carbon atoms, and the other and R11 are the same as or different from each other, and are each independently hydrogen; deuterium; or a monocyclic or polycyclic aryl group having 6 to 30 carbon atoms, which is unsubstituted or substituted with deuterium or a monocyclic or polycyclic aryl group having 6 to 30 carbon atoms, and

L is a direct bond; a monocyclic or polycyclic arylene group having 6 to 30 carbon atoms; or a monocyclic or polycyclic heteroarylene group having 2 to 30 carbon atoms.

According to an exemplary embodiment of the present specification, at least one of R1 to R3 is -L-Arl, at least one of the rest of R1 to R3, which are not -L-Arl, is a monocyclic or polycyclic heteroaryl group having 2 to 20 carbon atoms, which is unsubstituted or substituted with deuterium or a monocyclic or polycyclic aryl group having 6 to 20 carbon atoms, and the other and R11 are the same as or different from each other, and are each independently hydrogen; deuterium; or a monocyclic or polycyclic aryl group having 6 to 20 carbon atoms, which is unsubstituted or substituted with deuterium or a monocyclic or polycyclic aryl group having 6 to 20 carbon atoms, and

L is a direct bond; a monocyclic or polycyclic arylene group having 6 to 20 carbon atoms; or a monocyclic or polycyclic heteroarylene group having 2 to 20 carbon atoms.

According to an exemplary embodiment of the present specification, at least one of R1 to R3 is -L-Arl, at least one of the rest of R1 to R3, which are not -L-Arl, is a dibenzofuran group, which is unsubstituted or substituted with deuterium or a monocyclic or polycyclic aryl group having 6 to 30 carbon atoms; a naphthobenzofuran group which is unsubstituted or substituted with deuterium; a carbazole group which is unsubstituted or substituted with a monocyclic or polycyclic aryl group having 6 to 30 carbon atoms; a dibenzothiophene group which is unsubstituted or substituted with deuterium; a benzofuropyrimidine group; a benzoxazole group which is unsubstituted or substituted with a monocyclic or polycyclic aryl group having 6 to 30 carbon atoms; or a naphthobenzothiophene group, and

L is a direct bond; a phenylene group; a naphthylene group; or a divalent dibenzofuran group.

According to an exemplary embodiment of the present specification, at least one of R1 to R3 is -L-Arl, at least one of the rest of R1 to R3, which are not -L-Arl, is a dibenzofuran group, which is unsubstituted or substituted with deuterium or a monocyclic or polycyclic aryl group having 6 to 20 carbon atoms; a naphthobenzofuran group which is unsubstituted or substituted with deuterium; a carbazole group which is unsubstituted or substituted with a monocyclic or polycyclic aryl group having 6 to 20 carbon atoms; a dibenzothiophene group which is unsubstituted or substituted with deuterium; a benzofuropyrimidine group; a benzoxazole group which is unsubstituted or substituted with a monocyclic or polycyclic aryl group having 6 to 20 carbon atoms; or a naphthobenzothiophene group, and

L is a direct bond; a phenylene group; a naphthylene group; or a divalent dibenzofuran group.

According to an exemplary embodiment of the present specification, at least one of R1 to R3 is -L-Arl, at least one of the rest of R1 to R3, which are not -L-Arl, is a dibenzofuran group, which is unsubstituted or substituted with deuterium, a phenyl group or a naphthyl group; a naphthobenzofuran group which is unsubstituted or substituted with deuterium; a carbazole group which is unsubstituted or substituted with a phenyl group; a dibenzothiophene group which is unsubstituted or substituted with deuterium; a benzofuropyrimidine group; a benzoxazole group which is unsubstituted or substituted with a phenyl group or a naphthyl group; or a naphthobenzothiophene group, and

L is a direct bond; a phenylene group; a naphthylene group; or a divalent dibenzofuran group.

According to an exemplary embodiment of the present specification, any one of Y1, Y2 and Y7 to Y11 is a moiety bonded to L, and the others of Y1, Y2 and Y7 to Y11, which are not bonded to L, are the same as or different from each other, and are each independently hydrogen; deuterium; a substituted or unsubstituted straight-chained or branched alkyl group having 1 to 30 carbon atoms; a substituted or unsubstituted monocyclic or polycyclic cycloalkyl group having 3 to 30 carbon atoms; a substituted or unsubstituted monocyclic or polycyclic aryl group having 6 to 30 carbon atoms; a fused ring group of a monocyclic or polycyclic aromatic hydrocarbon ring having 6 to 30 carbon atoms and a monocyclic or polycyclic aliphatic hydrocarbon ring having 3 to 30 carbon atoms, which is substituted or unsubstituted; or a substituted or unsubstituted monocyclic or polycyclic heteroaryl group having 2 to 30 carbon atoms, or any one or more adjacent pairs are bonded to each other to form a substituted or unsubstituted aliphatic hydrocarbon ring having 3 to 30 carbon atoms.

According to an exemplary embodiment of the present specification, any one of Y1, Y2 and Y7 to Y11 is a

moiety bonded to L, and the others of Y1, Y2 and Y7 to Y11, which are not bonded to L, are the same as or different from each other, and are each independently hydrogen; deuterium; a substituted or unsubstituted straight-chained or branched alkyl group having 1 to 20 carbon atoms; a substituted or unsubstituted monocyclic or polycyclic cycloalkyl group having 3 to 20 carbon atoms; a substituted or unsubstituted monocyclic or polycyclic aryl group having 6 to 20 carbon atoms; a fused ring group of a monocyclic or polycyclic aromatic hydrocarbon ring having 6 to 20 carbon atoms and a monocyclic or polycyclic aliphatic hydrocarbon ring having 3 to 20 carbon atoms, which is substituted or unsubstituted; or a substituted or unsubstituted monocyclic or polycyclic heteroaryl group having 2 to 20 carbon atoms, or any one or more adjacent pairs are bonded to each other to form a substituted or unsubstituted aliphatic hydrocarbon ring having 3 to 20 carbon atoms.

According to an exemplary embodiment of the present specification, any one of Y1, Y2 and Y7 to Y11 is a moiety bonded to L, and the others of Y1, Y2 and Y7 to Y11, which are not bonded to L, are the same as or different from each other, and are each independently hydrogen; deuterium; a straight-chained or branched alkyl group having 1 to 30 carbon atoms; a monocyclic or polycyclic cycloalkyl group having 3 to 30 carbon atoms; a monocyclic or polycyclic aryl group having 6 to 30 carbon atoms, which is unsubstituted or substituted with deuterium or a straight-chained or branched alkyl group having 1 to 30 carbon atoms; a fused ring group of a monocyclic or polycyclic aromatic hydrocarbon ring having 6 to 30 carbon atoms and a monocyclic or polycyclic aliphatic hydrocarbon ring having 3 to 30 carbon atoms, which is unsubstituted or substituted with a straight-chained or branched alkyl group having 1 to 30 carbon atoms; or a monocyclic or polycyclic heteroaryl group having 2 to 30 carbon atoms, or any one or more adjacent pairs are bonded to each other to form an aliphatic hydrocarbon ring having 3 to 30 carbon atoms, which is unsubstituted or substituted with a monocyclic or polycyclic alkyl group having 1 to 30 carbon atoms.

According to an exemplary embodiment of the present specification, any one of Y1, Y2 and Y7 to Y11 is a moiety bonded to L, and the others of Y1, Y2 and Y7 to Y11, which are not bonded to L, are the same as or different from each other, and are each independently hydrogen; deuterium; a straight-chained or branched alkyl group having 1 to 20 carbon atoms; a monocyclic or polycyclic cycloalkyl group having 3 to 20 carbon atoms; a monocyclic or polycyclic aryl group having 6 to 20 carbon atoms, which is unsubstituted or substituted with deuterium or a straight-chained or branched alkyl group having 1 to 20 carbon atoms; a fused ring group of a monocyclic or polycyclic aromatic hydrocarbon ring having 6 to 20 carbon atoms and a monocyclic or polycyclic aliphatic hydrocarbon ring having 3 to 20 carbon atoms, which is unsubstituted or substituted with a straight-chained or branched alkyl group having 1 to 20 carbon atoms; or a monocyclic or polycyclic heteroaryl group having 2 to 20 carbon atoms, or any one or more adjacent pairs are bonded to each other to form an aliphatic hydrocarbon ring having 3 to 20 carbon atoms, which is unsubstituted or substituted with a monocyclic or polycyclic alkyl group having 1 to 20 carbon atoms.

According to an exemplary embodiment of the present specification, any one of Y1, Y2 and Y7 to Y11 is a moiety bonded to L, the others of Y1, Y2 and Y7 to Y11, which are not bonded to L, are the same as or different from each other, and are each independently hydrogen; deuterium; a methyl group; an isopropyl group; a tert-butyl group; a cyclopentyl group; a cyclohexyl group; an adamantyl group; a phenyl group which is unsubstituted or substituted with deuterium or a straight-chained or branched alkyl group having 1 to 30 carbon atoms; a biphenyl group; a naphthyl group; a tetrahydronaphthyl group which is unsubstituted or substituted with a straight-chained or branched alkyl group having 1 to 30 carbon atoms; or a benzothiophene group, or any one or more adjacent pairs are bonded to each other to form a cyclohexane which is unsubstituted or substituted with a straight-chained or branched alkyl group having 1 to 30 carbon atoms.

According to an exemplary embodiment of the present specification, any one of Y1, Y2 and Y7 to Y11 is a moiety bonded to L, the others of Y1, Y2 and Y7 to Y11, which are not bonded to L, are the same as or different from each other, and are each independently hydrogen; deuterium; a methyl group; an isopropyl group; a tert-butyl group; a cyclopentyl group; a cyclohexyl group; an adamantyl group; a phenyl group which is unsubstituted or substituted with deuterium or a straight-chained or branched alkyl group having 1 to 20 carbon atoms; a biphenyl group; a naphthyl group; a tetrahydronaphthyl group which is unsubstituted or substituted with a straight-chained or branched alkyl group having 1 to 20 carbon atoms; or a benzothiophene group, or any one or more adjacent pairs are bonded to each other to form a cyclohexane which is unsubstituted or substituted with a straight-chained or branched alkyl group having 1 to 20 carbon atoms.

According to an exemplary embodiment of the present specification, any one of Y1, Y2 and Y7 to Y11 is a moiety bonded to L, the others of Y1, Y2 and Y7 to Y11, which are not bonded to L, are the same as or different from each other, and are each independently hydrogen; deuterium; a methyl group; an isopropyl group; a tert-butyl group; a cyclopentyl group; a cyclohexyl group; an adamantyl group; a phenyl group which is unsubstituted or substituted with deuterium or an isopropyl group; a biphenyl group; a naphthyl group; a tetrahydronaphthyl group which is unsubstituted or substituted with a methyl group; or a benzothiophene group, or any one or more adjacent pairs are bonded to each other to form a cyclohexane which is unsubstituted or substituted with a methyl group.

According to an exemplary embodiment of the present specification, the definitions of Y1, Y2 and Y7 to Y11 may be applied to the definitions of Y3, Y4, Y5, Y6, Y12 and Y13.

According to an exemplary embodiment of the present specification, Chemical Formula 1 is any one selected from the following compounds.

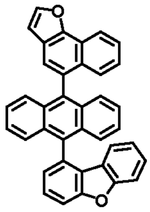
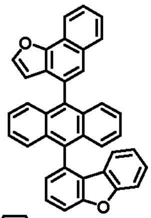
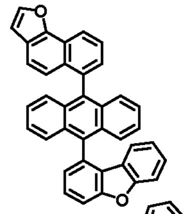
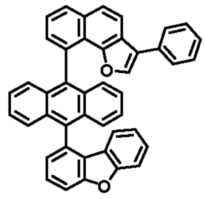
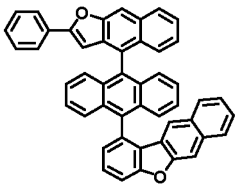
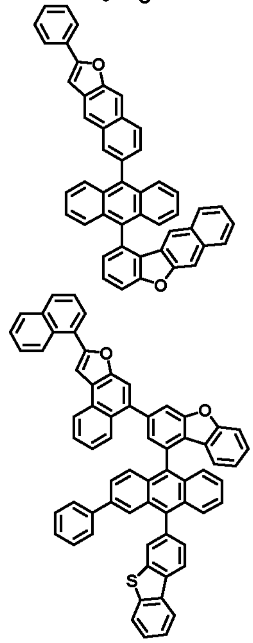
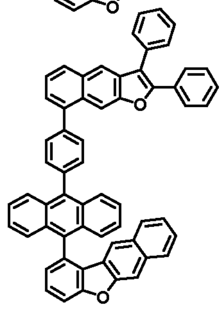
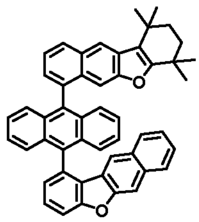
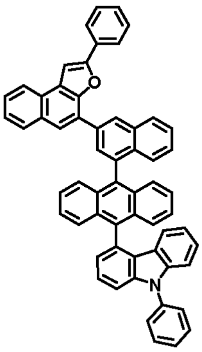
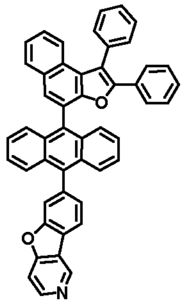
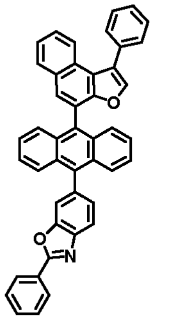
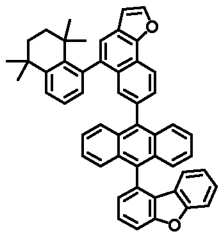
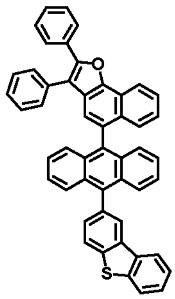
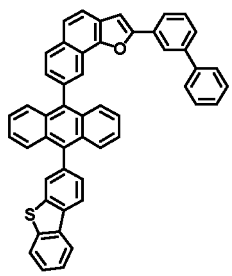
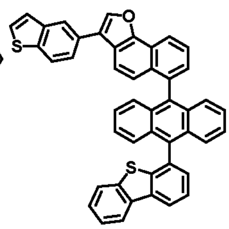
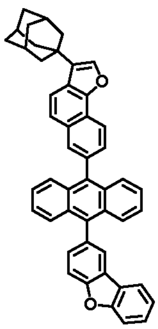
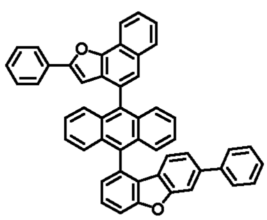
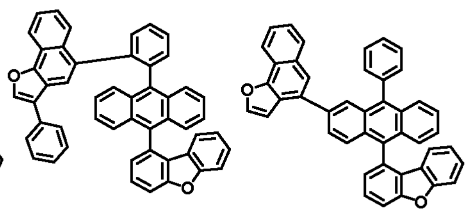

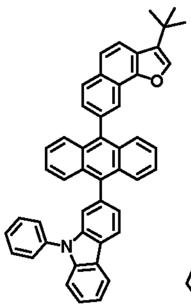
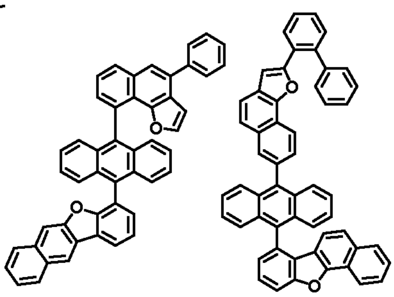
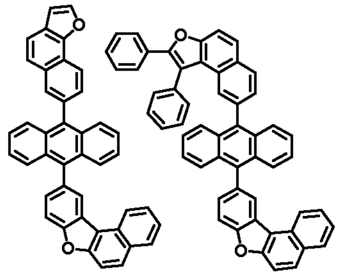

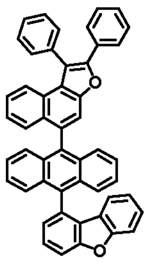
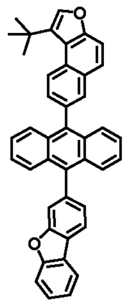
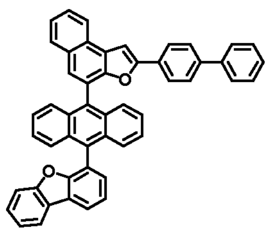
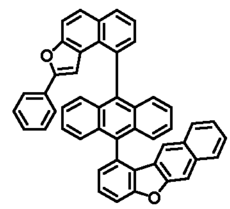

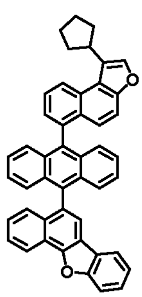
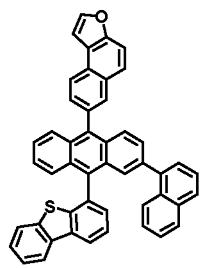
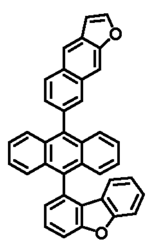
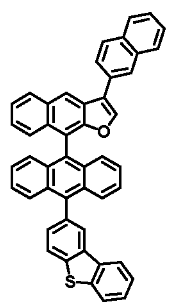

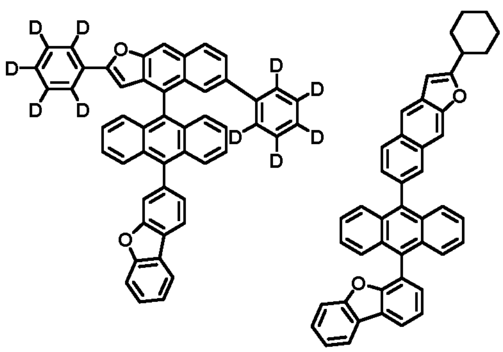
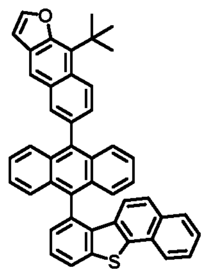
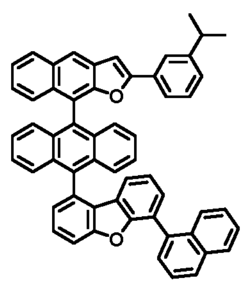

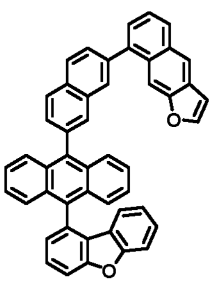
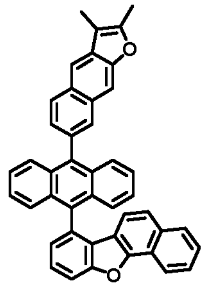
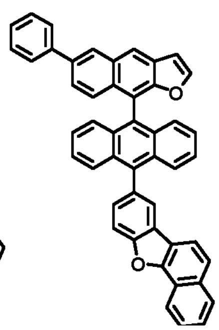
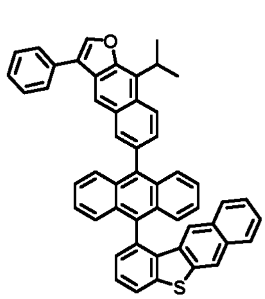

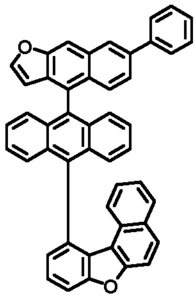

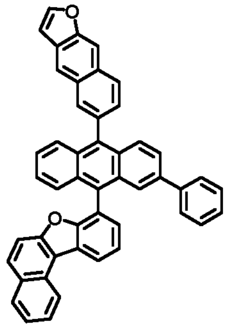

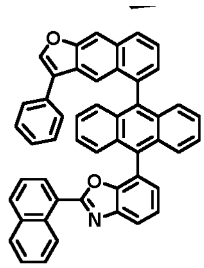

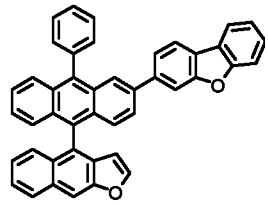

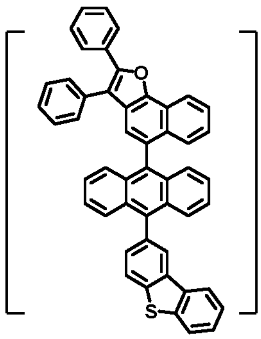

D26

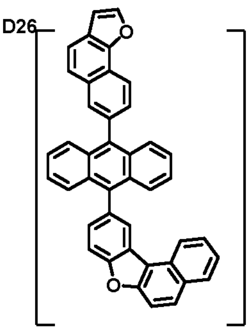

D22

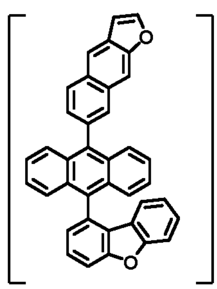

D21

The present specification provides an organic light emitting device including the above-described compound.

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

In the present specification, the ‘layer’ has a meaning compatible with a ‘film’ usually used in the art, and means a coating covering a target region. The size of the ‘layer’ is not limited, and the sizes of the respective ’layers’ may be the same as or different from one another. According to an exemplary embodiment, the size of the ‘layer’ may be the same as that of the entire device, may correspond to the size of a specific functional region, and may also be as small as a single sub-pixel.

In the present specification, when a specific A material is included in a B layer, this means both i) the fact that one or more A materials are included in one B layer and ii) the fact that the B layer is composed of one or more layers, and the A material is included in one or more layers of the multi-layered B layer.

In the present specification, when a specific A material is included in a C layer or a D layer, this means all of i) the fact that the A material is included in one or more layers of the C layer having one or more layers, ii) the fact that the A material is included in one or more layers of the D layer having one or more layers, and iii) the fact that the A material is included in each of the C layer having one or more layers and the D layer having one or more layers.

The present specification provides an organic light emitting device including: a first electrode; a second electrode provided to face the first electrode; and an organic material layer having one or more layers provided between the first electrode and the second electrode, in which one or more layers of the organic material layer include the compound represented by Chemical Formula 1.

The organic material layer of the organic light emitting device of the present specification may have a single-layered structure, but may also have a multi-layered structure in which two or more organic material layers are stacked. For example, the organic material layer may have a structure including a hole injection layer, a hole transport layer, a light emitting layer, an electron transport layer, an electron injection layer, an electron blocking layer, a hole blocking layer, and the like. However, the structure of the organic light emitting device is not limited thereto, and may include a fewer number of organic layers.

According to an exemplary embodiment of the present specification, the organic material layer includes a light emitting layer, and the light emitting layer includes the compound.

According to an exemplary embodiment of the present specification, the organic material layer includes a light emitting layer, and the light emitting layer includes the compound as a host of the light emitting layer.

According to an exemplary embodiment of the present specification, the light emitting layer includes a dopant,

and the dopant includes a fluorescent dopant.

According to an exemplary embodiment of the present specification, the fluorescent dopant is a pyrene-based compound or a non-pyrene-based compound.

According to an exemplary embodiment of the present specification, the non-pyrene-based compound includes a boron-based compound.

According to an exemplary embodiment of the present specification, the light emitting layer further includes one or more hosts different from the compound of Chemical Formula 1.

Any anthracene-based host used in the art can be used without limitation as long as the host different from the compound of Chemical Formula 1 is different from Chemical Formula 1, and the host is not limited thereto.

According to an exemplary embodiment of the present specification, the light emitting layer includes a host and a dopant.

According to an exemplary embodiment of the present specification, the light emitting layer includes a host and a dopant, and the host includes the compound represented by Chemical Formula 1.

According to an exemplary embodiment of the present specification, the dopant is a blue dopant.

According to an exemplary embodiment of the present specification, the organic light emitting device is a blue organic light emitting device.

According to an exemplary embodiment of the present specification, the light emitting layer includes two or more mixed hosts, and one or more of the two or more mixed hosts include the compound represented by Chemical Formula 1.

According to an exemplary embodiment of the present specification, the light emitting layer includes two or more mixed hosts, at least one of the two or more mixed hosts includes the compound represented by Chemical Formula 1, and the others include an anthracene-based compound different from Chemical Formula 1.

At least one of the two or more mixed hosts includes the compound represented by Chemical Formula 1, and an anthracene-based host used in the art can be used without limitation as long as the others are different from Chemical Formula 1, and the mixed hosts are not limited thereto.

An organic light emitting device using two or more mixed hosts according to an exemplary embodiment of the present specification is intended to improve the performance of the device by combining the advantages of each host, and for example, when two types of hosts are mixed, one type of host with high efficiency and low voltage effects and one type of host with long service life effects may be mixed to manufacture an organic light emitting device having the effects of high efficiency, low voltage and long service life.

According to an exemplary embodiment of the present specification, the organic light emitting device has a maximum emission wavelength ($\lambda_{max}$) of 400 nm to 470 nm in an emission spectrum.

According to an exemplary embodiment of the present specification, the light emitting layer includes a host and a dopant, and the dopant is a fluorescent dopant.

According to an exemplary embodiment of the present specification, the light emitting layer includes a host and a dopant, and the dopant includes one or more selected from a pyrene-based compound and a non-pyrene-based compound.

The pyrene-based compound and the non-pyrene-based compounds can be used without limitation as long as they are compounds used in the art, and are not limited thereto.

According to an exemplary embodiment of the present specification, the non-pyrene-based compound includes a boron-based compound.

According to an exemplary embodiment of the present specification, the light emitting layer includes a host and a dopant, the host includes the compound represented by Chemical Formula 1, and the dopant includes one or more selected from a pyrene-based compound and a non-pyrene-based compound.

According to an exemplary embodiment of the present specification, the light emitting layer includes the host and the dopant at a weight ratio of 0.1:99.9 to 20:80.

According to another exemplary embodiment of the present specification, the light emitting layer further includes a capping layer provided on a surface of at least one of the first electrode and the second electrode opposite to the surface facing the organic material layer.

The capping layer is formed to prevent a considerable amount of light from being lost through total reflection of light in the organic light emitting device, and the capping layer has the performance capable of sufficiently protecting the underlying negative electrode and light emitting layer from external moisture penetration or contamination, and has a high refractive index, and thus may prevent light loss caused by total reflection.

According to still another exemplary embodiment of the present specification, the capping layer may be provided on each of a surface of the first electrode opposite to the surface facing the organic layer and a surface of the second electrode opposite to the surface facing the organic layer.

According to yet another exemplary embodiment of the present specification, the capping layer may be provided on a surface of the first electrode opposite to the surface facing the organic layer.

According to still yet another exemplary embodiment of the present specification, the capping layer may be provided on a surface of the second electrode opposite to the surface facing the organic layer.

According to an exemplary embodiment of the present specification, the organic light emitting device further includes one or two or more layers selected from the group consisting of a hole injection layer, a hole transport layer, a light emitting layer, an electron transport layer, an electron injection layer, a hole blocking layer, and an electron blocking layer.

According to an exemplary embodiment of the present specification, the organic light emitting device includes: a first electrode; a second electrode provided to face the first electrode; a light emitting layer provided between the first electrode and the second electrode; and an organic material layer having two or more layers provided between the light emitting layer and the first electrode, or between the light emitting layer and the second electrode.

According to an exemplary embodiment of the present specification, as the organic material layer having two or more layers between the light emitting layer and the first electrode, or between the light emitting layer and the second electrode, two or more may be selected from the group consisting of a light emitting layer, a hole transport layer, a hole injection layer, a hole injection and transport layer, an electron blocking layer, a hole blocking layer, an electron injection layer, an electron transport layer, and an electron injection and transport layer.

According to an exemplary embodiment of the present specification, a hole transport layer having two or more layers is included between the light emitting layer and the first electrode. The hole transport layer having two or more layers may include materials which are the same as or different from each other.

According to an exemplary embodiment of the present specification, the first electrode is an anode or a cathode. According to an exemplary embodiment of the present specification, the second electrode is a cathode or an anode.

According to an exemplary embodiment of the present specification, the organic light emitting device may be a normal type organic light emitting device in which an anode, an organic material layer having one or more layers, and a cathode are sequentially stacked on a substrate.

According to an exemplary embodiment of the present specification, the organic light emitting device may be an inverted type organic light emitting device in which a cathode, an organic material layer having one or more layers, and an anode are sequentially stacked on a substrate.

For example, the structure of the organic light emitting device according to an exemplary embodiment of the present specification is exemplified in FIGS. 1 and 2. FIGS. 1 and 2 exemplify an organic light emitting device, and the organic light emitting device is not limited thereto.

FIG. 1 exemplifies a structure of an organic light emitting device in which a first electrode 2, an organic material layer 4, and a second electrode 3 are sequentially stacked on a substrate 1. The compound is included in the organic material layer.

FIG. 2 exemplifies the structure of an organic light emitting device in which a first electrode 2, a hole injection layer 5, a hole transport layer 6, a hole adjusting layer 7, a light emitting layer 4, an electron adjusting layer 8, an electron transport layer 9, an electron injection layer 10, a second electrode 3, and a capping layer 11 are sequentially stacked on a substrate 1. The compound is included in the light emitting layer.

The organic light emitting device of the present specification may be manufactured by the materials and methods known in the art, except that the light emitting layer includes the compound, that is, the compound represented by Chemical Formula 1.

When the organic light emitting device includes a plurality of organic material layers, the organic material layers may be formed of the same material or different materials.

For example, the organic light emitting device of the present specification may be manufactured by sequentially stacking a first electrode, an organic material layer, and a second electrode on a substrate. In this case, the organic light emitting device may be manufactured by depositing a metal or a metal oxide having conductivity, or an alloy thereof on a substrate to form an anode, forming an organic material layer including a hole injection layer, a hole transport layer, a light emitting layer, and an electron transport layer thereon, and then depositing a material, which may be used as a cathode, thereon, by using a physical vapor deposition (PVD) method such as sputtering or e-beam evaporation. In addition to the method described above, an organic light emitting device may be manufactured by sequentially depositing a second electrode material, an organic material layer, and a first electrode material on a substrate.

Further, the compound represented by Chemical Formula 1 may be formed as an organic material layer by not only a vacuum deposition method, but also a solution application method when an organic light emitting device is manufactured. Here, the solution application method means spin coating, dip coating, doctor blading, inkjet printing, screen printing, a spray method, roll coating, and the like, but is not limited thereto.

In addition to the method described above, an organic light emitting device may be made by sequentially depositing a second electrode material, an organic material layer, and a first electrode material on a substrate. However, the manufacturing method is not limited thereto.

As the first electrode material, materials having a high work function are usually preferred so as to facilitate the injection of holes into an organic material layer. Examples thereof include: a metal, such as vanadium, chromium, copper,

zinc, and gold, or an alloy thereof; a metal oxide, such as zinc oxide, indium oxide, indium tin oxide (ITO), and indium zinc oxide (IZO); a combination of a metal and an oxide, such as ZnO:Al or $SnO_2$:Sb; a conductive polymer, such as poly(3-methylthiophene), poly[3,4-(ethylene-1,2-dioxy)thiophene] (PEDOT), polypyrrole, and polyaniline; and the like, but are not limited thereto.

As the second electrode material, materials having a low work function are usually preferred so as to facilitate the injection of electrons into an organic material layer. Examples thereof include: a metal, such as magnesium, calcium, sodium, potassium, titanium, indium, yttrium, lithium, gadolinium, aluminum, silver, tin, and lead, or an alloy thereof; a multi-layer structured material, such as LiF/Al or $LiO_2$/Al; and the like, but are not limited thereto.

The light emitting layer may include a host material and a dopant material. When an additional light emitting layer is included in addition to a light emitting layer including the compound of Chemical Formula 1 according to an exemplary embodiment of the present specification, examples of a host material include a fused and/or non-fused aromatic ring derivative, a hetero ring-containing compound, or the like. Specific examples of the fused aromatic ring derivative include anthracene derivatives, pyrene derivatives, naphthalene derivatives, pentacene derivatives, phenanthrene compounds, fluoranthene compounds, and the like, and specific examples of the hetero ring-containing compound include dibenzofuran derivatives, ladder-type furan compounds, pyrimidine derivatives, and the like, but the examples are not limited thereto.

According to an exemplary embodiment of the present specification, the host includes a compound represented by the following Chemical Formula H-1, but is not limited thereto.

[Chemical Formula H-1]

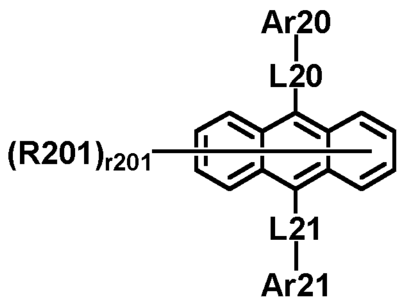

In Chemical Formula H-1,

L20 and L21 are the same as or different from each other, and each independently a direct bond; a substituted or unsubstituted arylene group; or a substituted or unsubstituted divalent heterocyclic group,

Ar20 and Ar21 are the same as or different from each other, and are each independently hydrogen; deuterium; a substituted or unsubstituted aryl group; or a substituted or unsubstituted heterocyclic group,

R201 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and

r201 is an integer from 1 to 8, and when r201 is 2 or higher, two or more R201's are the same as or different from each other.

In an exemplary embodiment of the present specification, L20 and L21 are the same as or different from each other, and are each independently a direct bond; a monocyclic or polycyclic arylene group having 6 to 30 carbon atoms; or a monocyclic or polycyclic divalent heterocyclic group having 2 to 30 carbon atoms.

In an exemplary embodiment of the present specification, L20 and L21 are the same as or different from each other, and are each independently a direct bond; a phenylene group which is unsubstituted or substituted with deuterium; a biphenylylene group which is unsubstituted or substituted with deuterium; a naphthylene group which is unsubstituted or substituted with deuterium; a divalent dibenzofuran group; or a divalent dibenzothiophene group.

In an exemplary embodiment of the present specification, Ar20 and Ar21 are the same as or different from each other, and are each independently a substituted or unsubstituted monocyclic or polycyclic aryl group having 6 to 30 carbon atoms; or a substituted or unsubstituted monocyclic or polycyclic heterocyclic group having 2 to 30 carbon atoms.

In an exemplary embodiment of the present specification, Ar20 and Ar21 are the same as or different from each other, and are each independently a substituted or unsubstituted monocyclic to tetracyclic aryl group having 6 to

20 carbon atoms; or a substituted or unsubstituted monocyclic to tetracyclic heterocyclic group having 6 to 20 carbon atoms.

In an exemplary embodiment of the present specification, Ar20 and Ar21 are the same as or different from each other, and are each independently a phenyl group which is unsubstituted or substituted with deuterium or a monocyclic or polycyclic aryl group having 6 to 20 carbon atoms; a biphenyl group which is unsubstituted or substituted with deuterium or a monocyclic or polycyclic aryl group having 6 to 20 carbon atoms; a naphthyl group which is unsubstituted or substituted with a monocyclic or polycyclic aryl group having 6 to 20 carbon atoms; a thiophene group which is unsubstituted or substituted with a monocyclic or polycyclic aryl group having 6 to 30 carbon atoms; a dibenzofuran group which is unsubstituted or substituted with a monocyclic or polycyclic aryl group having 6 to 20 carbon atoms; a naphthobenzofuran group which is unsubstituted or substituted with a monocyclic or polycyclic aryl group having 6 to 20 carbon atoms; a dibenzothiophene group which is unsubstituted or substituted with a monocyclic or polycyclic aryl group having 6 to 20 carbon atoms; or a naphthobenzothiophene group which is unsubstituted or substituted with a monocyclic or polycyclic aryl group having 6 to 20 carbon atoms.

In an exemplary embodiment of the present specification, Ar20 and Ar21 are the same as or different from each other, and are each independently a phenyl group which is unsubstituted or substituted with deuterium; a biphenyl group which is unsubstituted or substituted with deuterium; a terphenyl group; a naphthyl group which is unsubstituted or substituted with deuterium; a thiophene group which is unsubstituted or substituted with a phenyl group; a phenanthrene group; a dibenzofuran group; a naphthobenzofuran group; a dibenzothiophene group; or a naphthobenzothiophene group.

In an exemplary embodiment of the present specification, Ar20 and Ar21 are the same as or different from each other, and each independently a substituted or unsubstituted aryl group.

In an exemplary embodiment of the present specification, Ar20 and Ar21 are the same as or different from each other, and are each independently a phenyl group which is unsubstituted or substituted with deuterium; a biphenyl group which is unsubstituted or substituted with deuterium; a terphenyl group; or a naphthyl group which is unsubstituted or substituted with deuterium.

According to an exemplary embodiment of the present specification, R201 is hydrogen.

According to an exemplary embodiment of the present specification, Chemical Formula H-1 is represented by the following compound.

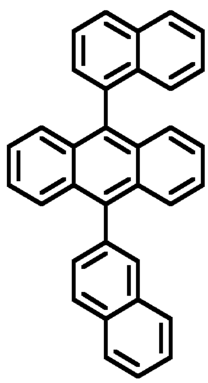

Examples of the dopant material include an aromatic amine derivative, a styrylamine compound, a boron complex, a fluoranthene compound, a metal complex, and the like. Specifically, the aromatic amine derivative is a fused aromatic ring derivative having a substituted or unsubstituted arylamine group, and examples thereof include pyrene, anthracene, chrysene, periflanthene, and the like having an arylamine group. Further, the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamine group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

According to an exemplary embodiment of the present specification, the dopant material includes a compound of the following Chemical Formula D-1 or D-2, but is not limited thereto.

[Chemical Formula D-1]

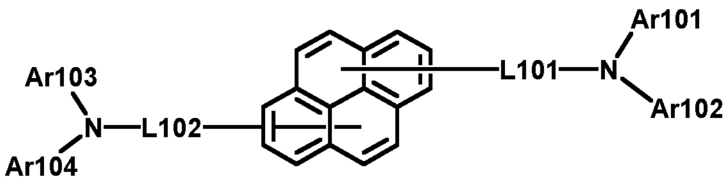

In Chemical Formula D-1,

L101 and L102 are the same as or different from each other, and are each independently a direct bond; or a substituted or unsubstituted arylene group, and
Ar101 to Ar104 are the same as or different from each other, and are each independently a substituted or unsubstituted aryl group; or a substituted or unsubstituted heteroaryl group,

[Chemical Formula D-2]

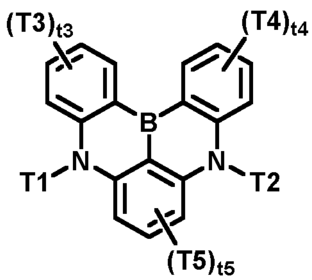

In Chemical Formula D-2,

T1 to T5 are the same as or different from each other, and are each independently hydrogen; a substituted or unsubstituted alkyl group; a substituted or unsubstituted amine group; or a substituted or unsubstituted aryl group,
t3 and t4 are each an integer from 1 to 4,
t5 is an integer from 1 to 3,
when t3 is 2 or higher, two or more T3's are the same as or different from each other,
when t4 is 2 or higher, two or more T4's are the same as or different from each other, and
when t5 is 2 or higher, two or more T5's are the same as or different from each other.

According to an exemplary embodiment of the present specification, L101 and L102 are a direct bond.
According to an exemplary embodiment of the present specification, Ar101 to Ar104 are the same as or different from each other, and are each independently a substituted or unsubstituted monocyclic or polycyclic aryl group having 6 to 30 carbon atoms; or a substituted or unsubstituted monocyclic or polycyclic heteroaryl group having 2 to 30 carbon atoms.
According to an exemplary embodiment of the present specification, Ar101 to Ar104 are the same as or different from each other, and are each independently a monocyclic or polycyclic aryl group having 6 to 30 carbon atoms, which is unsubstituted or substituted with a straight-chained or branched alkyl group having 1 to 30 carbon atoms; or a monocyclic or polycyclic heteroaryl group having 2 to 30 carbon atoms.
According to an exemplary embodiment of the present specification, Ar101 to Ar104 are the same as or different from each other, and are each independently a phenyl group substituted with a methyl group; or a dibenzofuran group.
According to an exemplary embodiment of the present specification, Chemical Formula D-1 is represented by the following compound.

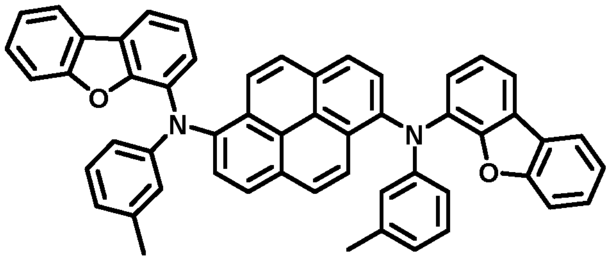

According to an exemplary embodiment of the present specification, T1 to T5 are the same as or different from each other, and are each independently hydrogen; a substituted or unsubstituted straight-chained or branched alkyl group having 1 to 30 carbon atoms; a substituted or unsubstituted monocyclic or polycyclic arylamine group having 6 to 30 carbon atoms; or a substituted or unsubstituted monocyclic or polycyclic aryl group having 6 to 30 carbon atoms.

According to an exemplary embodiment of the present specification, T1 to T5 are the same as or different from each other, and are each independently hydrogen; a straight-chained or branched alkyl group having 1 to 30 carbon atoms; a monocyclic or polycyclic arylamine group having 6 to 30 carbon atoms; or a monocyclic or polycyclic aryl group having 6 to 30 carbon atoms, which is unsubstituted or substituted with a straight-chained or branched alkyl group having 1 to 30 carbon atoms.

According to an exemplary embodiment of the present specification, T1 to T5 are the same as or different from each other, and are each independently hydrogen; a methyl group; a tert-butyl group; a diphenylamine group; or a phenyl group which is unsubstituted or substituted with a methyl group or tert-butyl group.

According to an exemplary embodiment of the present specification, Chemical Formula D-2 is represented by the following compound.

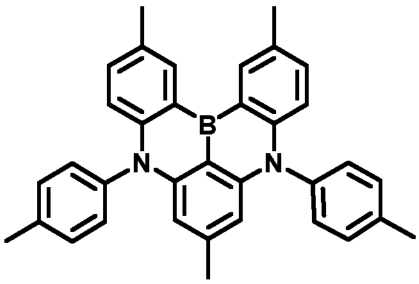

The hole injection layer is a layer which accepts holes from an electrode. It is preferred that hole injection material has an ability to transport holes, and has an effect of accepting holes from an anode and an excellent hole injection effect for a light emitting layer or a light emitting material. Further, the hole injection material is preferably a material which is excellent in ability to prevent excitons produced from a light emitting layer from moving to an electron injection layer or an electron injection material. In addition, the hole injection material is preferably a material which is excellent in ability to form a thin film. In addition, the highest occupied molecular orbital (HOMO) of the hole injection material is preferably a value between the work function of the anode material and the HOMO of the neighboring organic material layer. Specific examples of the hole injection material include: metal porphyrin, oligothiophene, and arylamine-based organic materials; hexanitrile hexaazatriphenylene-based organic materials; quinacridone-based organic materials; perylene-based organic materials; polythiophene-based conductive polymers such as anthraquinone and polyaniline; and the like, but are not limited thereto.

According to an exemplary embodiment of the present specification, the hole injection layer includes a compound of the following Chemical Formula HI-1, but is not limited thereto.

[Chemical Formula HI-1]

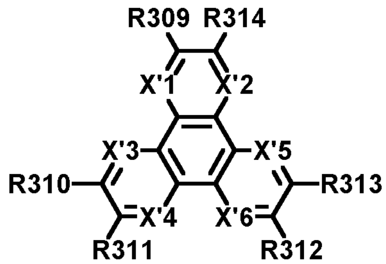

In Chemical Formula HI-1,

at least one of X'1 to X'6 is N, and the others are CH, and
R309 to R314 are the same as or different from each other, and are each independently hydrogen; deuterium; a cyano group; a substituted or unsubstituted alkyl group; a substituted or unsubstituted amine group; a substituted or unsubstituted aryl group; or a substituted or unsubstituted heteroaryl group, or are bonded to an adjacent group to form a substituted or unsubstituted ring.

According to an exemplary embodiment of the present specification, X'1 to X'6 are N.
According to an exemplary embodiment of the present specification, R309 to R314 are a cyano group.
According to an exemplary embodiment of the present specification, Chemical Formula HI-1 is represented by the following compound.

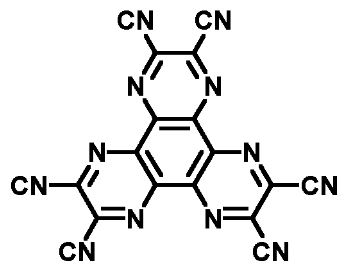

The hole transport layer is a layer which accepts holes from a hole injection layer and transports the holes to a light emitting layer. A hole transport material is preferably a material having high hole mobility which may accept holes from an anode or a hole injection layer and transfer the holes to a light emitting layer. Specific examples thereof include arylamine-based organic materials, conductive polymers, block copolymers having both conjugated portions and non-conjugated portions, and the like, but are not limited thereto.
According to an exemplary embodiment of the present specification, the hole transport layer or hole adjusting layer includes a compound of following Chemical Formula HT-1, but is not limited thereto.

[Chemical Formula HT-1]

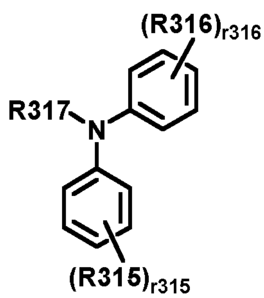

In Chemical Formula HT-1,

R315 to R317 are the same as or different from each other, and are each independently any one selected from the group consisting of hydrogen; deuterium; a substituted or unsubstituted alkyl group; a substituted or unsubstituted aryl group; a substituted or unsubstituted heteroaryl group; and a combination thereof, or are bonded to an adjacent group to form a substituted or unsubstituted ring,

r315 is an integer from 1 to 5, and when r315 is 2 or higher, two or more R315's are the same as or different from each other, and

r316 is an integer from 1 to 5, and when r316 is 2 or higher, two or more R316's are the same as or different from each other.

According to an exemplary embodiment of the present specification, R317 is any one selected from the group consisting of a substituted or unsubstituted aryl group; a substituted or unsubstituted heteroaryl group; and a combination thereof.

According to an exemplary embodiment of the present specification, R317 is any one selected from the group consisting of a carbazole group; a phenyl group; a biphenyl group; and a combination thereof.

According to an exemplary embodiment of the present specification, R315 and R316 are the same as or different from each other, and are each independently a substituted or unsubstituted aryl group, or are bonded to an adjacent group to form an aromatic hydrocarbon ring substituted with an alkyl group.

According to an exemplary embodiment of the present specification, R315 and R316 are the same as or different from each other, and are each independently a phenyl group or a phenanthrene group, or are bonded to an adjacent group to form indene substituted with a methyl group.

According to an exemplary embodiment of the present specification, Chemical Formula HT-1 is represented by any one of the following compounds.

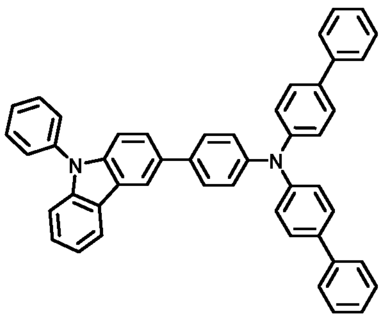

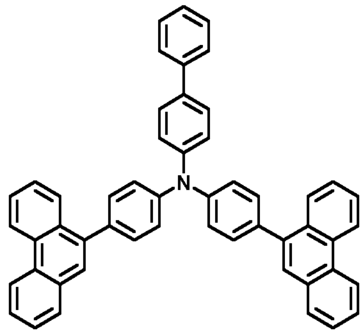

The electron adjusting layer is a layer that adjusts electrons transferred from the electron transport layer to be smoothly injected into the light emitting layer, and known materials can be used without limitation.

According to an exemplary embodiment of the present specification, the electron adjusting layer includes a compound of the following Chemical Formula EG-1, but is not limited thereto.

[Chemical Formula EG-1]

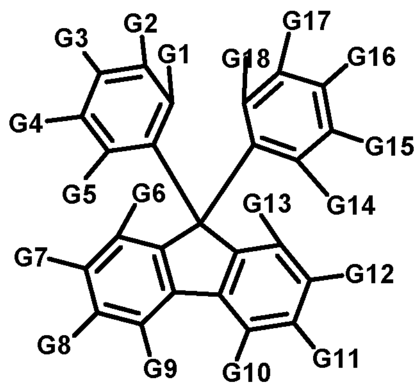

In Chemical Formula EG-1,

at least one of G1 to G18 is -L5-Ar5, the others are hydrogen, or G1 and G18 are linked through -L51- to form a substituted or unsubstituted ring,
L5 is a direct bond; or a substituted or unsubstituted arylene group,
Ar5 is a substituted or unsubstituted aryl group; or a substituted or unsubstituted heteroaryl group, and L51 is O; or S.

According to an exemplary embodiment of the present specification, L51 is O.
According to an exemplary embodiment of the present specification, L51 is S.
According to an exemplary embodiment of the present specification, G1 and G18 are linked through -L51- to form a substituted or unsubstituted hetero ring.
According to an exemplary embodiment of the present specification, G1 and G18 are linked through -L51- to form a substituted or unsubstituted xanthene ring; or a substituted or unsubstituted thioxanthene ring.
According to an exemplary embodiment of the present specification, G1 and G18 are linked through -O- to form a substituted or unsubstituted xanthene ring.
According to an exemplary embodiment of the present specification, G1 and G18 are linked through -S- to form a substituted or unsubstituted thioxanthene ring.
According to an exemplary embodiment of the present specification, G1 and G18 are linked through -O- to form a xanthene ring.
According to an exemplary embodiment of the present specification, G1 and G18 are linked through -S- to form a thioxanthene ring.
According to an exemplary embodiment of the present specification, L5 is a direct bond; or a substituted or unsubstituted monocyclic or polycyclic arylene group having 6 to 30 carbon atoms.
According to an exemplary embodiment of the present specification, L5 is a direct bond; or a substituted or unsubstituted monocyclic or polycyclic arylene group having 6 to 20 carbon atoms.
According to an exemplary embodiment of the present specification, L5 is a direct bond; or a monocyclic or polycyclic arylene group having 6 to 30 carbon atoms.
According to an exemplary embodiment of the present specification, L5 is a direct bond; or a monocyclic or polycyclic arylene group having 6 to 20 carbon atoms.
According to an exemplary embodiment of the present specification, L5 is a direct bond; or a phenylene group.
According to an exemplary embodiment of the present specification, Ar5 is a substituted or unsubstituted triazine group.
According to an exemplary embodiment of the present specification, Ar5 is a triazine group which is unsubstituted or substituted with a monocyclic or polycyclic aryl group having 6 to 30 carbon atoms.
According to an exemplary embodiment of the present specification, Ar5 is a triazine group substituted with a phenyl group.
According to an exemplary embodiment of the present specification, EG-1 is represented by the following compound.

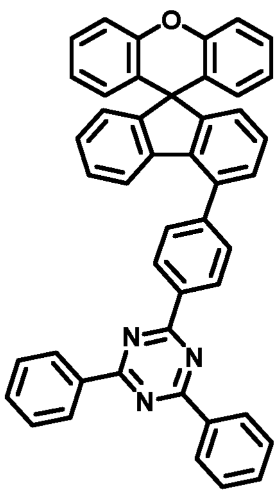

The electron transport layer is a layer which accepts electrons from an electron injection layer and transports the electrons to a light emitting layer. An electron transport material is preferably a material having high electron mobility which may proficiently accept electrons from a cathode and transfer the electrons to a light emitting layer. Specific examples thereof include: Al complexes of 8-hydroxyquinoline; complexes including $Alq_3$; organic radical compounds;

hydroxyflavone-metal complexes; and the like, but are not limited thereto. The electron transport layer may be used with any desired cathode material, as used according to the related art. In particular, an appropriate cathode material is a typical material which has a low work function, followed by an aluminum layer or a silver layer. Specific examples thereof include cesium, barium, calcium, ytterbium, and samarium, in each case followed by an aluminum layer or a silver layer.

According to an exemplary embodiment of the present specification, the electron transport layer includes a compound of the following Chemical Formula ET-1, but is not limited thereto.

[Chemical Formula ET-1]

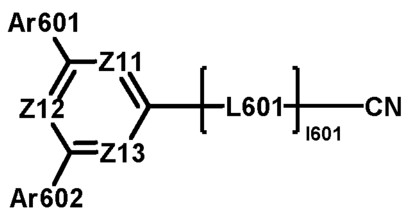

In Chemical Formula ET-1,

at least one of Z11 to Z13 is N, and the others are CH,
L601 is a direct bond; a substituted or unsubstituted arylene group; or a substituted or unsubstituted heteroarylene group,
Ar601 and Ar602 are the same as or different from each other, and are each independently a substituted or unsubstituted aryl group; or a substituted or unsubstituted heteroaryl group, and
l601 is an integer from 1 to 5, and when l601 is 2 or higher, two or more L601's are the same as or different from each other.

According to an exemplary embodiment of the present specification, L601 is a substituted or unsubstituted monocyclic or polycyclic arylene group having 6 to 30 carbon atoms.

According to an exemplary embodiment of the present specification, L601 is a phenylene group; a biphenylylene group; or a naphthylene group.

According to an exemplary embodiment of the present specification, Ar601 and Ar602 are the same as or different from each other, and are each independently a substituted or unsubstituted monocyclic or polycyclic aryl group having 6 to 30 carbon atoms.

According to an exemplary embodiment of the present specification, Ar601 and Ar602 are a phenyl group.

According to an exemplary embodiment of the present specification, Chemical Formula ET-1 is represented by the following compound.

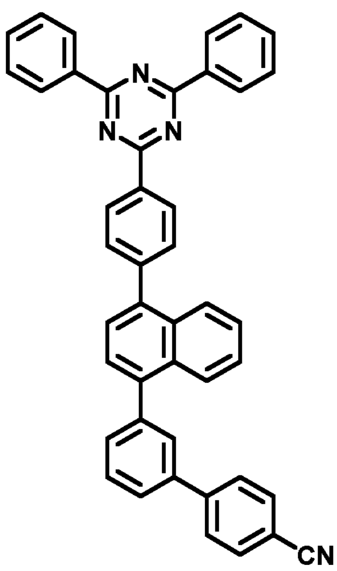

The electron injection layer is a layer which accepts electrons from an electrode. It is preferred that an electron

injection material is excellent in ability to transport electrons and has an effect of accepting electrons from the second electrode and an excellent electron injection effect for a light emitting layer or a light emitting material. Further, the electron injection material is preferably a material which prevents excitons produced from a light emitting layer from moving to a hole injection layer and is excellent in ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, metal complex compounds, nitrogen-containing 5-membered ring derivatives, and the like, but are not limited thereto.

Examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato)(o-cresolato) gallium, bis(2-methyl-8-quinolinato)(1-naphtholato) aluminum, bis(2-methyl-8-quinolinato)(2-naphtholato) gallium, and the like, but are not limited thereto.

According to an exemplary embodiment of the present specification, the electron injection and transport layer is a layer that transports electrons to the light emitting layer. For the electron injection and transport layer, materials exemplified for the electron transport layer and the electron injection layer may be used, but the materials are not limited thereto.

According to an exemplary embodiment of the present specification, the electron injection and transport layer may further include a metal complex compound. The metal complex compound is as described above.

The electron blocking layer is a layer which may improve the service life and efficiency of a device by preventing electrons injected from an electron injection layer from passing through a light emitting layer and entering a hole injection layer. As the electron blocking layer, the publicly-known material can be used without limitation, and the electron blocking layer may be formed between a light emitting layer and a hole injection layer, between a light emitting layer and a hole transport layer, or between a light emitting layer and a layer which simultaneously injects and transports holes.

As a material for the electron blocking layer, the examples of Chemical Formula HI-1 can be applied, but the material is not limited thereto.

The hole blocking layer is a layer which blocks holes from reaching a cathode, and may be generally formed under the same conditions as those of the electron injection layer. Specific examples thereof include oxadiazole derivatives or triazole derivatives, phenanthroline derivatives, aluminum complexes, and the like, but are not limited thereto.

The capping layer is formed to prevent a considerable amount of light from being lost through total reflection of light in the organic light emitting device, and the capping layer has the performance capable of sufficiently protecting the underlying negative electrode and light emitting layer from external moisture penetration or contamination, and has a high refractive index, and thus may prevent light loss caused by total reflection, and materials in the related art can be used without limitation.

According to an exemplary embodiment of the present specification, the capping layer includes a compound represented by the following Chemical Formula CP-1, but is not limited thereto.

[Chemical Formula CP-1]

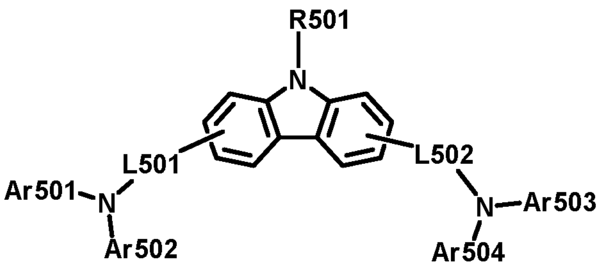

In Chemical Formula CP-1,

L501 and L502 are the same as or different from each other, and are each independently a direct bond; a substituted or unsubstituted arylene group; or a substituted or unsubstituted heteroarylene group, and

R501 and Ar501 to Ar504 are the same as or different from each other, and are each independently a substituted or unsubstituted aryl group; or a substituted or unsubstituted heteroaryl group, or adjacent groups are bonded to each other to form a substituted or unsubstituted ring.

According to an exemplary embodiment of the present specification, L501 and L502 are the same as or different

from each other, and are each independently a substituted or unsubstituted monocyclic or polycyclic arylene group having 6 to 30 carbon atoms.

According to an exemplary embodiment of the present specification, L501 and L502 are a phenylene group.

According to an exemplary embodiment of the present specification, R501 and Ar501 to Ar504 are the same as or different from each other, and are each independently a substituted or unsubstituted monocyclic or polycyclic aryl group having 6 to 30 carbon atoms, or are bonded to an adjacent group to form a substituted or unsubstituted monocyclic or polycyclic hetero ring having 2 to 30 carbon atoms.

According to an exemplary embodiment of the present specification, R501 and Ar501 to Ar504 are a phenyl group, or are bonded to an adjacent group to form a carbazole group which is unsubstituted or substituted with a phenyl group.

According to an exemplary embodiment of the present specification, Ar501 and L501 are bonded to form a carbazole substituted with a phenyl group.

According to an exemplary embodiment of the present specification, Ar503 is bonded to L503 to form a carbazole substituted with a phenyl group.

According to an exemplary embodiment of the present specification, Chemical Formula CP-1 is represented by the following compound.

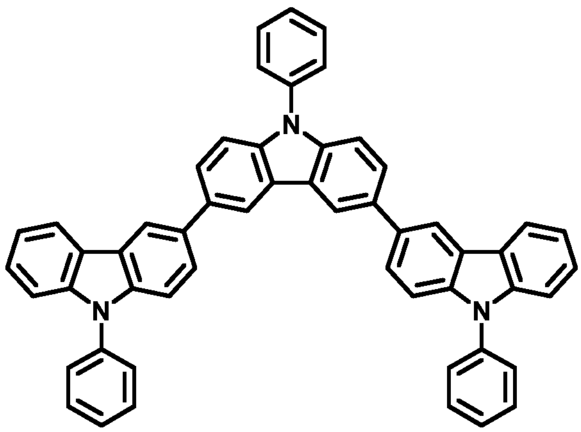

The organic light emitting device according to the present specification may be a top-emission type, a bottom-emission type or a dual-emission type depending on the materials used.

The organic light emitting device according to the present specification may be included and used in various electronic devices. For example, the electronic device may be a display panel, a touch panel, a solar module, a lighting device, and the like, and is not limited thereto.

[Mode for Invention]

Hereinafter, the present specification will be described in detail with reference to Examples, Comparative Examples, and the like for specifically describing the present specification. However, the Examples and the Comparative Examples according to the present specification may be modified in various forms, and it is not interpreted that the scope of the present specification is limited to the Examples and the Comparative Examples described below in detail. The Examples and the Comparative Examples of the present specification are provided to more completely explain the present specification to a person with ordinary skill in the art.

Preparation Example 1. Synthesis of Chemical Formulae A1 and B1

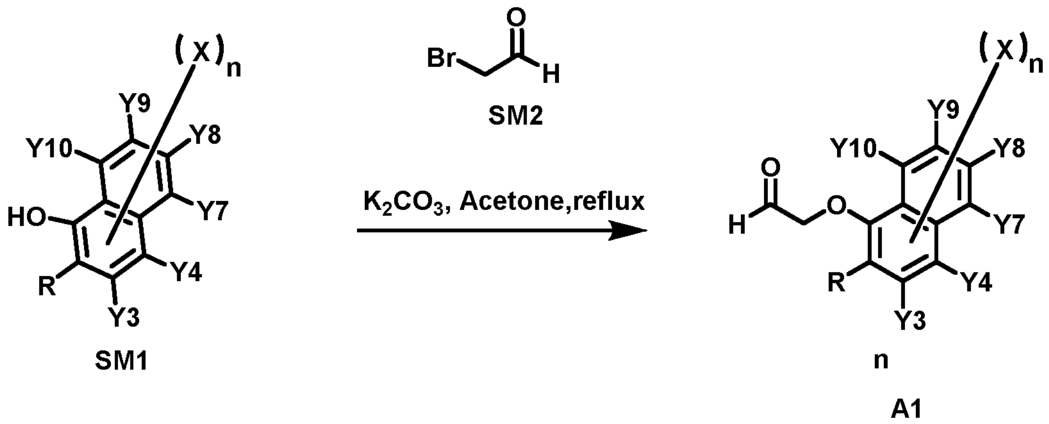

SM1 SM2 n A1

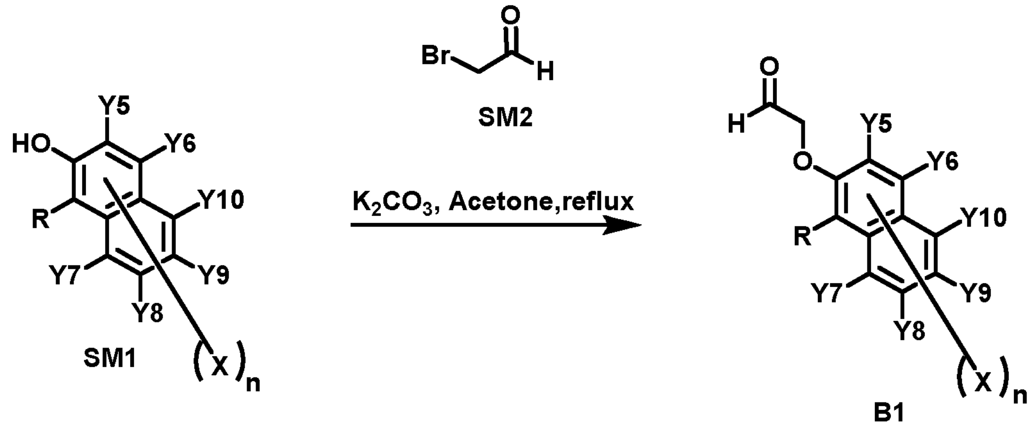

SM1 SM2 B1

After SM1 (1 eq) and SM2 (1.1 eq) were added to acetone (excess), potassium carbonate (2 eq) was added thereto, and the resulting mixture was stirred under reflux for 24 hours. After the temperature was lowered to room temperature and the reaction was terminated, water and ethanol were added thereto, and the resulting mixture was filtered to prepare Chemical Formulae A1 and B1 (Chemical Formulae A1-1 to A1-7 and B1-1 to B1-6), respectively.

In the reaction scheme, X is a halogen group, n is an integer from 1 to 7, and the definition of R is the same as the above-described definitions of Y7 to Y11.

Chemical Formulae A1-1 to A1-7 and B1-1 to B1-6 of the following Table 1 were synthesized in the same manner in the synthesis method of Chemical Formulae A1 and B1, except that SM1 and SM2 were changed to SM1 and SM2 of the following Table 1, respectively.

[Table 1]

| Chemica l Formula | SM1 | SM2 | Product | Yiel d | MS [M+H] + |
|---|---|---|---|---|---|
| A1-1 | 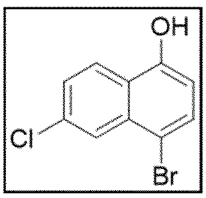 | 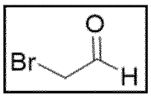 | 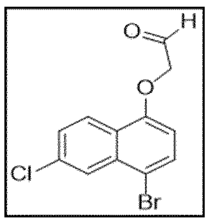 | 74% | 300.55 |
| A1-2 | 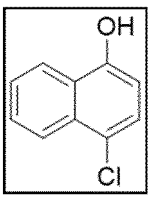 | 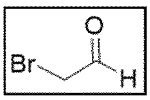 | 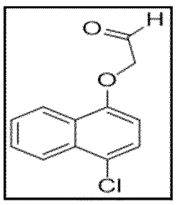 | 73% | 221.65 |

(continued)

| Chemica l Formula | SM1 | SM2 | Product | Yiel d | MS [M+H] + |
|---|---|---|---|---|---|
| A1-3 | 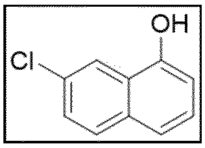 | 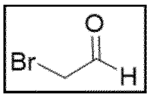 | 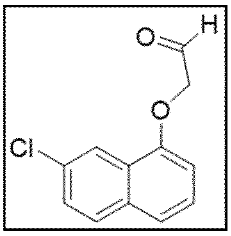 | 77% | 221.65 |
| A1-4 | 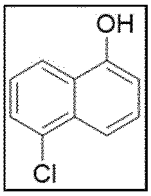 | 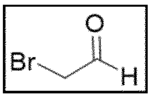 | 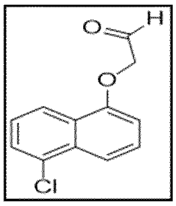 | 71% | 221.65 |
| A1-5 | 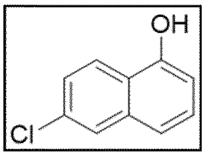 | 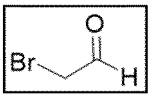 | 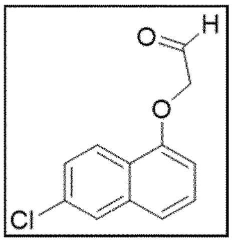 | 70% | 229.29 |
| A1-6 | 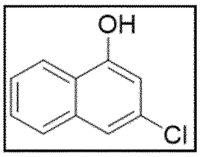 | 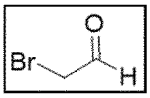 | 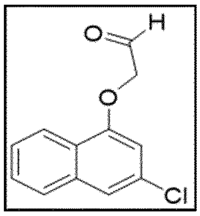 | 75% | 221.65 |
| A1-7 | 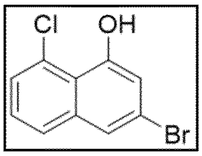 | 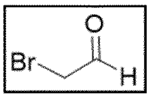 | 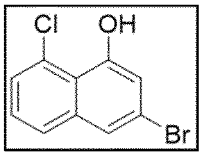 | 72% | 300.55 |
| B1-1 | 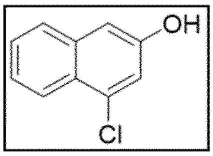 | 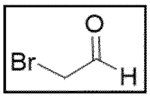 | 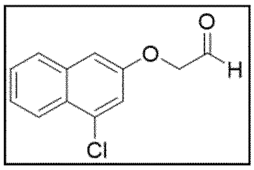 | 70% | 221.65 |
| B1-2 | 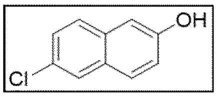 | 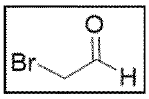 | 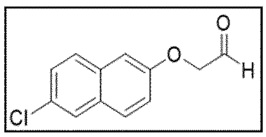 | 69% | 221.65 |
| B1-3 | 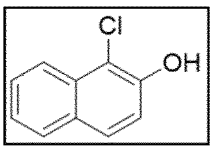 | 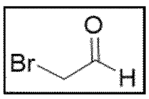 | 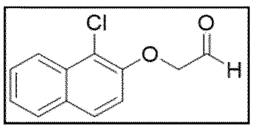 | 69% | 221.65 |

(continued)

| Chemica l Formula | SM1 | SM2 | Product | Yiel d | MS [M+H] + |
|---|---|---|---|---|---|
| B1-4 | 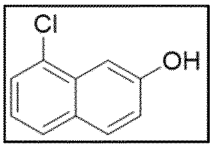 | 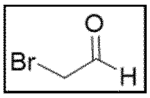 | 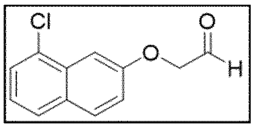 | 70% | 221.65 |
| B1-5 | 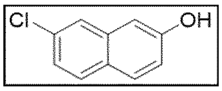 | 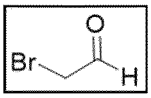 | 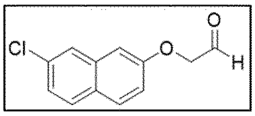 | 71% | 221.65 |
| B1-6 | 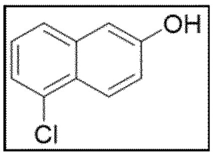 | 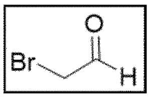 | 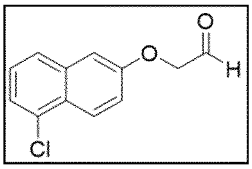 | 73% | 221.65 |

Preparation Example 2. Synthesis of Chemical Formulae A2, B2 and C2

1) Synthesis of Chemical Formulae A2 and B2

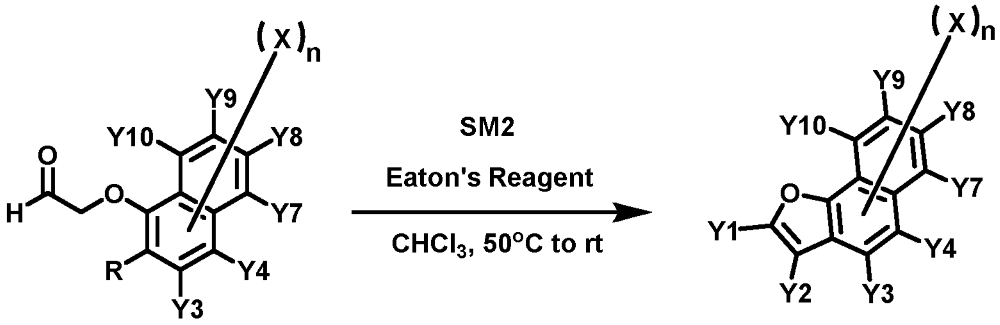


A1
SM1

A2

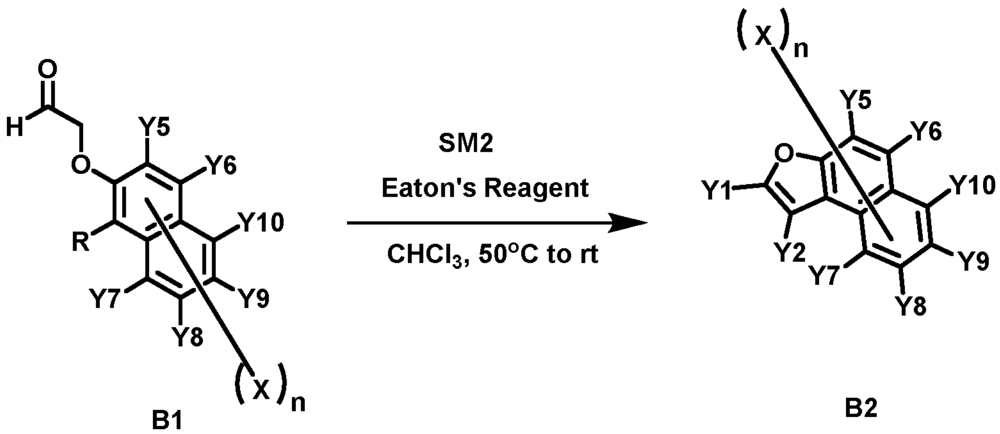


B1
SM1

B2

After SM1 (1 eq, Chemical Formula A1 or B1) was put into chloroform (excess), the resulting mixture was refluxed under heating and dissolved, and then Eaton's reagent (1 eq, phosphorus pentoxide, 7.7 wt% in methanesulfonic acid, Sigma-Aldrich) was added thereto, and the resulting mixture was stirred for 1 hour. The temperature was lowered to room temperature, water was added thereto, the resulting mixture was stirred for 30 minutes, and then layers were separated, and the organic layer was extracted until the pH became neutral with a saturated aqueous solution of sodium hydrogen carbonate ($NaHCO_3$). Thereafter, the organic layer was columned with hexane and ethyl acetate to prepare Chemical Formulae A2 and B2 (Chemical Formulae A2-1 to A2-7 and B2-1 to B2-6), respectively.

In the reaction scheme, X is a halogen group, and n is an integer from 1 to 6.

Chemical Formulae A2-1 to A2-7 and B2-1 to B2-6 of the following Table 2 were synthesized in the same manner in the synthesis method of Chemical Formulae A2 and B2, except that SM1 and SM2 were changed to SM1 and SM2 of the following Table 2, respectively.

2) Synthesis of Chemical Formula C2

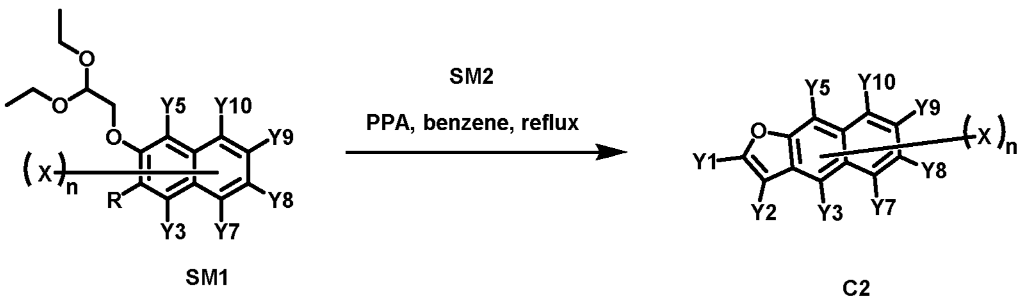


SM1 (1 eq) was put into benzene (excess), polyphosphoric acid (PPA, excess) was added thereto under stirring, and the resulting mixture was stirred for 4 hours. The temperature was lowered to room temperature, water was added thereto, the resulting mixture was stirred for 30 minutes, and then layers were separated, and the organic layer was extracted until the pH became neutral with a saturated aqueous solution of sodium hydrogen carbonate ($NaHCO_3$). Thereafter, the organic layer was columned with hexane and ethyl acetate to prepare Chemical Formula C2 (Chemical Formulae C2-1 to C2-11), respectively.

In the reaction scheme, X is a halogen group, and n is an integer from 1 to 6.

Chemical Formulae C2-1 to C2-11 of the following Table 2 were synthesized in the same manner in the synthesis method of Chemical Formula C2, except that SM1 and SM2 were changed to SM1 and SM2 of the following Table 2, respectively.

[Table 2]

| Chemical Formula | SM1 | SM2 | Product | Yield | MS [M+H]+ |
|---|---|---|---|---|---|
| A2-1 | A1-1 | Eaton's reagent ($P_2O_5$ in MSA 7.5 w/w%) | 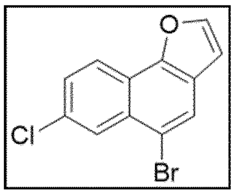 | 58% | 282.53 |
| A2-2 | A1-2 | Eaton's reagent ($P_2O_5$ in MSA 7.5 w/w%) | 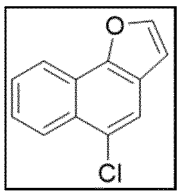 | 50% | 203.64 |

(continued)

| Chemical Formula | SM1 | SM2 | Product | Yield | MS [M+H] + |
|---|---|---|---|---|---|
| A2-3 | A1-3 | Eaton's reagent ($P_2O_5$ in MSA 7.5 w/w%) | 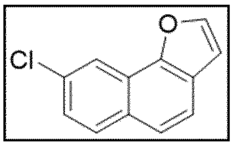 | 49% | 203.64 |
| A2-4 | A1-4 | Eaton's reagent ($P_2O_5$ in MSA 7.5 w/w%) | 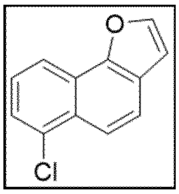 | 60% | 203.64 |
| A2-5 | A1-5 | Eaton's reagent ($P_2O_5$ in MSA 7.5 w/w%) | 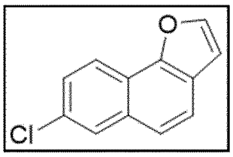 | 62% | 203.64 |
| A2-6 | A1-6 | Eaton's reagent ($P_2O_5$ in MSA 7.5 w/w%) | 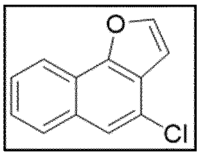 | 63% | 203.64 |
| A2-7 | A1-7 | Eaton's reagent ($P_2O_5$ in MSA 7.5 w/w%) | 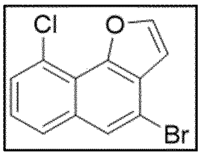 | 55% | 282.53 |
| B2-1 | B1-1 | Eaton's reagent ($P_2O_5$ in MSA 7.5 w/w%) | 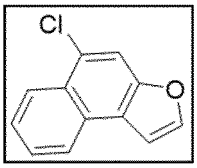 | 53% | 203.64 |
| B2-2 | B1-2 | Eaton's reagent ($P_2O_5$ in MSA 7.5 w/w%) | 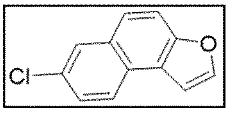 | 54% | 203.64 |
| B2-3 | B1-3 | Eaton's reagent ($P_2O_5$ in MSA 7.5 w/w%) | 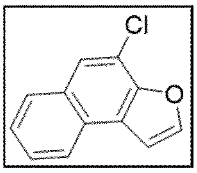 | 55% | 203.64 |

(continued)

| Chemical Formula | SM1 | SM2 | Product | Yield | MS [M+H] + |
|---|---|---|---|---|---|
| B2-4 | B1-4 | Eaton's reagent ($P_2O_5$ in MSA 7.5 w/w%) | 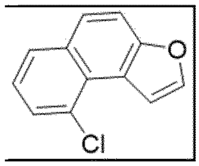 | 57% | 203.64 |
| B2-5 | B1-5 | Eaton's reagent ($P_2O_5$ in MSA 7.5 w/w%) | 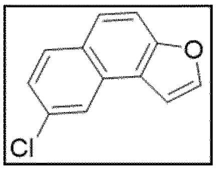 | 60% | 203.64 |
| B2-6 | B1-6 | Eaton's reagent ($P_2O_5$ in MSA 7.5 w/w%) | 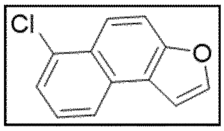 | 61% | 203.64 |
| C2-1 | 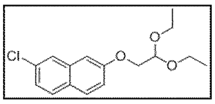 | PPA, benzene | 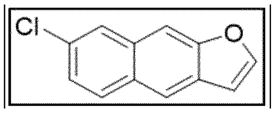 | 66% | 203.64 |
| C2-2 | 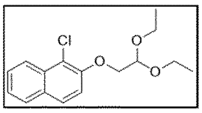 | PPA, benzene | 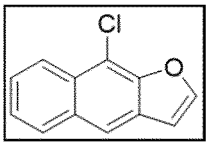 | 53% | 203.64 |
| C2-3 | 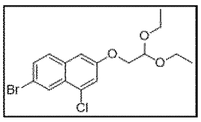 | PPA, benzene | 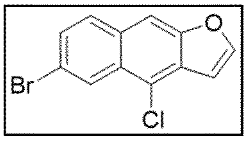 | 55% | 282.53 |
| C2-4 | 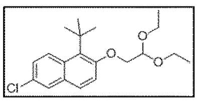 | PPA, benzene | 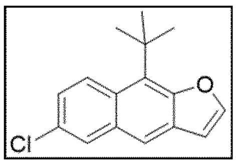 | 51% | 259.75 |
| C2-5 | 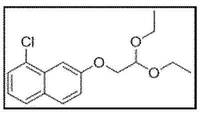 | PPA, benzene | 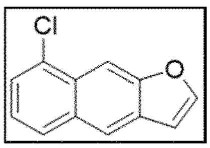 | 58% | 203.64 |
| C2-6 | 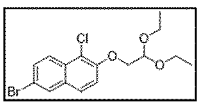 | PPA, benzene | 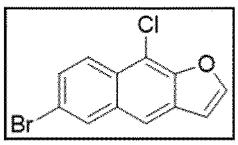 | 60% | 282.53 |

(continued)

| Chemical Formula | SM1 | SM2 | Product | Yield | MS [M+H] + |
|---|---|---|---|---|---|
| C2-7 | 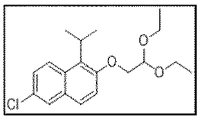 | PPA, benzene | 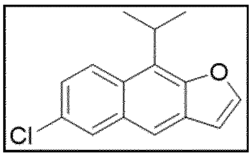 | 57% | 245.72 |
| C2-8 | 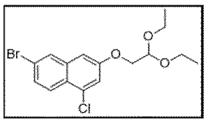 | PPA, benzene | 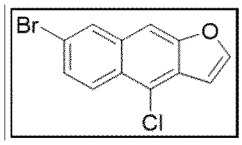 | 53% | 282.53 |
| C2-9 | 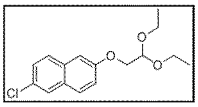 | PPA, benzene | 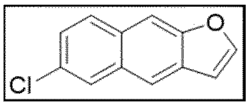 | 55% | 203.64 |
| C2-10 | 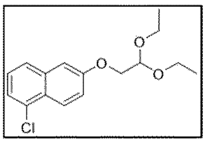 | PPA, benzene | 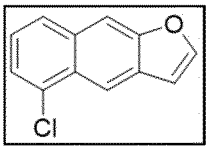 | 54% | 203.64 |
| C2-11 | 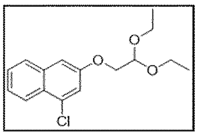 | PPA, benzene | 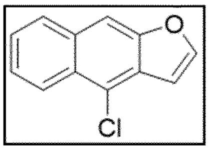 | 59% | 203.64 |

Preparation Example 3. Synthesis of Chemical Formulae A3-1 to A3-3, B3-1 to B3-3 and C3-1 to C3-3

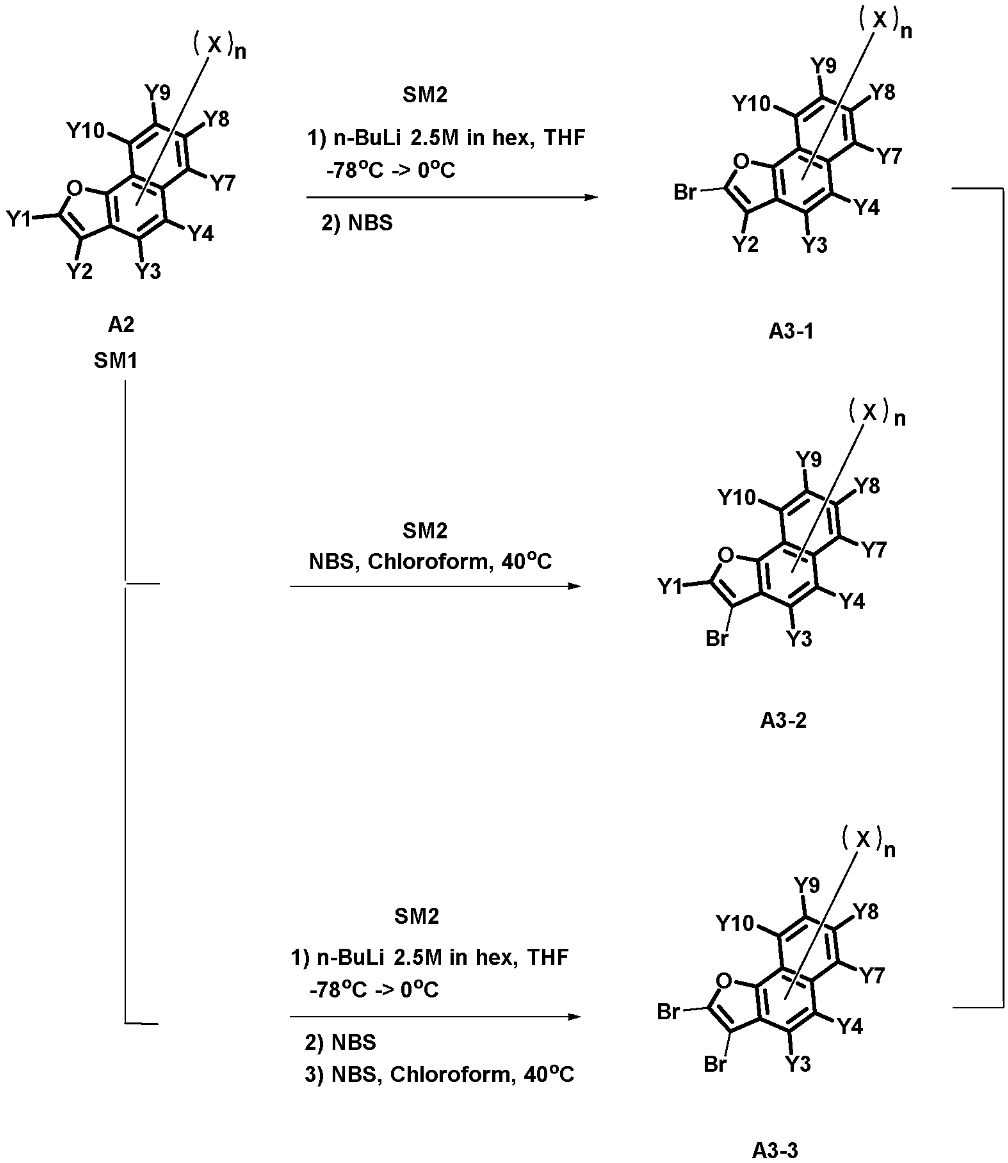


A2
SM1

A3-1

A3-2

A3-3

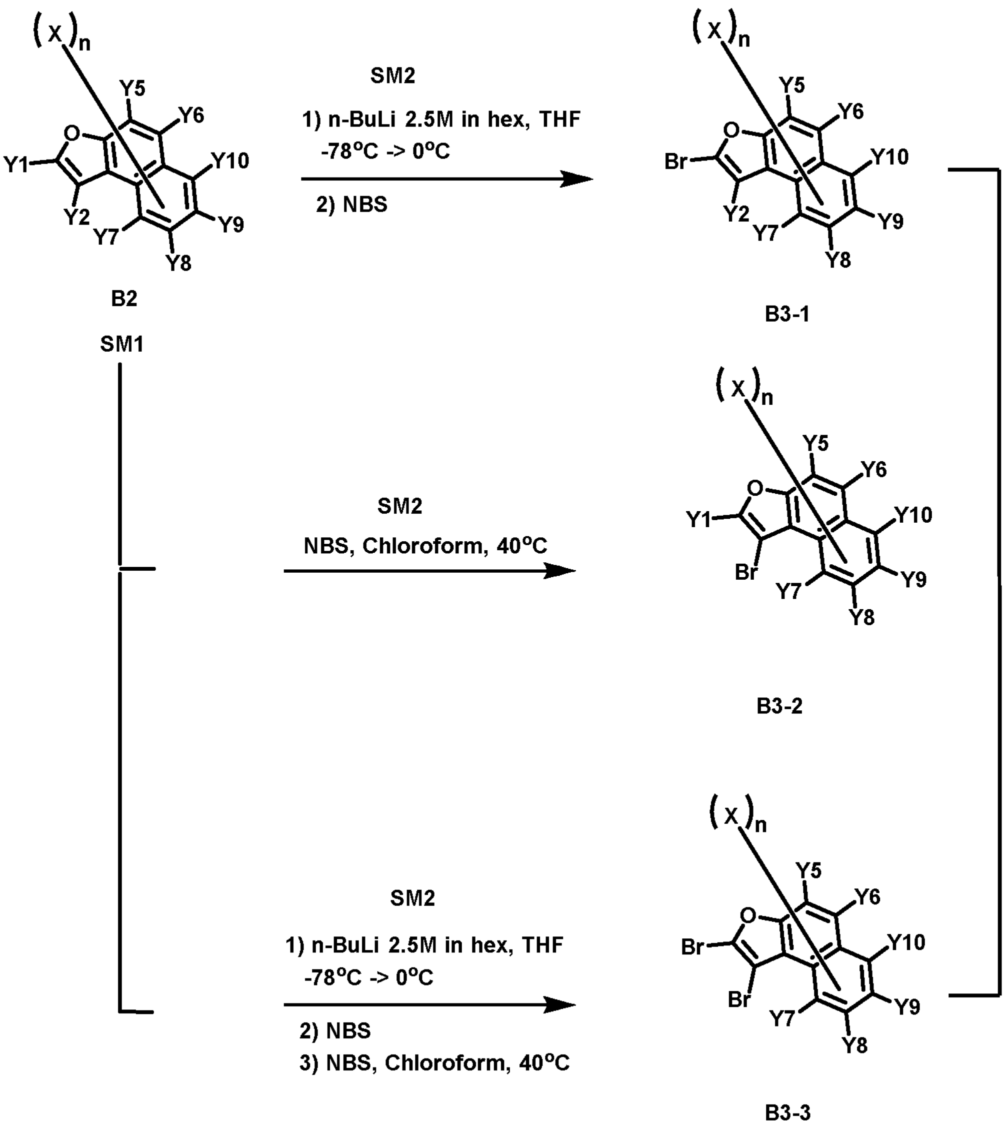


B2

SM1

B3-1

B3-2

B3-3

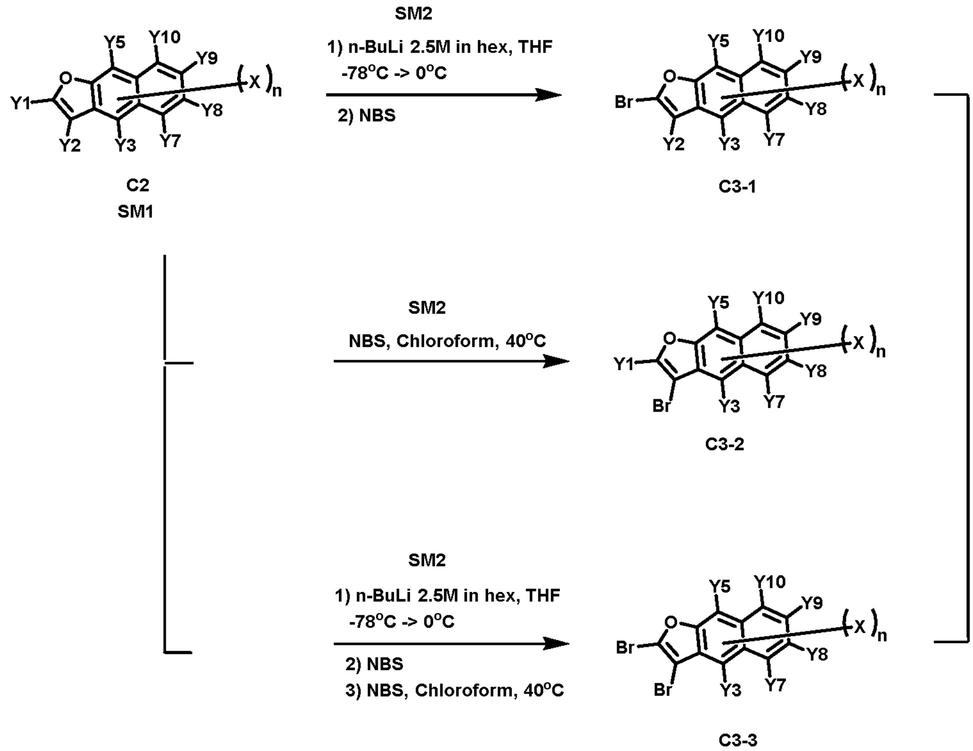


C2
SM1

C3-1

C3-2

C3-3

1) Synthesis of Chemical Formulae A3-1, B3-1 and C3-1

After SM1 (1 eq, Chemical Formula A2, B2 or C2) was dissolved in THF (excess), the temperature was lowered to - 78°C, then 2.5 M n-BuLi (1 eq) was added dropwise thereto, the resulting mixture was stirred for 1 hour, and then N-bromosuccinimide (1 eq) was added thereto. Thereafter, the reactant was warmed to room temperature and stirred for 4 hours, and 1 N HCl (excess) was added thereto to terminate the reaction. After the reaction was completed, the solvent was removed by separating layers, and then the residue was subjected to silica column chromatography (ethyl acetate/hexane 1:15) to prepare Chemical Formulae A3-1, B3-1 and C3-1, respectively.

In the reaction scheme, X is a halogen group, and n is an integer from 1 to 6.

The chemical formulae of the following Table 3 were synthesized in the same manner in the synthesis method of Chemical Formulae A3-1, B3-1 and C3-1, except that SM1 and SM2 were changed to SM1 and SM2 of the following Table 3, respectively.

2) Synthesis of Chemical Formulae A3-2, B3-2 and C3-2

After SM1 (1 eq, Chemical Formula A2, B2 or C2) was dissolved in chloroform (excess), N-bromosuccinimide (1 eq) was added thereto, the temperature was increased to 40°C, and the resulting mixture was heated and stirred for 3 hours. After the resulting product was cooled to room temperature, 1 N HCl (excess) was added thereto to terminate the reaction. After the reaction was completed, the solvent was removed by separating layers, and then the residue was subjected to silica column chromatography (ethyl acetate/hexane 1:15) to prepare Chemical Formulae A3-2, B3-2 and C3-2, respectively.

In the reaction scheme, X is a halogen group, and n is an integer from 1 to 6.

The chemical formulae of the following Table 3 were synthesized in the same manner in the synthesis method of Chemical Formulae A3-2, B3-2 and C3-2, except that SM1 and SM2 were changed to SM1 and SM2 of the following Table 3, respectively.

3) Synthesis of Chemical Formulae A3-3, B3-3 and C3-3

After SM1 (1 eq, Chemical Formula A2, B2 or C2) was dissolved in THF (excess), the temperature was lowered to - 78°C, then 2.5 M n-BuLi (1 eq) was added dropwise thereto, the resulting mixture was stirred for 1 hour, and then N-bromosuccinimide (1 eq) was added thereto. Thereafter, the reactant was warmed to room temperature and stirred for 4 hours, and 1 N HCl (excess) was added thereto to terminate the reaction. After the reaction was completed, the solvent was removed by separating layers, then the residue was dissolved in chloroform (excess), then N-bromosuccinimide (1 eq) was added thereto, the temperature was increased to 40°C, and the resulting mixture was heated and stirred for 3 hours. After the resulting product was cooled to room temperature, 1 N HCl (excess) was added thereto to terminate the reaction. After the reaction was completed, the solvent was removed by separating layers, and then the residue was subjected to silica column chromatography (ethyl acetate/hexane 1:15) to prepare Chemical Formulae A3-3, B3-3 and C3-3, respectively.

In the reaction scheme, X is a halogen group, and n is an integer from 1 to 6.

The chemical formulae of the following Table 3 were synthesized in the same manner in the synthesis method of Chemical Formulae A3-3, B3-3 and C3-3, except that SM1 and SM2 were changed to SM1 and SM2 of the following Table 3, respectively.

[Table 3]

| Chemical Formula | SM1 | SM2 | Product | Yield | MS [M+H] + |
|---|---|---|---|---|---|
| A3-3-1 | A2-2 | 1) n-BuLi 2.5M in hex 2) NBS 3) NBS, chlroform, 40°C | 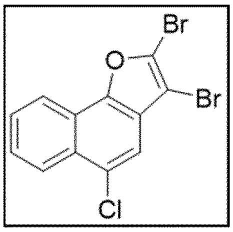 | 71% | 361.43 |
| A3-1-1 | A2-3 | 1) n-BuLi 2.5M in hex 2) NBS | 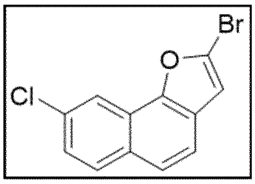 | 72% | 282.53 |
| A3-2-1 | A2-4 | NBS, $CHCl_3$ | 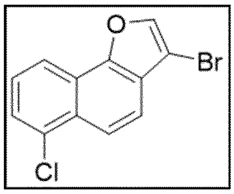 | 70% | 282.53 |
| A3-2-2 | A2-5 | NBS, $CHCl_3$ | 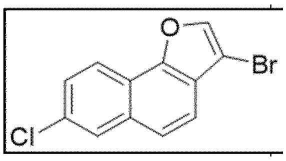 | 73% | 282.53 |
| A3-1-2 | A2-6 | 1) n-BuLi 2.5M in hex 2) NBS | 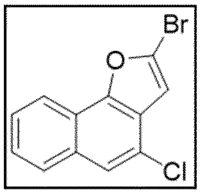 | 75% | 282.53 |
| A3-1-3 | A2-2 | 1) n-BuLi 2.5M in hex 2) NBS | 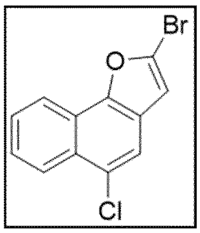 | 77% | 282.53 |

(continued)

| Chemical Formula | SM1 | SM2 | Product | Yield | MS [M+H] + |
|---|---|---|---|---|---|
| A3-2-3 | A2-3 | NBS, $CHCl_3$ | 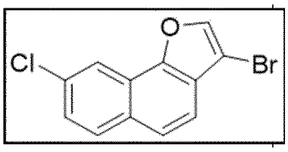 | 75% | 282.53 |
| A3-1-4 | A2-5 | 1) n-BuLi 2.5M in hex<br>2) NBS | 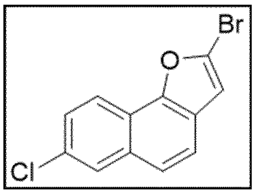 | 69% | 282.53 |
| B3-3-1 | B2-1 | 1) n-BuLi 2.5M in hex<br>2) NBS<br>3) NBS, chlroform, 40°C | 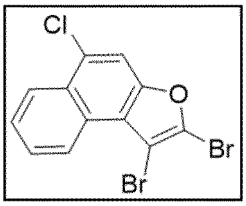 | 75% | 361.43 |
| B3-2-1 | B2-2 | NBS, $CHCl_3$ | 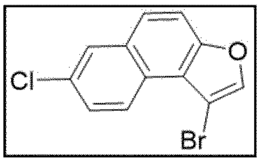 | 73% | 282.53 |
| B3-1-1 | B2-3 | 1) n-BuLi 2.5M in hex<br>2) NBS | 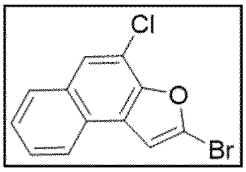 | 74% | 282.53 |
| B3-1-2 | B2-4 | 1) n-BuLi 2.5M in hex<br>2) NBS | 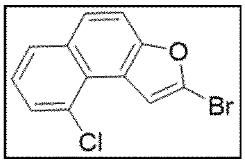 | 70% | 282.53 |
| B3-3-2 | B2-5 | 1) n-BuLi 2.5M in hex<br>2) NBS<br>3) NBS, chlroform, 40°C | 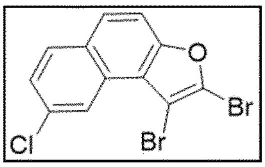 | 71% | 361.43 |
| B3-2-2 | B2-6 | NBS, $CHCl_3$ | 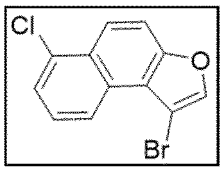 | 77% | 282.53 |

(continued)

| Chemical Formula | SM1 | SM2 | Product | Yield | MS [M+H] + |
|---|---|---|---|---|---|
| C3-2-1 | C2-2 | NBS, $CHCl_3$ | 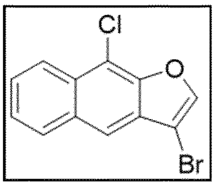 | 73% | 282.53 |
| C3-1-1 | C2-3 | 1) n-BuLi 2.5M in hex<br>2) NBS | 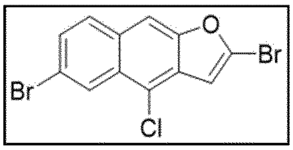 | 75% | 361.43 |
| C3-1-2 | C2-1 | 1) n-BuLi 2.5M in hex<br>2) NBS | 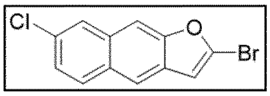 | 72% | 282.53 |
| C3-1-3 | C2-2 | 1) n-BuLi 2.5M in hex<br>2) NBS | 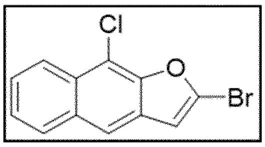 | 75% | 282.53 |
| C3-3-1 | C2-1 | 1) n-BuLi 2.5M in hex<br>2) NBS<br>3) NBS, chlroform, 40°C | 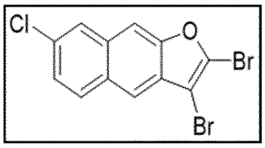 | 70% | 361.43 |
| C3-2-2 | B4-3 | NBS, $CHCl_3$ | 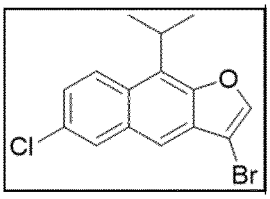 | 71% | 324.61 |
| C3-2-3 | B4-3 | NBS, $CHCl_3$ | 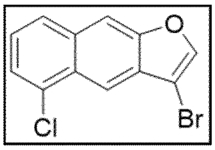 | 68% | 282.53 |

Preparation Example 4. Synthesis of Chemical Formulae A4, B4 and C4

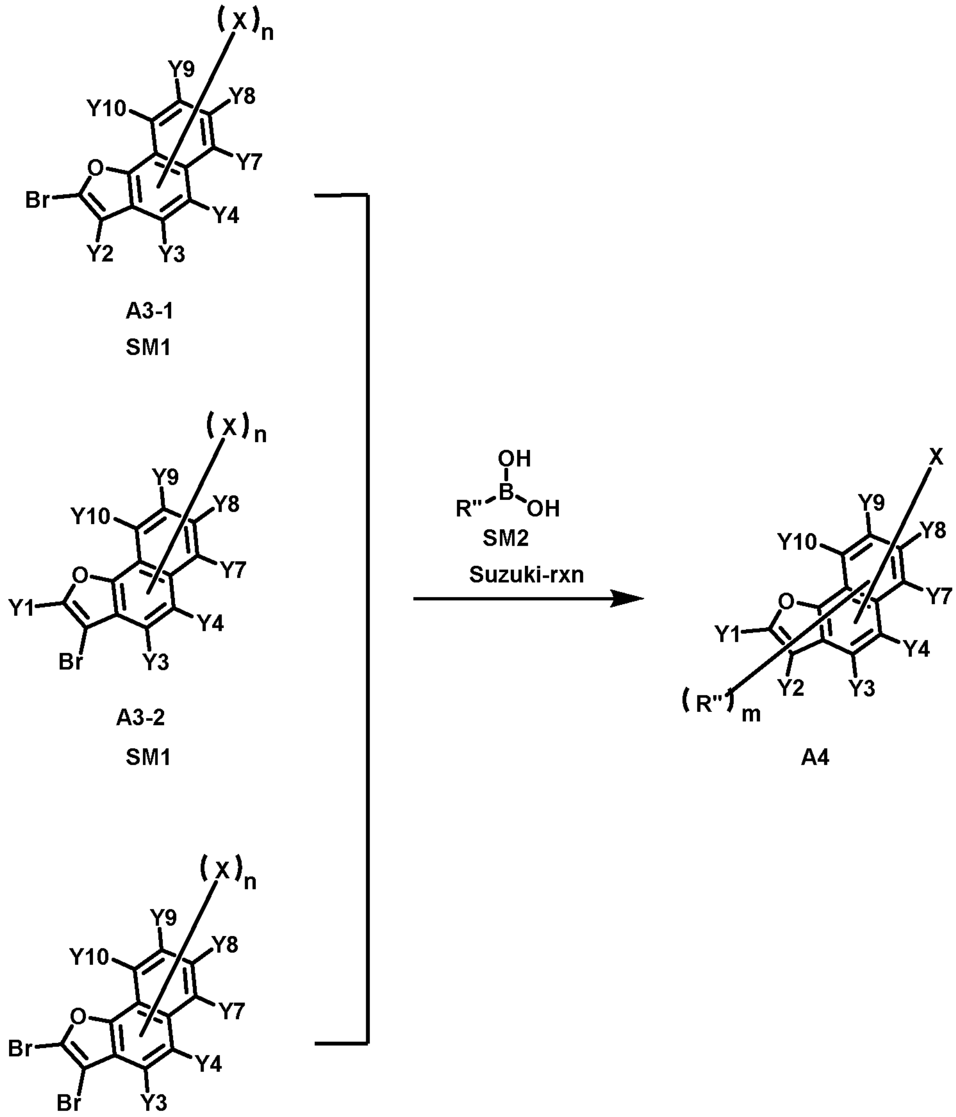


A3-1
SM1

A3-2
SM1

A4

A3-3
SM1

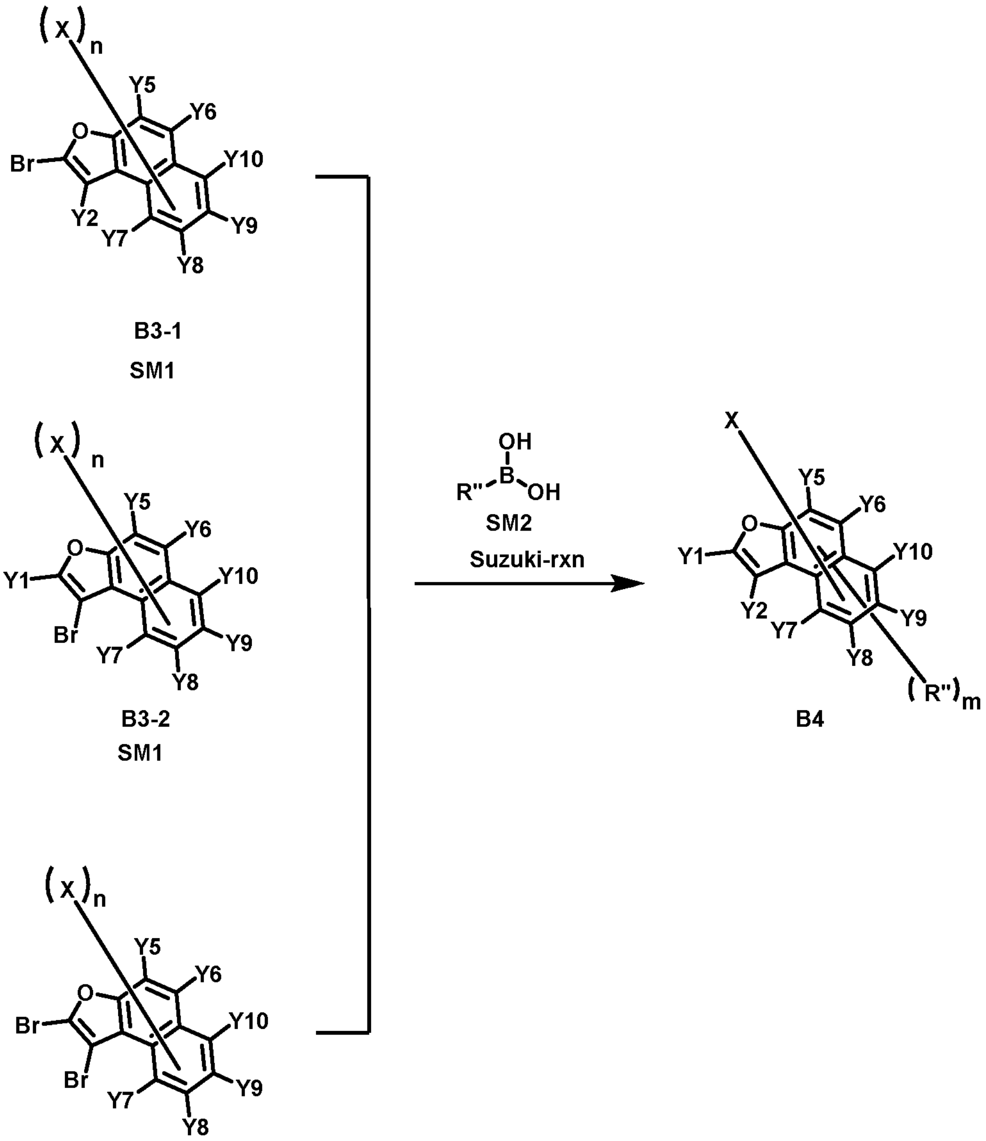


B3-1
SM1

B3-2
SM1

B4

B3-3
SM1

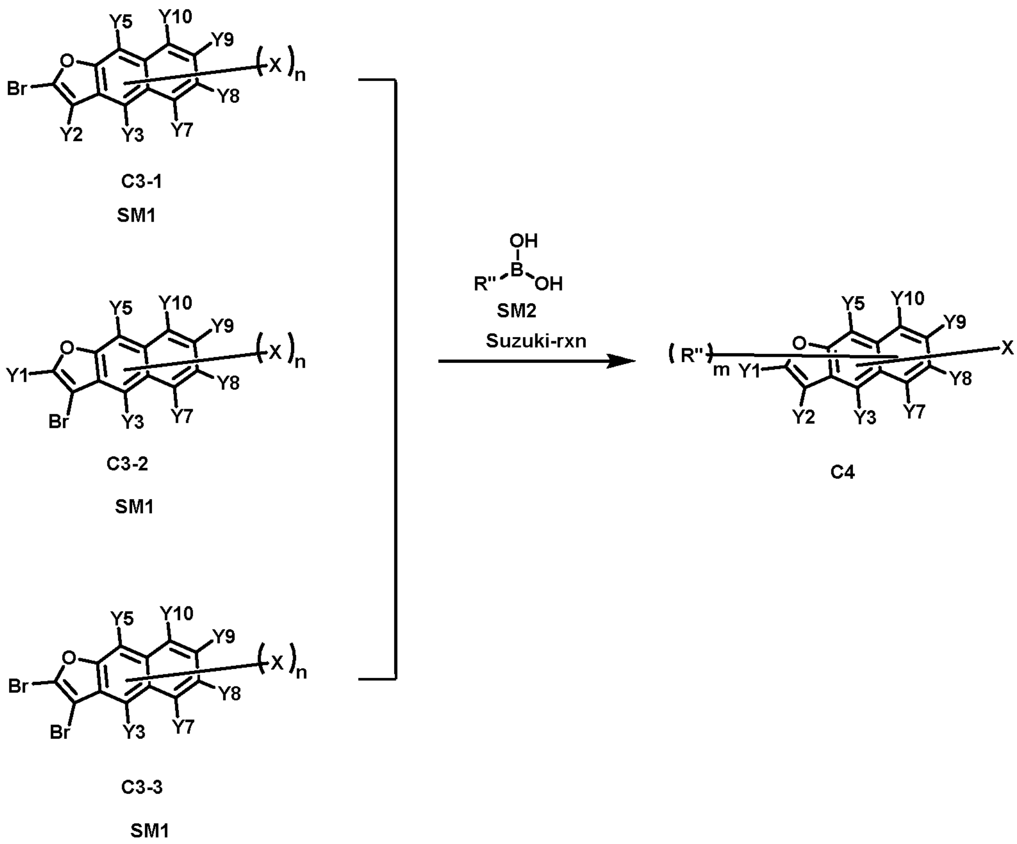


C3-1
SM1

C3-2
SM1

C3-3
SM1

C4

After SM1 (1 eq, Chemical Formulae A3-1 to A3-3, B3-1 to B3-3, C3-1, C3-2 or C3-3) and SM2 (1.02 eq) were added to tetrahydrofuran (excess), a 2 M aqueous potassium carbonate solution (30-fold volume ratio compared to THF) was added thereto, and tetrakis triphenyl-phosphine palladium (2 mol%) was added thereto, and then the resulting mixture was heated and stirred for 10 hours. After the temperature was lowered to room temperature and the reaction was terminated, the aqueous potassium carbonate solution was removed to separate the layers. After the solvent was removed, the residue was distilled under vacuum and recrystallized with ethyl acetate and hexane to prepare Chemical Formulae A4, B4 and C4, respectively.

In the reaction scheme, X is a halogen group, n is an integer from 1 to 5, the definition of R" is the same as the above-described definitions of Y7 to Y11, and m is an integer from 1 to 7.

The chemical formulae of the following Table 4 were synthesized in the same manner in the synthesis method of Chemical Formulae A4, B4 and C4, except that SM1 and SM2 were changed to SM1 and SM2 of the following Table 4, respectively.

[Table 4]

| Chemical Formula | SM1 | SM2 | Product | Yield | MS [M+H] + |
|---|---|---|---|---|---|
| A4-1 | A2-1 | 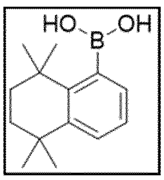 | 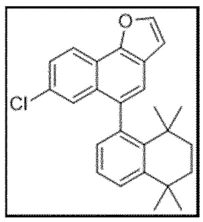 | 74% | 389.94 |

(continued)

| Chemical Formula | SM1 | SM2 | Product | Yield | MS [M+H] + |
|---|---|---|---|---|---|
| A4-2 | A3-3-1 | 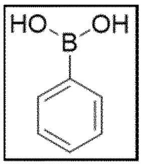 | 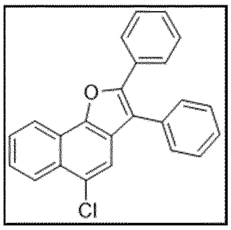 | 75% | 355.83 |
| A4-3 | A3-1-1 | 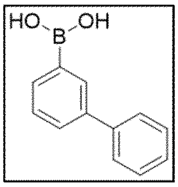 | 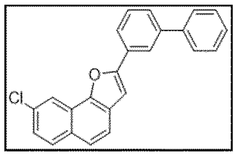 | 73% | 355.83 |
| A4-4 | A3-2-1 | 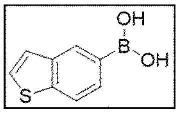 | 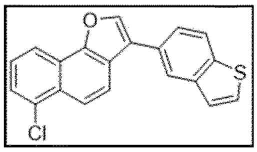 | 77% | 335.82 |
| A4-5 | A3-2-2 | 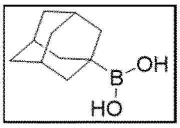 | 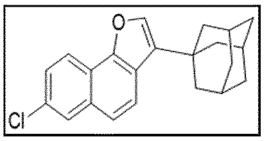 | 73% | 337.86 |
| A4-6 | A3-1-2 | 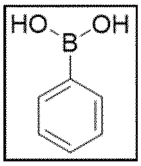 | 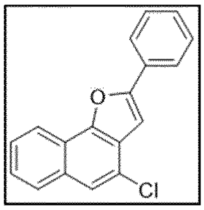 | 71% | 279.74 |
| A4-7 | A3-1-3 | 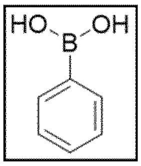 | 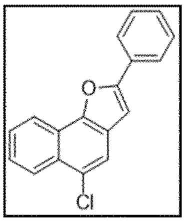 | 70% | 279.74 |
| A4-8 | A3-2-3 | 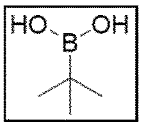 | 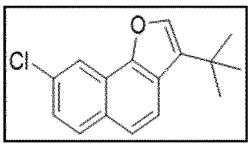 | 72% | 259.75 |
| A4-9 | A2-7 | 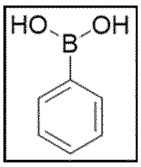 | 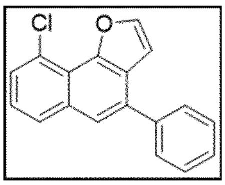 | 75% | 279.74 |

(continued)

| Chemical Formula | SM1 | SM2 | Product | Yield | MS [M+H] + |
|---|---|---|---|---|---|
| A4-10 | A3-1-4 | 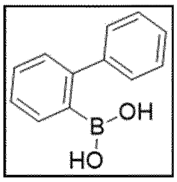 | 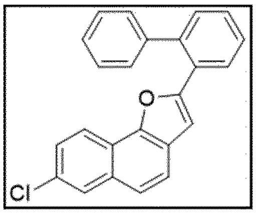 | 73% | 355.83 |
| B4-1 | B3-3-1 | 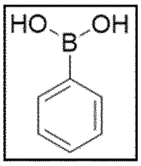 | 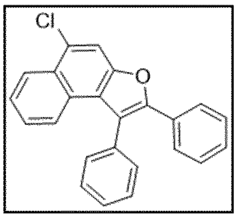 | 76% | 355.83 |
| B4-2 | B3-2-1 | 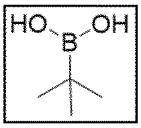 | 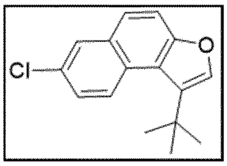 | 70% | 259.75 |
| B4-3 | B3-1-1 | 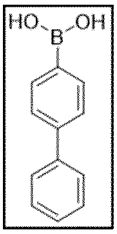 | 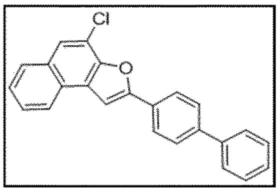 | 78% | 355.83 |
| B4-4 | B3-1-2 | 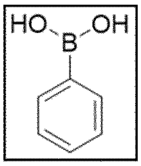 | 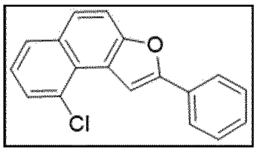 | 77% | 279.74 |
| B4-5 | B3-3-2 | 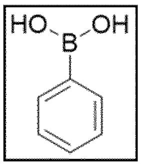 | 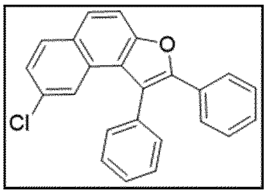 | 71% | 355.83 |
| B4-6 | B3-2-2 | 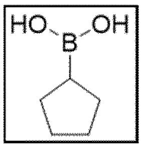 | 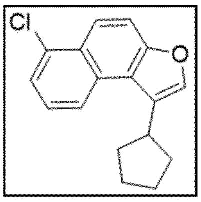 | 70% | 271.76 |

(continued)

| Chemical Formula | SM1 | SM2 | Product | Yield | MS [M+H] + |
|---|---|---|---|---|---|
| C4-1 | C3-2-1 | 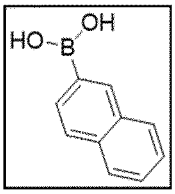 | 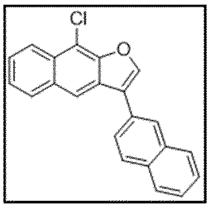 | 75% | 329.80 |
| C4-2 | C3-1-1 | 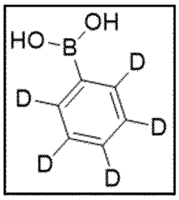 | 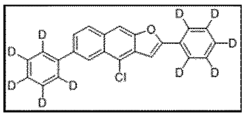 | 73% | 365.89 |
| C4-3 | C3-1-2 | 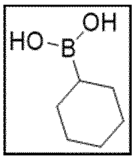 | 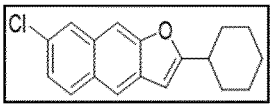 | 71% | 285.78 |
| C4-4 | C3-1-3 | 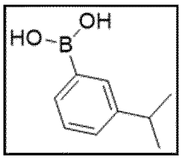 | 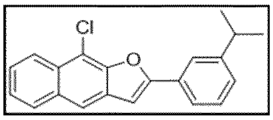 | 72% | 321.82 |
| C4-5 | C3-3-1 | 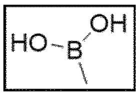 | 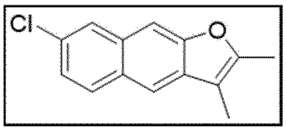 | 73% | 231.69 |
| C4-6 | C2-6 | 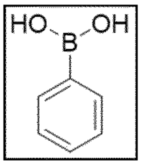 | 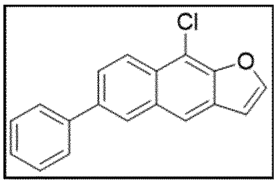 | 80% | 279.74 |
| C4-7 | C3-2-2 | 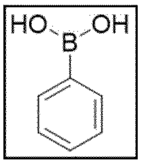 | 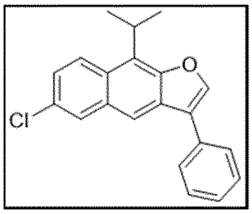 | 71% | 321.82 |
| C4-8 | C2-8 | 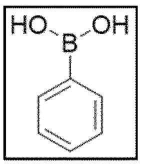 | 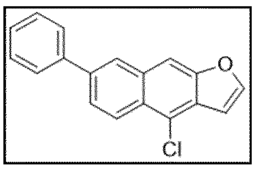 | 70% | 279.74 |

(continued)

| Chemical Formula | SM1 | SM2 | Product | Yield | MS [M+H] + |
|---|---|---|---|---|---|
| C4-9 | C3-2-3 | 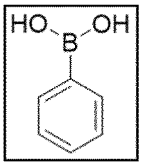 | 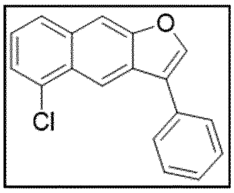 | 77% | 279.74 |

Preparation Example 5. Synthesis of Chemical Formulae A5, B5 and C5

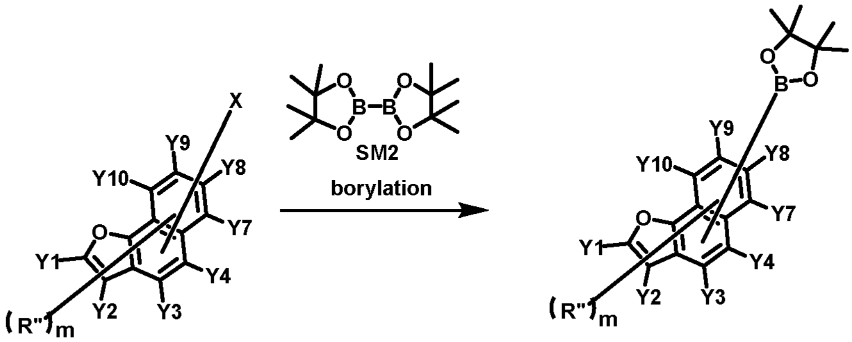


A4
SM1

A5

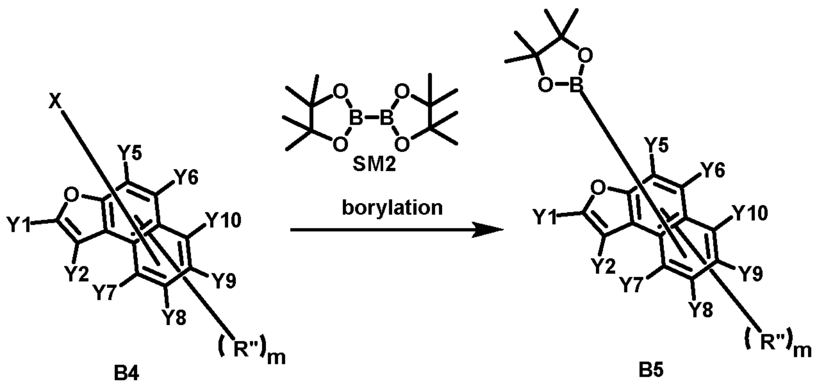


B4
SM1

B5

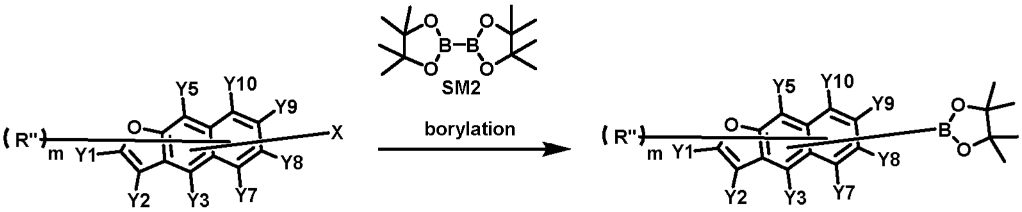


C4
SM1

C5

SM1 (1 eq, Chemical Formula A4, B4 or C4) and SM2 (1.3 eq) were put into 1,4-dioxane (12-fold mass ratio

compared to SM1), potassium acetate (3 eq) was added thereto, and the resulting mixture was stirred and refluxed. After palladium acetate (0.02 eq) and tricyclohexylphosphine (0.04 eq) are stirred in 1,4-dioxane for 5 minutes, the resulting mixture was added thereto, and 10 hours later, the mixture was cooled to room temperature after confirming that the reaction was terminated. After ethanol and water were added thereto, the resulting mixture was filtered and purified by recrystallization with ethyl acetate and ethanol to prepare Chemical Formulae A5, B5 and C5, respectively.

In the reaction scheme, the definition of R" is the same as the above-described definitions of Y7 to Y11, and m is an integer from 1 to 7.

The chemical formulae of the following Table 5 were synthesized in the same manner in the synthesis method of Chemical Formulae A5, B5 and C5, except that SM1 and SM2 were changed to SM1 and SM2 of the following Table 5, respectively.

[Table 5]

| Chemical Formula | SM1 | SM2 | Product | Yield | MS [M+H] + |
|---|---|---|---|---|---|
| A5-1 | A4-1 | 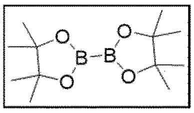 | 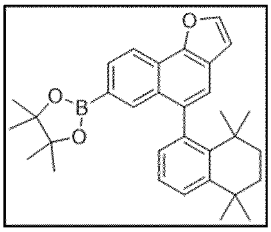 | 78% | 481.49 |
| A5-2 | A4-2 | 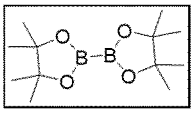 | 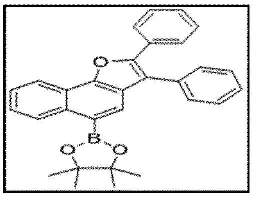 | 77% | 447.35 |
| A5-3 | A4-3 | 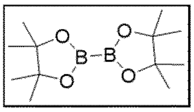 | 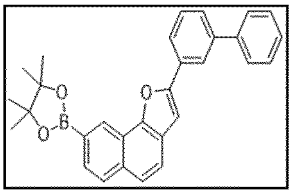 | 74% | 447.35 |
| A5-4 | A4-4 | 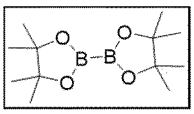 | 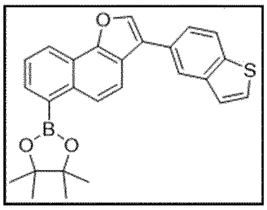 | 80% | 427.34 |
| A5-5 | A4-5 | 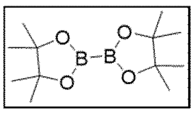 | 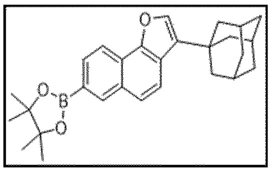 | 74% | 429.38 |
| A5-6 | A4-6 | 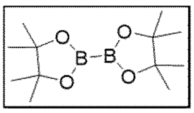 | 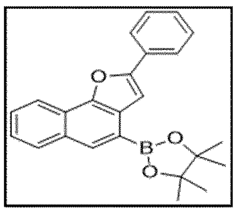 | 79% | 371.26 |

(continued)

| Chemical Formula | SM1 | SM2 | Product | Yield | MS [M+H] + |
|---|---|---|---|---|---|
| A5-7 | A4-7 | 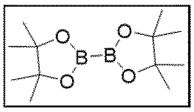 | 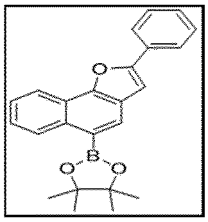 | 80% | 371.26 |
| A5-8 | A2-6 | 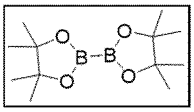 | 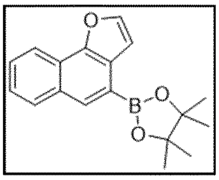 | 71% | 295.16 |
| A5-9 | A4-8 | 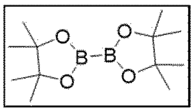 | 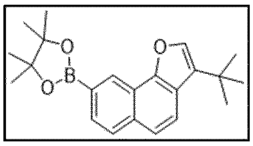 | 74% | 351.27 |
| A5-10 | A4-9 | 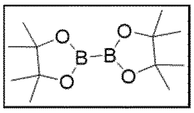 | 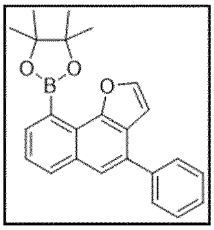 | 77% | 371.26 |
| A5-11 | A4-10 | 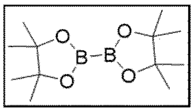 | 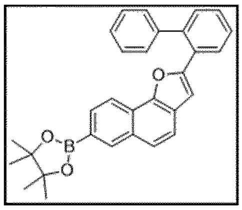 | 75% | 447.35 |
| A5-12 | A2-5 | 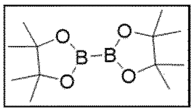 | 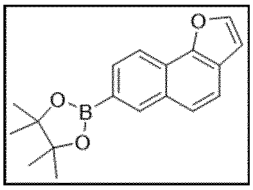 | 76% | 295.16 |
| B5-1 | B4-1 | 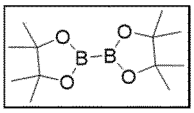 | 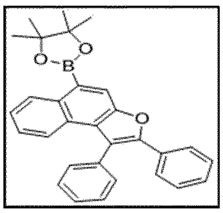 | 77% | 447.35 |

(continued)

| Chemical Formula | SM1 | SM2 | Product | Yield | MS [M+H] + |
|---|---|---|---|---|---|
| B5-2 | B4-2 | 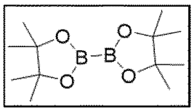 | 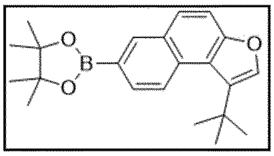 | 76% | 351.27 |
| B5-3 | B4-3 | 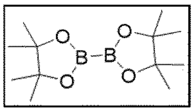 | 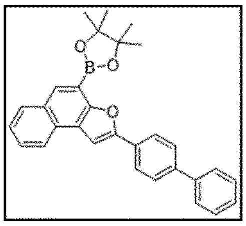 | 70% | 447.35 |
| B5-4 | B4-4 | 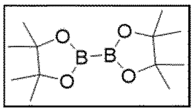 | 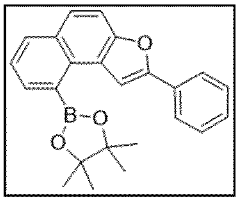 | 79% | 371.26 |
| B5-5 | B4-5 | 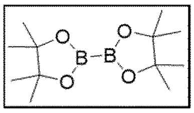 | 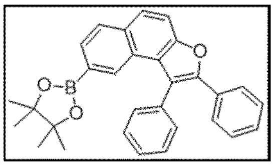 | 73% | 447.35 |
| B5-6 | B4-6 | 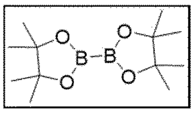 | 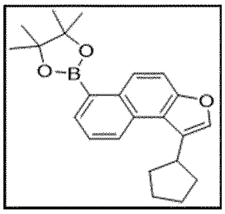 | 71% | 362.28 |
| B5-7 | B2-2 | 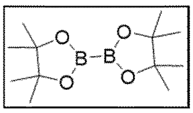 | 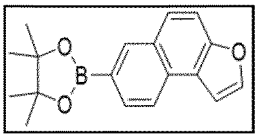 | 70% | 295.16 |
| C5-1 | C2-1 | 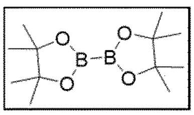 | 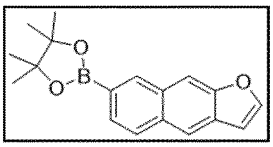 | 77% | 295.16 |
| C5-2 | C4-1 | 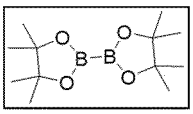 | 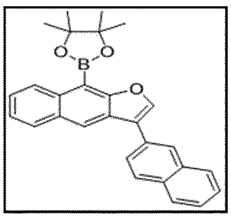 | 73% | 421.32 |

(continued)

| Chemical Formula | SM1 | SM2 | Product | Yield | MS [M+H] + |
|---|---|---|---|---|---|
| C5-3 | C4-2 | 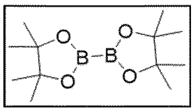 | 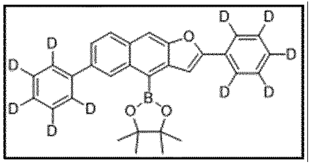 | 72% | 457.41 |
| C5-4 | C4-3 | 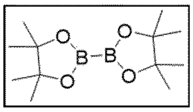 | 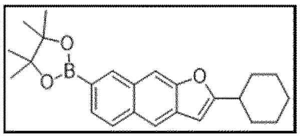 | 70% | 377.30 |
| C5-5 | C2-4 | 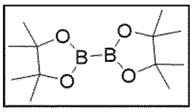 | 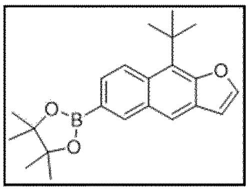 | 77% | 351.27 |
| C5-6 | C4-4 | 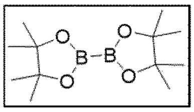 | 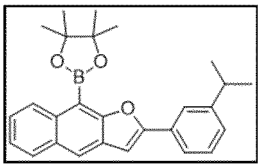 | 73% | 413.34 |
| C5-7 | C2-5 | 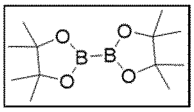 | 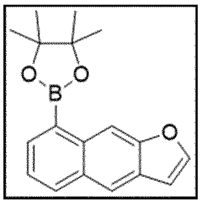 | 74% | 295.16 |
| C5-8 | C4-5 | 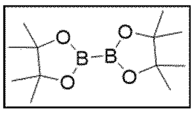 | 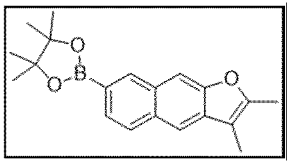 | 75% | 323.21 |
| C5-9 | C4-6 | 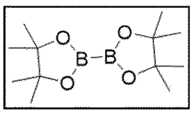 | 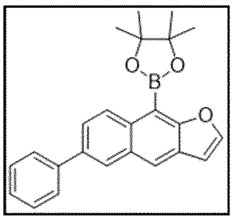 | 71% | 371.26 |
| C5-10 | C4-7 | 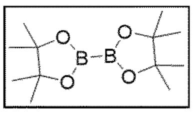 | 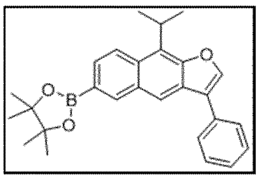 | 73% | 413.34 |

(continued)

| Chemical Formula | SM1 | SM2 | Product | Yield | MS [M+H] + |
|---|---|---|---|---|---|
| C5-11 | C4-8 | 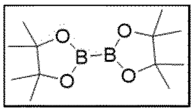 | 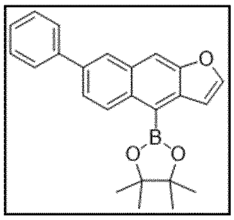 | 77% | 371.26 |
| C5-12 | C2-9 | 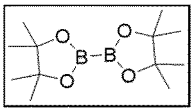 | 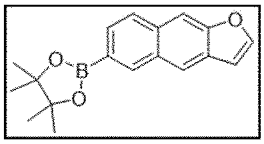 | 72% | 295.16 |
| C5-13 | C2-10 | 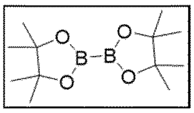 | 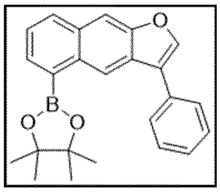 | 73% | 371.26 |
| C5-14 | C2-11 | 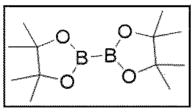 | 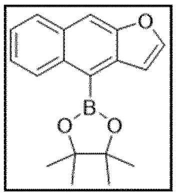 | 70% | 295.16 |

Preparation Example 6. Synthesis of Compounds 1 to 33

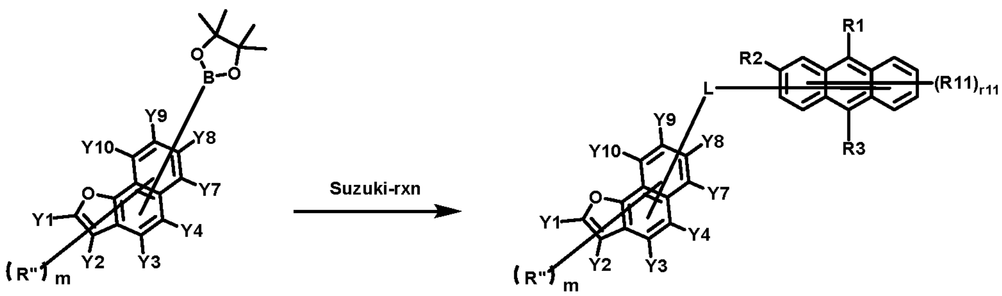


A5
SM1

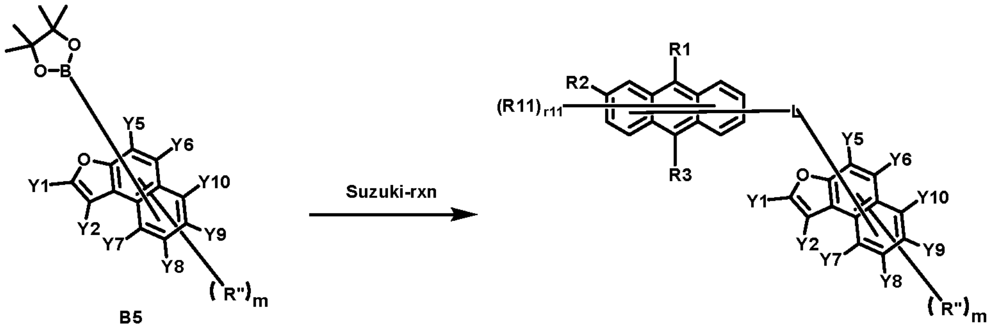


B5

SM1

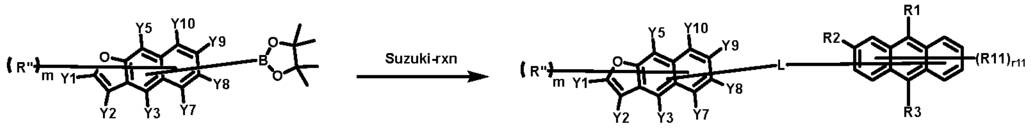


C5

SM1

After SM1 (1.05 eq, Chemical Formula A5, B5 or C5) and SM2 (1 eq) were added to tetrahydrofuran (excess), a 2 M aqueous potassium carbonate solution (30-fold volume ratio compared to THF) was added thereto, and tetrakis triphenyl-phosphine palladium (2 mol%) was added thereto, and then the resulting mixture was heated and stirred for 10 hours. After the temperature was lowered to room temperature and the reaction was terminated, the aqueous potassium carbonate solution was removed to separate the layers. After it was confirmed that the reaction was terminated, the temperature was lowered to room temperature to remove the solvent, and the residue was purified with recrystallization using toluene to prepare the following Compounds 1 to 33, respectively.

In the reaction scheme, the definition of R" is the same as the above-described definitions of Y7 to Y11, and m is an integer from 1 to 7.

Compounds 1 to 33 of the following Table 6 were synthesized in the same manner as described above, except that SM1 and SM2 were changed to SM1 and SM2 of the following Table 6, respectively.

SM2 of the following Table 6 was prepared in the following scheme.

SM2 scheme

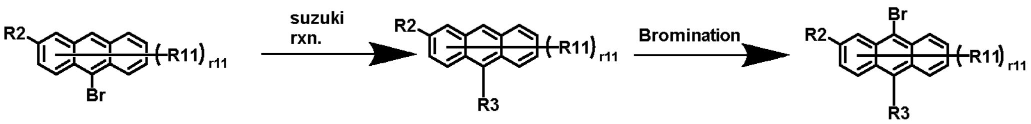


SM2

SM2 Example 1>

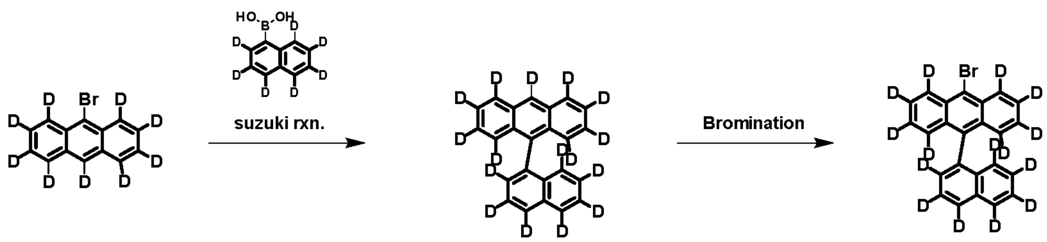

SM2 Example 2>

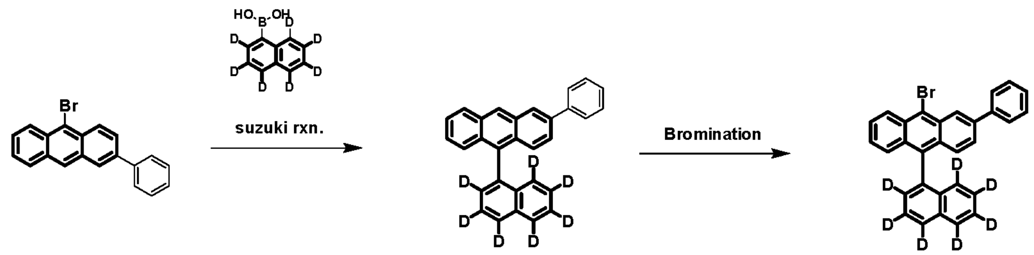


SM2 of Compounds 1 to 33 were prepared as the synthesis examples as described above.

In addition, the Suzuki reaction is the same method as in the synthesis of Chemical formulae A4, B4, and C4, and the bromination is the same method as in the synthesis of Chemical Formulae A3-1 to A3-3, B3-1 to B3-3 and C3-1 to C3-3, except that SM1 and SM2 were changed.

[Table 6]

| Compoun d | SM1 | SM2 | Product | Yiel d | MS [M+H]+ |
|---|---|---|---|---|---|
| 1 | A5-1 | 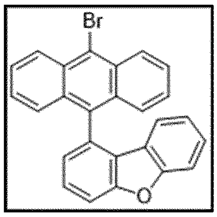 | | 71% | 697.89 |
| 2 | A5-2 | 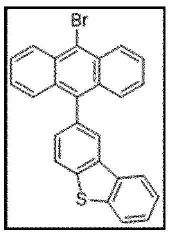 | | 72% | 679.85 |
| 3 | A5-3 | 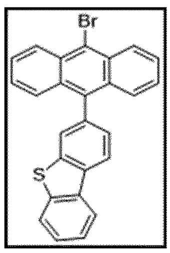 | | 69% | 679.85 |
| 4 | A5-4 | 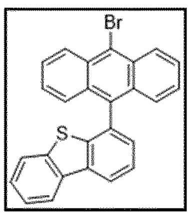 | | 68% | 659.83 |

(continued)

| Compoun d | SM1 | SM2 | Product | Yiel d | MS [M+H]+ |
|---|---|---|---|---|---|
| 5 | A5-5 | 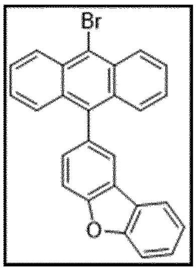 | | 70% | 645.81 |
| 6 | A5-6 | 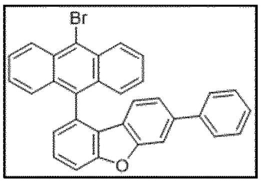 | | 75% | 663.79 |
| 7 | A5-7 | 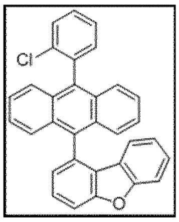 | | 75% | 663.79 |
| 8 | A5-8 | 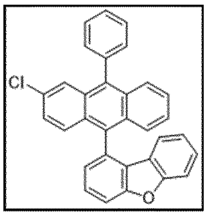 | | 71% | 587.69 |
| 9 | A5-9 | 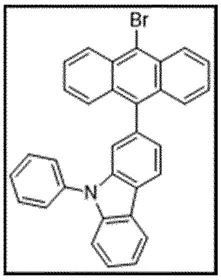 | | 65% | 642.81 |
| 10 | A5-10 | 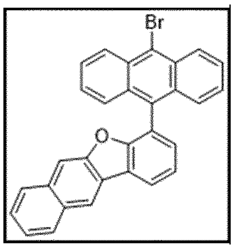 | | 79% | 637.75 |

(continued)

| Compoun d | SM1 | SM2 | Product | Yiel d | MS [M+H] + |
|---|---|---|---|---|---|
| 11 | A5-11 | 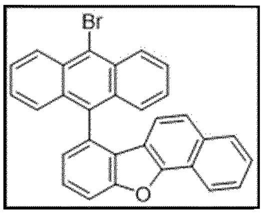 | | 74% | 713.85 |
| 12 | A5-12 | 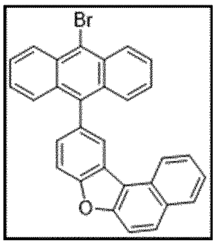 | | 77% | 561.65 |
| 13 | B5-1 | 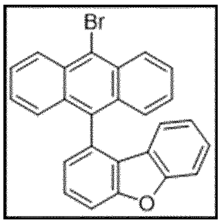 | | 72% | 663.79 |
| 14 | B5-2 | 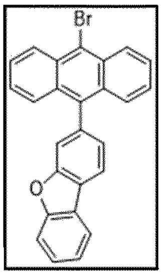 | | 70% | 567.70 |
| 15 | B5-3 | 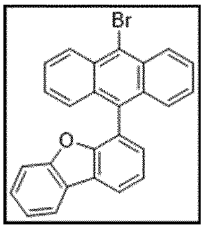 | | 71% | 663.79 |
| 16 | B5-4 | 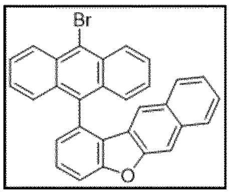 | | 73% | 637.75 |

(continued)

| Compoun d | SM1 | SM2 | Product | Yiel d | MS [M+H]+ |
|---|---|---|---|---|---|
| 17 | B5-5 | 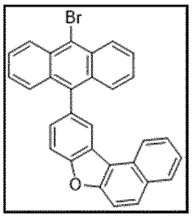 | | 70% | 713.85 |
| 18 | B5-6 | 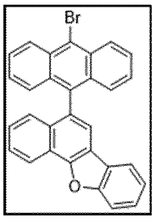 | | 76% | 629.77 |
| 19 | B5-7 | 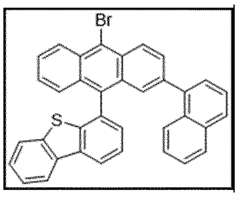 | | 68% | 653.81 |
| 20 | C5-1 | 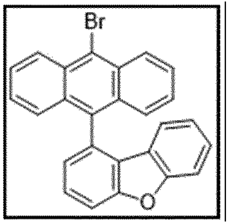 | | 67% | 511.59 |
| 21 | C5-2 | 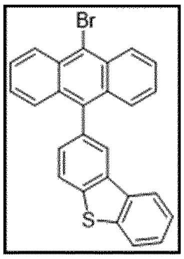 | | 68% | 653.81 |
| 22 | C5-3 | 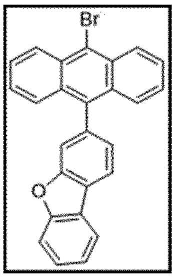 | | 73% | 673.85 |

(continued)

| Compoun d | SM1 | SM2 | Product | Yiel d | MS [M+H] + |
|---|---|---|---|---|---|
| 23 | C5-4 | 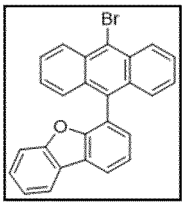 | | 75% | 593.74 |
| 24 | C5-5 | 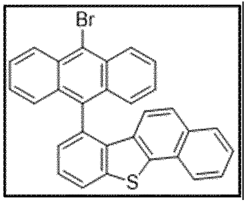 | | 70% | 633.82 |
| 25 | C5-6 | 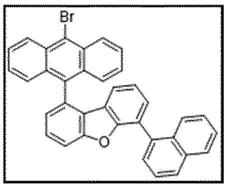 | | 73% | 755.93 |
| 26 | C5-7 | 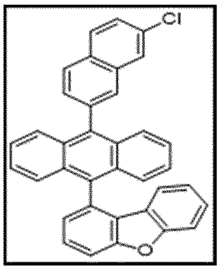 | | 69% | 637.75 |
| 27 | C5-8 | 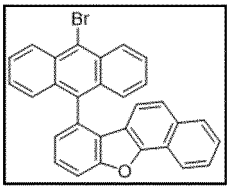 | | 68% | 589.71 |
| 28 | C5-9 | 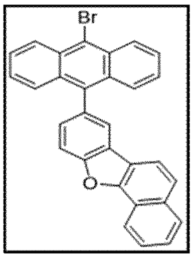 | | 65% | 637.75 |
| 29 | C5-10 | 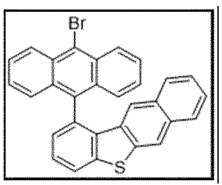 | | 76% | 695.89 |

(continued)

| Compoun d | SM1 | SM2 | Product | Yiel d | MS [M+H] + |
|---|---|---|---|---|---|
| 30 | C5-11 | 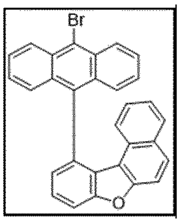 | | 77% | 637.75 |
| 31 | C5-12 | 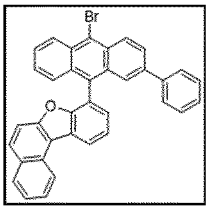 | | 70% | 637.75 |
| 32 | C5-13 | 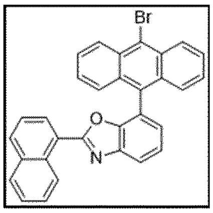 | | 71% | 664.78 |
| 33 | C5-14 | 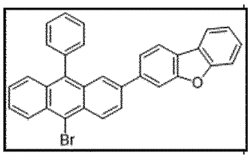 | | 70% | 587.69 |

Preparation Example 7. Synthesis of Compounds 34 to 36

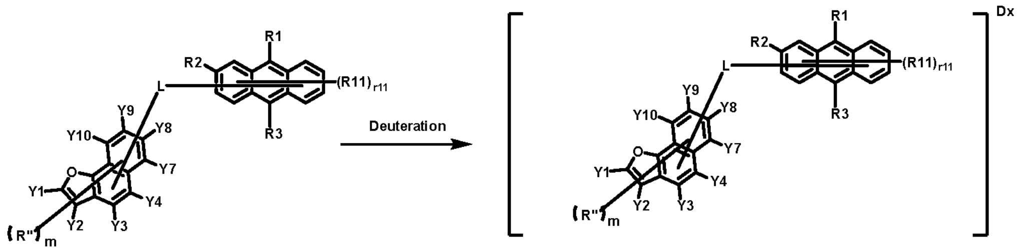


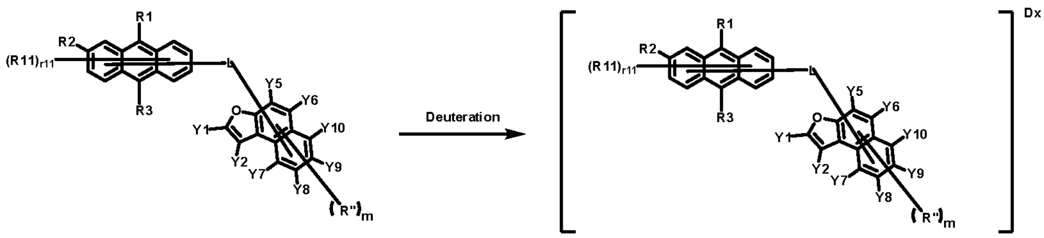

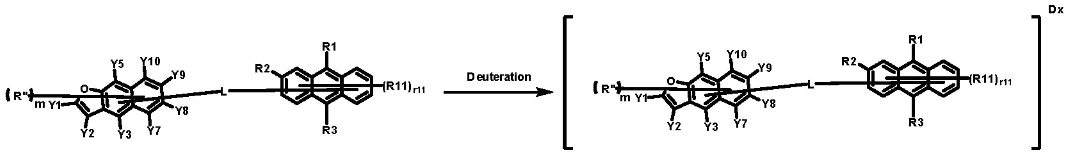


The reactant (1 eq) and trifluoromethanesulfonic acid (cat.) were put into $C_6D_6$ (10- to 50-fold mass ratio compared to the reactant), and the resulting mixture was stirred at 70°C for 10 minutes to 100 minutes. After the reaction was completed, $D_2O$ (excess) was added thereto, the resulting solution was stirred for 30 minutes, and then trimethylamine (excess) was added dropwise thereto. The reaction solution was transferred to a separatory funnel, and an extraction with water and chloroform was performed. The extract was dried over $MgSO_4$, and then recrystallized by heating with toluene to obtain Compounds 34 to 36 of the following Table 7, respectively.

In the reaction scheme, the substituents are defined as described above, and Dx means the number of deuterium atoms substituted in the compound.

Compounds 34 to 36 of the following Table 7 were synthesized in the same manner as described above, except that SM1 and SM2 were changed to the reactant in the following Table 7 and $C_6D_6$.

The reactants of the following Table 7 were prepared by changing SM1 and SM2 in the same manner as in the method of Preparation Example 6. (non-deuterated compound)

[Each product has a different degree of deuterium substitution depending on the reaction time, and the substitution rate was determined according to the maximum m/z (M+) value]

The prior literature of KR1538534 was referenced for the deuterium substitution of the aforementioned product.

[Table 7]

| Compound | Reactant | Product | Reactant m/z | Product theory max m/z | Reaction time (min) |
|---|---|---|---|---|---|
| 34 | Compou nd 2 | 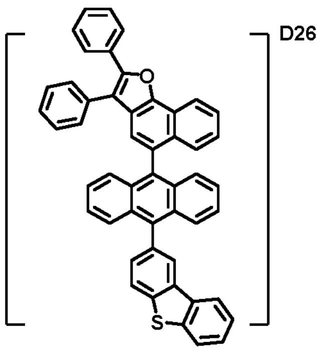 | 678 | 708 | 50 |
| 35 | Compou nd 12 | 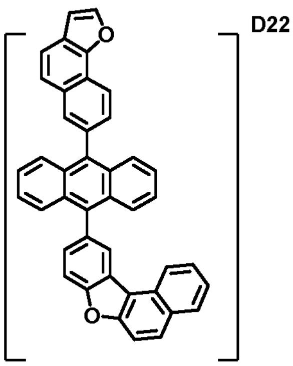 | 560 | 584 | 50 |

(continued)

| Compound | Reactant | Product | Reactant m/z | Product theory max m/z | Reaction time (min) |
|---|---|---|---|---|---|
| 36 | Compou nd 20 | 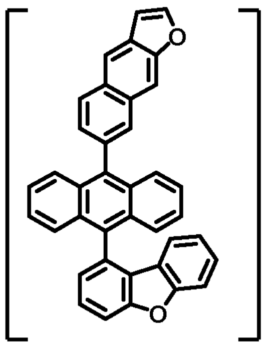 D21 | 510 | 532 | 50 |
| Compound | Product actual max m/z | number of total hydrogens | Number of D substitutions | D substitution ratio (%) | Yield |
| 34 | 704 | 30 | 26 | 87% | 72% |
| 35 | 582 | 24 | 22 | 92% | 75% |
| 36 | 531 | 22 | 21 | 95% | 70% |

<Example 1> Manufacture of OLED

As a positive electrode, a substrate on which ITO/Ag/ITO were deposited to have a thickness of 70/1,000/70 Å was cut into a size of 50 mm x 50 mm x 0.5 mm, put into distilled water in which a dispersant was dissolved, and washed with ultrasonic waves. A product manufactured by Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing using distilled water was completed, ultrasonic washing was conducted using isopropyl alcohol, acetone, and methanol solvents in this order, and drying was then conducted.

HI-1 was thermally vacuum-deposited to have a thickness of 50 Å on the positive electrode thus prepared, thereby forming a hole injection layer, and HT1 as a material which transports holes was vacuum-deposited to have a thickness of 1,150 Å thereon, thereby forming a hole transport layer, Next, a hole adjusting layer was formed using EB1 (150 Å), and then Compound 1 (host) and dopant BD1 (2 wt%) were vacuum-deposited to have a thickness of 360 Å, thereby forming a light emitting layer. Thereafter, ET1 was deposited to have a thickness of 50 Å, thereby forming an electron adjusting layer, and Compounds ET2 and Liq were mixed at a weight ratio of 7:3, thereby forming an electron transport layer having a thickness of 250 Å. Sequentially, after magnesium and lithium fluoride (LiF) were deposited to have a thickness of 50 Å to form a film as an electron injection layer (EIL), magnesium and silver (1:4, weight ratio) were used to form a negative electrode having a thickness of 200 Å, and then CP1 was deposited to have a thickness of 600 Å as a capping layer, thereby completing a device. In the aforementioned procedure, the deposition rate of the organic material was maintained at 1 Å/sec.

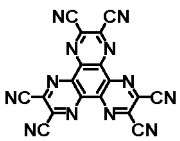

HI-1

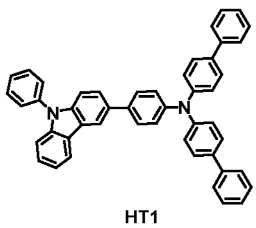

HT1

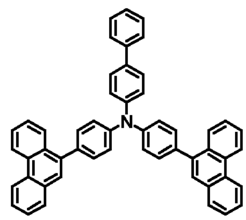

EB1

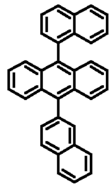

BH1

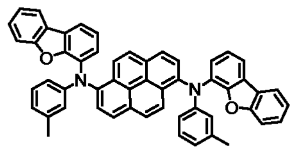

BD1

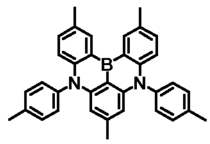

BD2

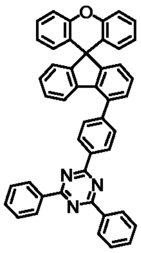

ET1

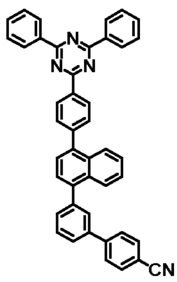

ET2

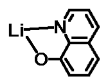

Liq

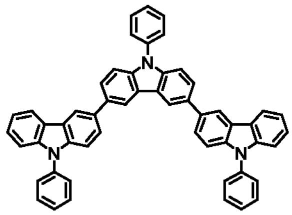

CP1

Examples 2 to 41 and Comparative Examples 1 to 10

Organic light emitting devices of Examples 2 to 41 and Comparative Examples 1 to 10 were manufactured in the same manner as in Example 1, except that compounds described in the following Table 8 were used, respectively, instead of Compound 1 and BD1 in Example 1.

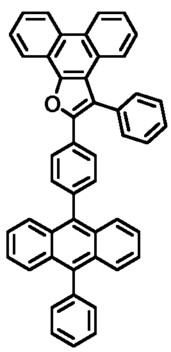

BH2

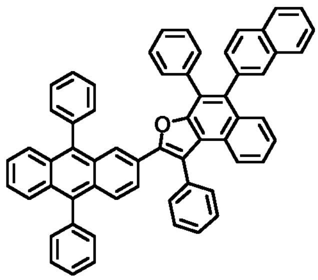

BH3

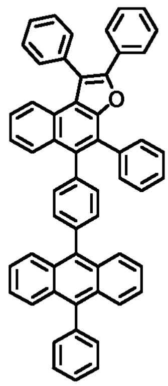

BH4

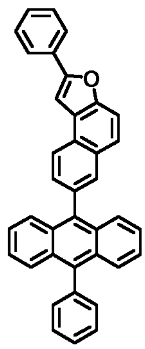

BH5

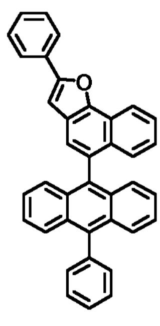

BH6

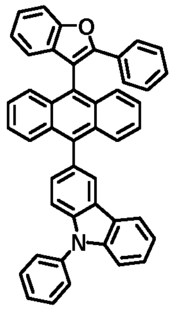

BH7

Device evaluation

A current of 20 mA/$cm^2$ was applied to the organic light emitting devices manufactured in Examples 1 to 41 and Comparative Examples 1 to 10 to measure the voltage, efficiency, color coordinates, and service life (T95), and the results are shown in the following Table 8. In this case, T95 means the time taken for the initial luminance to drop to 95% at a current density of 20 mA/$cm^2$.

[Table 8]

| Experimental Example 20 mA/$cm^2$ | Host | Dopant | Voltage (V) (@20 mA/$cm^2$) | Cd/A (@20 mA/$cm^2$) | Color coordinate (x,y) | Service life (T95, h) (@20 mA/$cm^2$) |
|---|---|---|---|---|---|---|
| Example 1 | Compound 1 | BD1 | 3.32 | 6.70 | (0.133, 0.135) | 60.3 |
| Example 2 | Compound 2 | BD1 | 3.33 | 6.71 | (0.132, 0.136) | 62.4 |
| Example 3 | Compound 3 | BD1 | 3.35 | 6.73 | (0.134, 0.135) | 63.8 |
| Example 4 | Compound 4 | BD1 | 3.38 | 6.69 | (0.133, 0.135) | 61.2 |
| Example 5 | Compound 5 | BD1 | 3.36 | 6.70 | (0.132, 0.136) | 62.8 |
| Example 6 | Compound 6 | BD1 | 3.32 | 6.68 | (0.134, 0.135) | 68.0 |
| Example 7 | Compound 7 | BD1 | 3.34 | 6.60 | (0.134, 0.135) | 63.1 |
| Example 8 | Compound 8 | BD1 | 3.24 | 6.70 | (0.135, 0.149) | 66.1 |
| Example 9 | Compound 9 | BD1 | 3.38 | 6.67 | (0.134, 0.135) | 60.8 |
| Example 10 | Compound 10 | BD1 | 3.29 | 6.71 | (0.134, 0.141) | 65.5 |
| Example 11 | Compound 11 | BD1 | 3.30 | 6.63 | (0.134, 0.142) | 65.1 |
| Example 12 | Compound 12 | BD1 | 3.31 | 6.66 | (0.134, 0.141) | 63.1 |
| Example 13 | Compound 13 | BD1 | 3.40 | 6.67 | (0.132, 0.136) | 60.3 |
| Example 14 | Compound 14 | BD1 | 3.41 | 6.72 | (0.134, 0.135) | 66.5 |
| Example 15 | Compound 15 | BD1 | 3.36 | 6.71 | (0.134, 0.135) | 62.1 |
| Example 16 | Compound 16 | BD1 | 3.34 | 6.73 | (0.134, 0.141) | 60.6 |
| Example 17 | Compound 17 | BD1 | 3.35 | 6.70 | (0.134, 0.141) | 66.8 |
| Example 18 | Compound 18 | BD1 | 3.33 | 6.69 | (0.134, 0.142) | 67.0 |
| Example 19 | Compound 19 | BD1 | 3.29 | 6.61 | (0.135, 0.149) | 63.8 |

(continued)

| Experimental Example 20 mA/cm$^2$ | Host | Dopant | Voltage (V) (@20 mA/cm$^2$) | Cd/A (@20 mA/cm$^2$) | Color coordinate (x,y) | Service life (T95, h) (@20 mA/cm$^2$) |
|---|---|---|---|---|---|---|
| Example 20 | Compound 20 | BD1 | 3.40 | 6.63 | (0.132, 0.136) | 64.2 |
| Example 21 | Compound 21 | BD1 | 3.39 | 6.73 | (0.134, 0.135) | 63.5 |
| Example 22 | Compound 22 | BD1 | 3.41 | 6.66 | (0.134, 0.135) | 65.0 |
| Example 23 | Compound 23 | BD1 | 3.38 | 6.70 | (0.134, 0.135) | 63.2 |
| Example 24 | Compound 24 | BD1 | 3.37 | 6.69 | (0.134, 0.135) | 62.5 |
| Example 25 | Compound 25 | BD1 | 3.33 | 6.66 | (0.134, 0.142) | 63.1 |
| Example 26 | Compound 26 | BD1 | 3.31 | 6.68 | (0.134, 0.144) | 61.8 |
| Example 27 | Compound 27 | BD1 | 3.35 | 6.64 | (0.134, 0.135) | 62.0 |
| Example 28 | Compound 28 | BD1 | 3.38 | 6.72 | (0.134, 0.135) | 63.8 |
| Example 29 | Compound 29 | BD1 | 3.39 | 6.63 | (0.134, 0.136) | 63.4 |
| Example 30 | Compound 30 | BD1 | 3.40 | 6.66 | (0.134, 0.137) | 64.0 |
| Example 31 | Compound 31 | BD1 | 3.37 | 6.65 | (0.134, 0.135) | 65.2 |
| Example 32 | Compound 32 | BD1 | 3.36 | 6.60 | (0.134, 0.136) | 66.3 |
| Example 33 | Compound 33 | BD1 | 3.28 | 6.67 | (0.135, 0.148) | 60.4 |
| Example 34 | Compound 34 | BD1 | 3.34 | 6.72 | (0.132, 0.136) | 80.2 |
| Example 35 | Compound 35 | BD1 | 3.32 | 6.65 | (0.134, 0.141) | 78.9 |
| Example 36 | Compound 36 | BD1 | 3.39 | 6.61 | (0.132, 0.136) | 81.3 |
| Example 37 | Compound 3 | BD2 | 3.43 | 7.01 | (0.131, 0.124) | 55.4 |
| Example 38 | Compound 11 | BD2 | 3.49 | 6.90 | (0.129, 0.125) | 53.8 |
| Example 39 | Compound 17 | BD2 | 3.40 | 6.98 | (0.130, 0.126) | 58.4 |
| Example 40 | Compound 26 | BD2 | 3.42 | 6.99 | (0.129, 0.125) | 53.1 |

(continued)

| Experimental Example 20 mA/cm$^2$ | Host | Dopant | Voltage (V) (@20 mA/cm$^2$) | Cd/A (@20 mA/cm$^2$) | Color coordinate (x,y) | Service life (T95, h) (@20 mA/cm$^2$) |
|---|---|---|---|---|---|---|
| Example 41 | Compound 31 | BD2 | 3.48 | 7.00 | (0.129, 0.125) | 57.4 |
| Comparative Example 1 | BH1 | BD1 | 3.90 | 5.88 | (0.134, 0.139) | 40.8 |
| Comparative Example 2 | BH2 | BD1 | 3.72 | 5.12 | (0.134, 0.149) | 8.2 |
| Comparative Example 3 | BH3 | BD1 | 3.80 | 5.30 | (0.134, 0.156) | 7.4 |
| Comparative Example 4 | BH4 | BD1 | 3.71 | 5.23 | (0.135, 0.148) | 11.5 |
| Comparative Example 5 | BH5 | BD1 | 3.81 | 5.51 | (0.134, 0.138) | 23.0 |
| Comparative Example 6 | BH6 | BD1 | 3.78 | 5.53 | (0.134, 0.137) | 20.8 |
| Comparative Example 7 | BH7 | BD1 | 3.69 | 4.98 | (0.134, 0.137) | 21.5 |
| Comparative Example 8 | BH1 | BD2 | 4.03 | 6.03 | (0.130, 0.129) | 28.4 |
| Comparative Example 9 | BH5 | BD2 | 3.90 | 6.11 | (0.134, 0.138) | 13.2 |
| Comparative Example 10 | BH7 | BD2 | 3.74 | 5.23 | (0.130, 0.129) | 18.4 |

Chemical Formula 1 according to an exemplary embodiment of the present specification is intended to improve the blue light emitting device by improving the characteristics of an anthracene blue fluorescent host into which a naphthofuran unit including oxygen was introduced.

By showing various derivatives known in the related art in Comparative Examples 1 to 10, it is possible to observe a clear improvement in the device performance of the blue device compared to those in the related art.

The organic electroluminescent device combined with the compound derivative of Chemical Formula 1 according to an exemplary embodiment of the present specification may adjust the role of smoothly injecting holes into the light emitting layer by using the compounds of Preparation Examples 6 and 7 as the host of the blue organic electroluminescent device, and the device according to the present specification exhibits excellent characteristics in terms of efficiency, driving voltage and stability through the balance between holes and electrons of the organic light emitting device according to the chemical structure.

## Claims

**1.** A compound of the following Chemical Formula 1:

[Chemical Formula 1]

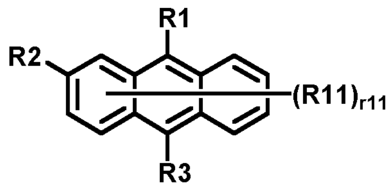

wherein, in Chemical Formula 1,

at least one of R1 to R3 is -L-Ar1, at least one of the rest of R1 to R3, which are not -L-Ar1, is a substituted or unsubstituted heteroaryl group, and the other and R11 are the same as or different from each other, and are each independently hydrogen; deuterium; a substituted or unsubstituted aryl group; or a substituted or unsubstituted heteroaryl group,
r11 is an integer from 1 to 7, and when r11 is 2 or higher, two or more R11's are the same as or different from each other,
L is a direct bond; a substituted or unsubstituted arylene group; or a substituted or unsubstituted heteroarylene group, and
Ar1 is the following Chemical Formula A,

[Chemical Formula A]

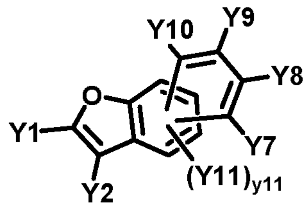

in Chemical Formula A,

any one of Y1, Y2 and Y7 to Y11 is a moiety bonded to L, and the others of Y1, Y2 and Y7 to Y11 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring, and
y11 is 1 or 2, and when y11 is 2, two Y11's are the same as or different from each other.

**2.** The compound of claim 1, wherein Chemical Formula A is any one of the following Chemical Formulae A-1 to A-3:

[Chemical Formula A-1]

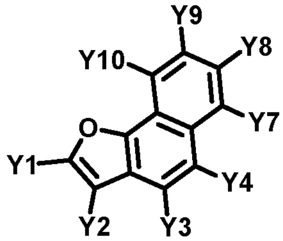

[Chemical Formula A-2]

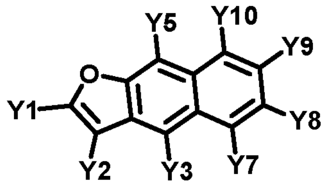

[Chemical Formula A-3]

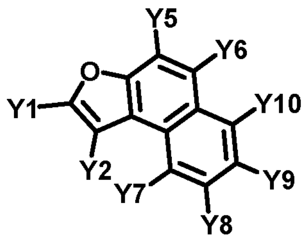

in Chemical Formula A-1,

any one of Y1 to Y4 and Y7 to Y10 is a moiety bonded to L, and the others of Y1 to Y4 and Y7 to Y10 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,

in Chemical Formula A-2,

any one of Y1 to Y3, Y5 and Y7 to Y10 is a moiety bonded to L, and the others of Y1 to Y3, Y5 and Y7 to Y10 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; or a substituted or unsubstituted heteroaryl group, or any one or more adjacent pairs are bonded to each other to form a substituted or unsubstituted ring,

in Chemical Formula A-3,

any one of Y1, Y2 and Y5 to Y10 is a moiety bonded to L, and the others of Y1, Y2 and Y5 to Y10 which are not bonded to L are the same as or different from each other, and are each independently hydrogen;

deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring.

3. The compound of claim 1, wherein Chemical Formula 1 is any one of the following Chemical Formulae 1-1 to 1-3:

[Chemical Formula 1-1]

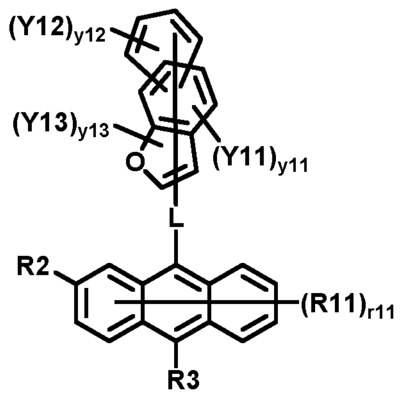

[Chemical Formula 1-2]

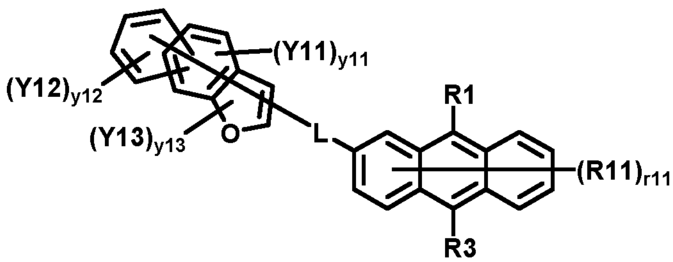

[Chemical Formula 1-3]

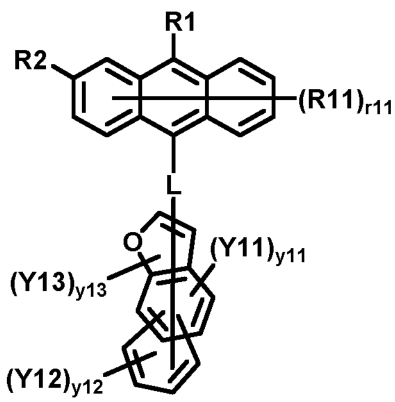

in Chemical Formulae 1-1 to 1-3,

the definitions of R1 to R3, R11, r11 and L are the same as the definitions in Chemical Formula 1,
any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
y11 and y13 are each 1 or 2,
y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

4. The compound of claim 1, wherein Chemical Formula 1 is any one of the following Chemical Formulae 1-4 to 1-6:

[Chemical Formula 1-4]

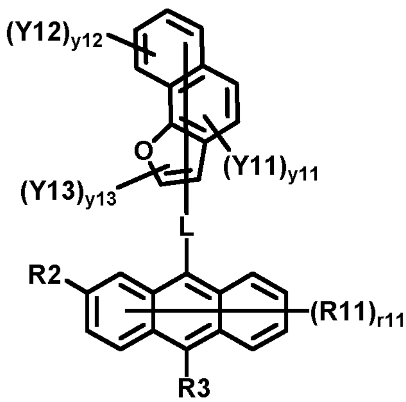

[Chemical Formula 1-5]

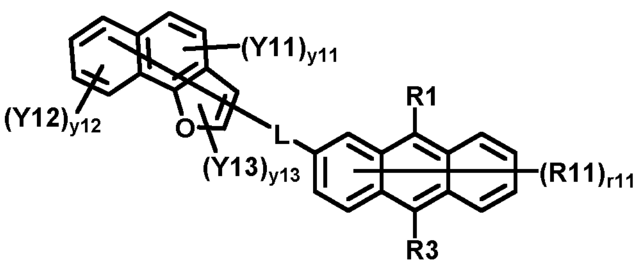

[Chemical Formula 1-6]

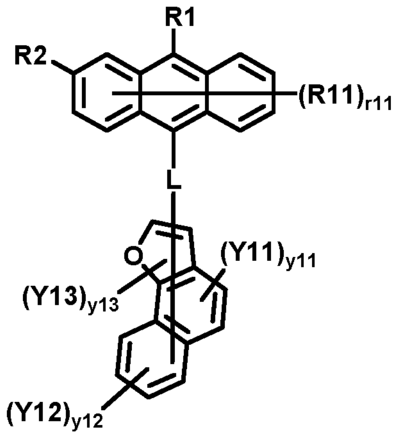

in Chemical Formulae 1-4 to 1-6,
the definitions of R1 to R3, R11, r11 and L are the same as the definitions in Chemical Formula 1,
any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
y11 and y13 are each 1 or 2,
y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

**5.** The compound of claim 1, wherein Chemical Formula 1 is any one of the following Chemical Formulae 1-7 to 1-9:

[Chemical Formula 1-7]

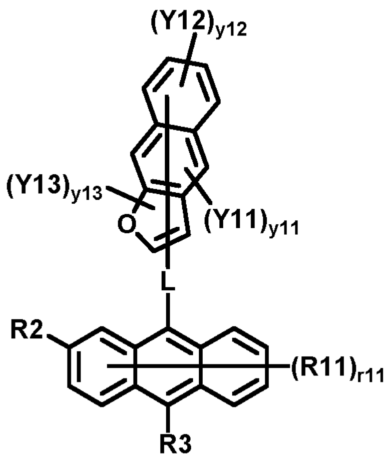

[Chemical Formula 1-8]

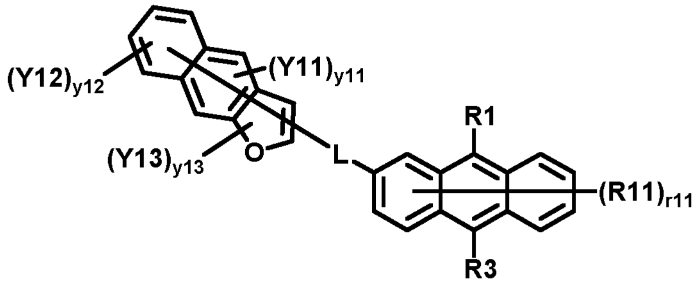

[Chemical Formula 1-9]

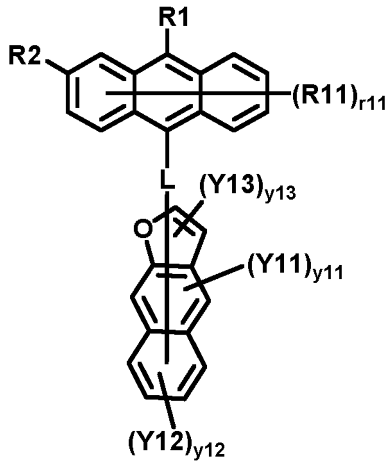

in Chemical Formulae 1-7 to 1-9,

the definitions of R1 to R3, R11, r11 and L are the same as the definitions in Chemical Formula 1,
any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent pairs are bonded to each other to form a substituted or unsubstituted ring,
y11 and y13 are each 1 or 2,
y12 is an integer from 1 to 4,
$y11 + y12 + y13 \leq 7$,
when y11 is 2, two Y11's are the same as or different from each other,
when y13 is 2, two Y13's are the same as or different from each other, and
when y12 is 2 or higher, two or more Y12's are the same as or different from each other.

**6.** The compound of claim 1, wherein Chemical Formula 1 is any one of the following Chemical Formulae 1-10 to 1-12:

[Chemical Formula 1-10]

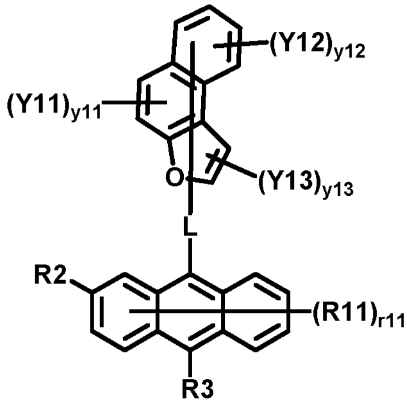

[Chemical Formula 1-11]

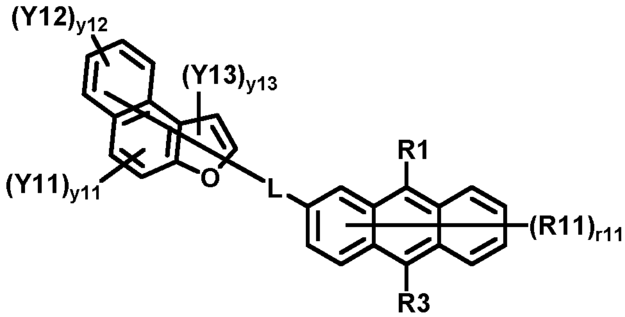

[Chemical Formula 1-12]

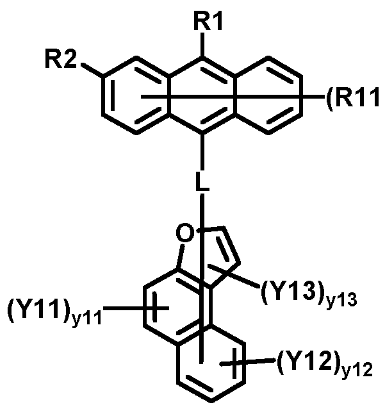

in Chemical Formulae 1-10 to 1-12,

the definitions of R1 to R3, R11, r11 and L are the same as the definitions in Chemical Formula 1,
any one of Y11 to Y13 is a moiety bonded to L, and the others of Y11 to Y13 which are not bonded to L are the same as or different from each other, and are each independently hydrogen; deuterium; a halogen group; a substituted or unsubstituted alkyl group; a substituted or unsubstituted cycloalkyl group; a substituted or unsubstituted alkenyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted silyl group; a substituted or unsubstituted amine group; a substituted or unsubstituted aryl group; a fused ring group of an aromatic hydrocarbon ring and an aliphatic hydrocarbon ring, which is substituted or unsubstituted; or a substituted or unsubstituted heteroaryl group, or any one or more adjacent

pairs are bonded to each other to form a substituted or unsubstituted ring,
y11 and y13 are each 1 or 2,
y12 is an integer from 1 to 4,

$$y11 + y12 + y13 \leq 7,$$

when y11 is 2, two Y11's are the same as or different from each other,
when y13 is 2, two Y13's are the same as or different from each other, and
when y12 is 2 or higher, two or more Y12's are the same as or different from each other.

**7.** The compound of claim 1, wherein at least one of R1 to R3 is -L-Ar1, at least one of the rest of R1 to R3, which are not -L-Ar1, is a monocyclic or polycyclic heteroaryl group having 2 to 30 carbon atoms, which is unsubstituted or substituted with deuterium or a monocyclic or polycyclic aryl group having 6 to 30 carbon atoms, and the other and R11 are the same as or different from each other, and are each independently hydrogen; deuterium; or a monocyclic or polycyclic aryl group having 6 to 30 carbon atoms, which is unsubstituted or substituted with deuterium or a monocyclic or polycyclic aryl group having 6 to 30 carbon atoms, and
L is a direct bond; a monocyclic or polycyclic arylene group having 6 to 30 carbon atoms; or a monocyclic or polycyclic heteroarylene group having 2 to 30 carbon atoms.

**8.** The compound of claim 1, wherein any one of Y1, Y2 and Y7 to Y11 is a moiety bonded to L, and the others of Y1, Y2 and Y7 to Y11, which are not bonded to L, are the same as or different from each other, and are each independently hydrogen; deuterium; a straight-chained or branched alkyl group having 1 to 30 carbon atoms; a monocyclic or polycyclic cycloalkyl group having 3 to 30 carbon atoms; a monocyclic or polycyclic aryl group having 6 to 30 carbon atoms, which is unsubstituted or substituted with deuterium or a straight-chained or branched alkyl group having 1 to 30 carbon atoms; a fused ring group of a monocyclic or polycyclic aromatic hydrocarbon ring having 6 to 30 carbon atoms and a monocyclic or polycyclic aliphatic hydrocarbon ring having 3 to 30 carbon atoms, which is unsubstituted or substituted with a straight-chained or branched alkyl group having 1 to 30 carbon atoms; or a monocyclic or polycyclic heteroaryl group having 2 to 30 carbon atoms, or any one or more adjacent pairs are bonded to each other to form an aliphatic hydrocarbon ring having 3 to 30 carbon atoms, which is unsubstituted or substituted with a monocyclic or polycyclic alkyl group having 1 to 30 carbon atoms.

**9.** The compound of claim 1, wherein Chemical Formula 1 is any one selected from the following compounds:

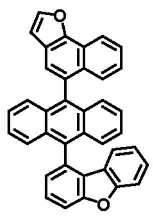
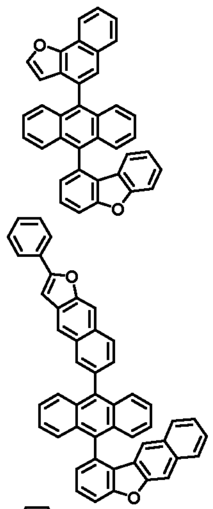
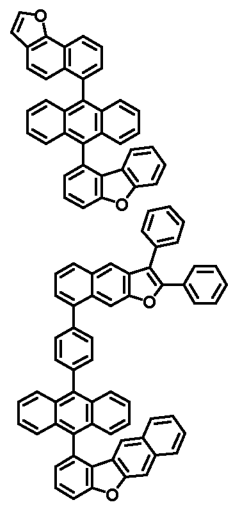
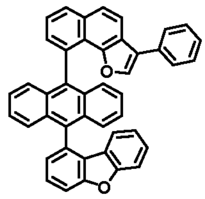
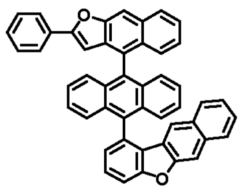
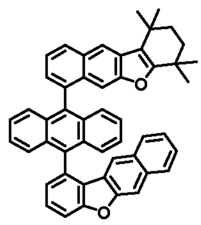
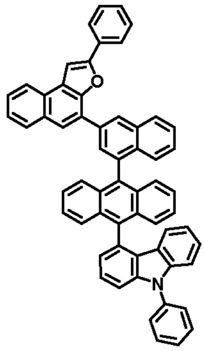
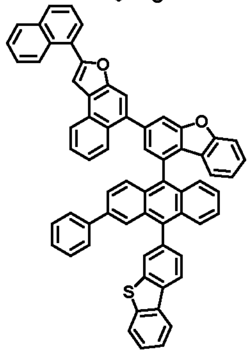
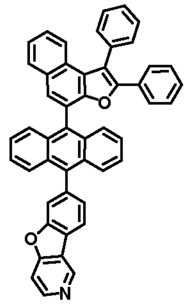
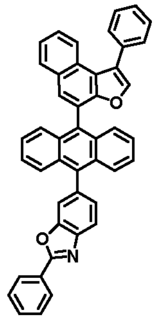
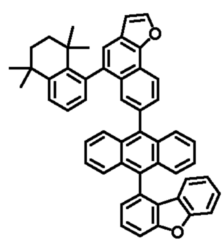
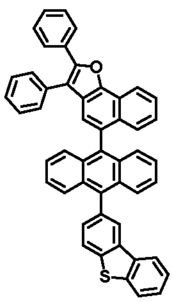
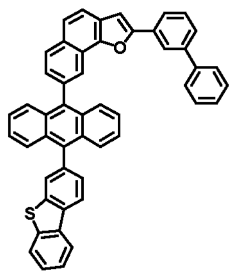
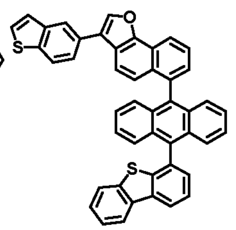
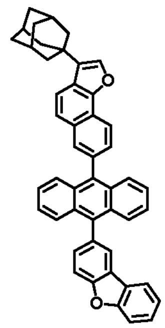
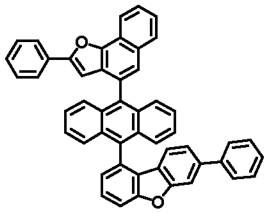
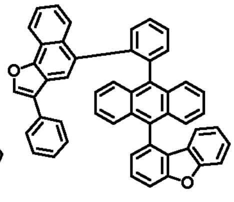
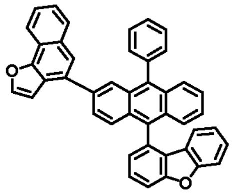

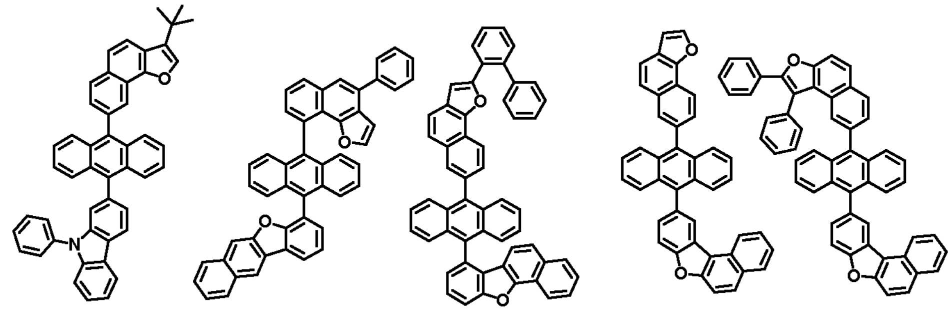

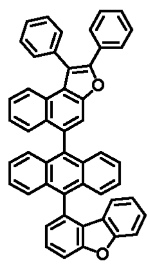

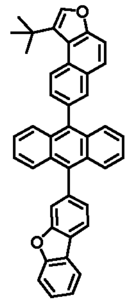

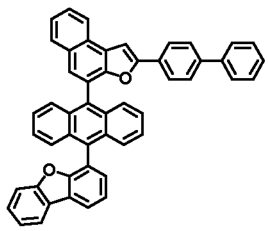

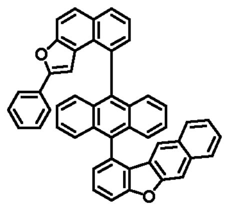

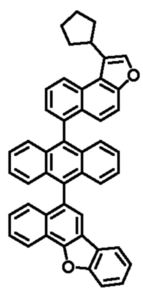

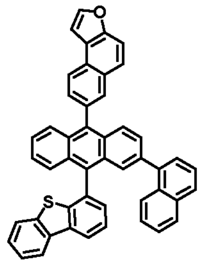

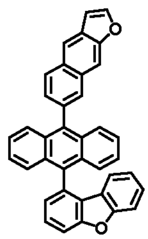

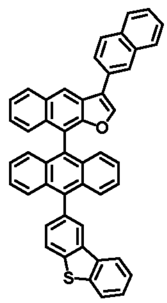

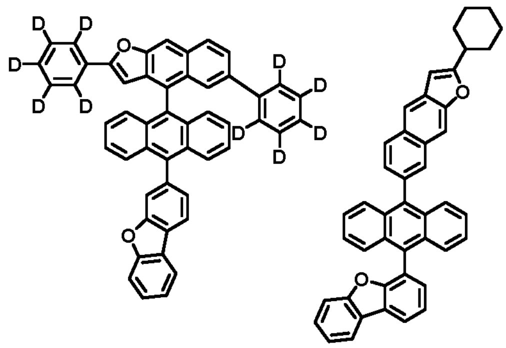

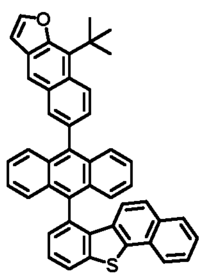

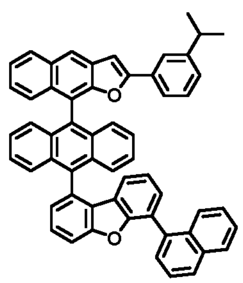

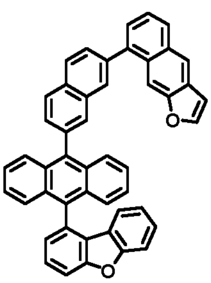

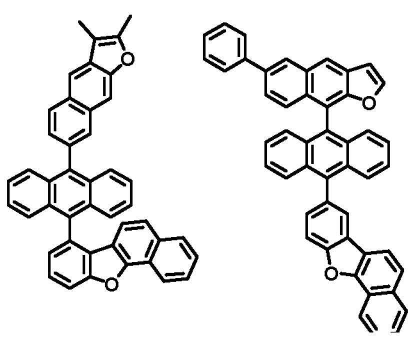

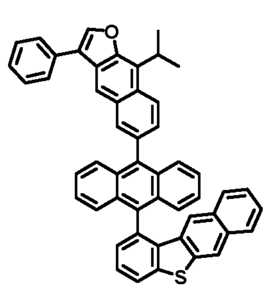

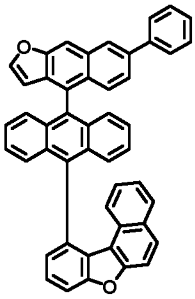 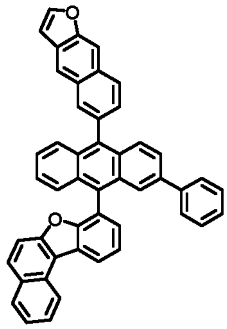 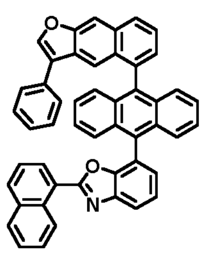 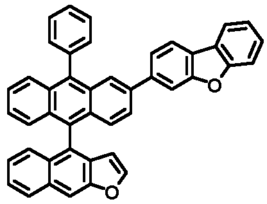

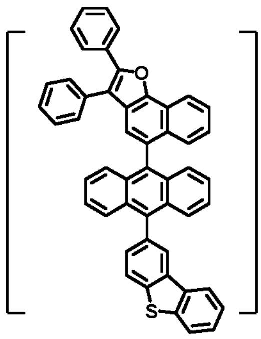 D26 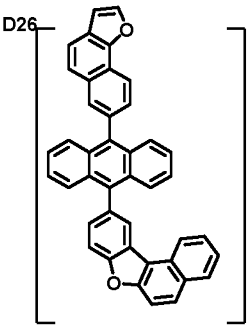 D22 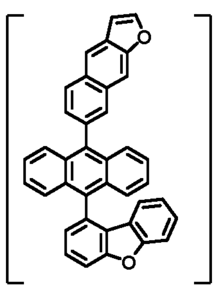 D21

**10.** An organic light emitting device comprising: a first electrode; a second electrode; and an organic material layer having one or more layers provided between the first electrode and the second electrode, wherein one or more layers of the organic material layer comprise the compound of any one of claims 1 to 9.

**11.** The organic light emitting device of claim 10, wherein the organic material layer comprises a light emitting layer, and the light emitting layer comprises the compound as a host of the light emitting layer.

**12.** The organic light emitting device of claim 11, wherein the light emitting layer comprises a dopant, and the dopant comprises a fluorescent dopant.

**13.** The organic light emitting device of claim 12, wherein the fluorescent dopant comprises one or more selected from among a pyrene-based compound and a non-pyrene-based compound.

**14.** The organic light emitting device of claim 13, wherein the non-pyrene-based compound comprises a boron-based compound.

**15.** The organic light emitting device of claim 11, wherein the light emitting layer further comprises one or more hosts different from the compound.

[Figure 1]

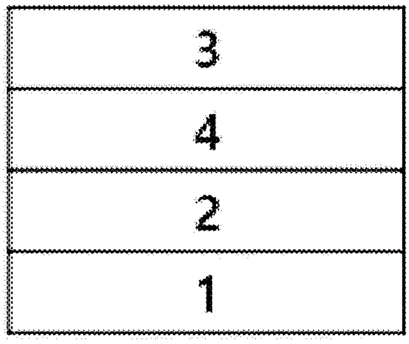


[Figure 2]

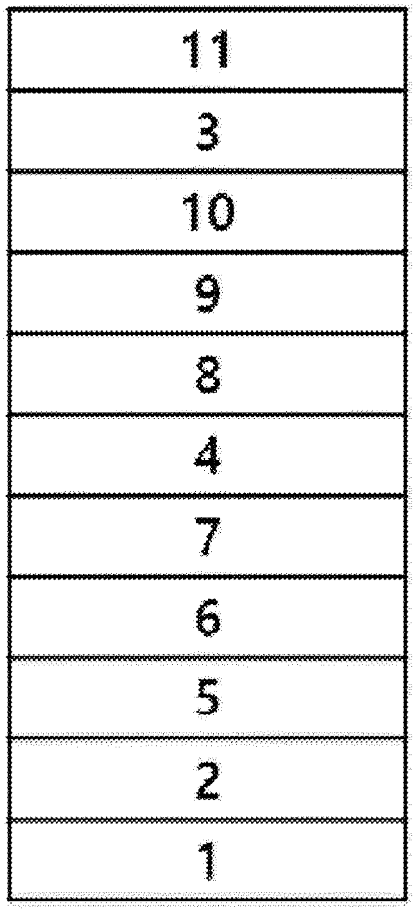

**INTERNATIONAL SEARCH REPORT**

International application No.
**PCT/KR2023/008514**

**A. CLASSIFICATION OF SUBJECT MATTER**

**C07D 407/10**(2006.01)i; **C07D 405/10**(2006.01)i; **C07D 409/14**(2006.01)i; **C07D 491/048**(2006.01)i; **C07D 413/10**(2006.01)i; **C07D 409/10**(2006.01)i; **H10K 85/60**(2023.01)i

According to International Patent Classification (IPC) or to both national classification and IPC

**B. FIELDS SEARCHED**

Minimum documentation searched (classification system followed by classification symbols)

C07D 407/10(2006.01); C07D 209/08(2006.01); C07D 209/10(2006.01); C07D 307/79(2006.01); C07D 307/92(2006.01); C07D 405/10(2006.01); C07D 407/14(2006.01); H01L 51/00(2006.01); H01L 51/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 유기발광소자(OLED, organic light emitting diode), 호스트(host), 발광층(light emitting layer), 안트라센(anthracene), 나프토퓨란(naphthofuran)

**C. DOCUMENTS CONSIDERED TO BE RELEVANT**

| Category* | Citation of document, with indication, where appropriate, of the relevant passages | Relevant to claim No. |
|---|---|---|
| X | JP 2005-047868 A (MITSUI CHEMICALS INC.) 24 February 2005 (2005-02-24)<br>See claims 1, 3 and 4; paragraphs [0074] and [0094]; and page 30. | 1-15 |
| X | KR 10-2022-0081057 A (LG CHEM, LTD.) 15 June 2022 (2022-06-15)<br>See claims 1 and 6-9; paragraphs [0211] and [0212]; and pages 12, 14 and 15. | 1-7,10-15 |
| X | KR 10-2022-0065451 A (LG CHEM, LTD.) 20 May 2022 (2022-05-20)<br>See claims 1 and 9-12; page 6, third compound, sixth compound and eleventh compound; and paragraphs [0294] and [0348]. | 1-7,10-15 |
| A | KR 10-2021-0125621 A (SAMSUNG DISPLAY CO., LTD.) 19 October 2021 (2021-10-19)<br>See entire document. | 1-7,10-15 |
| A | KR 10-2022-0042160 A (DUPONT ELECTRONICS, INC.) 04 April 2022 (2022-04-04)<br>See entire document. | 1-7,10-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2023** | **20 September 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2023/008514**

| Patent document cited in search report | | | Publication date (day/month/year) | Patent family member(s) | | | Publication date (day/month/year) |
|---|---|---|---|---|---|---|---|
| JP | 2005-047868 | A | 24 February 2005 | JP | 4392206 | B2 | 24 December 2009 |
| KR | 10-2022-0081057 | A | 15 June 2022 | None | | | |
| KR | 10-2022-0065451 | A | 20 May 2022 | None | | | |
| KR | 10-2021-0125621 | A | 19 October 2021 | CN | 113497199 | A | 12 October 2021 |
| | | | | US | 2021-0320256 | A1 | 14 October 2021 |
| KR | 10-2022-0042160 | A | 04 April 2022 | CN | 114514225 | A | 17 May 2022 |
| | | | | US | 2022-0162222 | A1 | 26 May 2022 |
| | | | | WO | 2021-021840 | A1 | 04 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020220079957
- KR 1538534